# EUROPEAN PATENT APPLICATION

(11) **EP 4 029 514 A1**
(43) Date of publication of application: **20.07.2022**
(21) Application number: 20863506.0
(22) Date of filing: 11.09.2020
(51) Int. Cl.: A61K 38/13, A61P 13/12, A61P 43/00, C12N 5/10

(54) **DRUG FOR CURATIVE THERAPY OF INTRACTABLE HEREDITARY RENAL ALPORT SYNDROME**

(30) Priority: 11.09.2019 JP 2019165583
(71) Applicant: National University Corporation Kumamoto University, Kumamoto 860-8555 (JP)
(72) Inventor: KAI, Hirofumi, Kumamoto-shi, Kumamoto 862-0973 (JP); SHUTO, Tsuyoshi, Kumamoto-shi, Kumamoto 862-0973 (JP); SUICO, Mary Ann, Kumamoto-shi, Kumamoto 862-0973 (JP); TSUKAMOTO, Sachiko, Kumamoto-shi, Kumamoto 862-0973 (JP); MISUMI, Shogo, Kumamoto-shi, Kumamoto 862-0973 (JP); KAWAHARA, Teppei, Kumamoto-shi, Kumamoto 862-0973 (JP); HITORA, Yuki, Kumamoto-shi, Kumamoto 862-0973 (JP); KATO, Hikaru, Kumamoto-shi, Kumamoto 862-0973 (JP); KOTANI, Shunsuke, Kumamoto-shi, Kumamoto 862-0973 (JP); ARAKI, Kimi, Kumamoto-shi, Kumamoto 860-8555 (JP)
(74) Representative: J A Kemp LLP
(86) International application number: PCT/JP2020/034538
(87) International publication number: WO 2021/049633

(57) **Abstract**

by screening using a high-throughput evaluation system relating to the promotion of the formation and extracellular secretion of trimers of the causative proteins COL4A3/A4/A5, which decreased in the renal tissue of patients with Alport syndrome (AS), it was found that cyclosporin A has an effect of promoting formation and extracellular secretion of trimers of type-IV collagen. From further studies, it was found that Alisporivir and NIM258, which do not inhibit calcineurin, also have an action to promote extracellular secretion of type-IV collagen. Furthermore, the present inventors have found that these actions are based on the cyclophilin D inhibitory mechanism, and have found a radical therapeutic agent for AS by inhibition of cyclophilin D. The present invention provides a composition and an AS therapeutic/prophylactic drug, which contain a cyclophilin D inhibitor as an active ingredient, for promoting the secretion of collagen trimers in cells having a mutated type-IV collagen gene.

## Description

### [CROSS REFERENCES TO RELATED APPLICATIONS]

This international application claims priority based on Japanese Patent Application No. 2019-165583 filed with the Japan Patent Office on September 11, 2019, and the entire contents of Japanese Patent Application No. 2019-165583 are incorporated in this international application by reference.

### [Technical Field]

The present invention relates to a collagen trimer formation promoter, particularly to the field of treatment of Alport syndrome.

### [Background Art]

Alport syndrome (AS) is a hereditary disease caused by a mutation in any of the type-IV collagen genes (COL4A3, COL4A4, COL4A5) that constitute the basement membrane of the glomerulus of the kidney. It is developed from childhood in many cases and is a serious disease that reaches the end-stage renal disease in the teens and twenties. Currently, the treatment of AS is symptomatic therapy with a rennin-angiotensin (RAS) inhibitor, as in other chronic kidney diseases. Patients eventually progress to end-stage renal failure without exception, and are forced to undergo dialysis or kidney transplantation.

Cyclosporin A (CsA) is a hydrophobic cyclic polypeptide consisting of 11 amino acids. CsA is used as an immunosuppressant for suppressing rejection during transplantation, and the like because it suppresses the production of cytokines by inhibiting calcineurin. In 1992, Callis et al. reported that administration of CsA delays the onset of AS, and suggested the possibility of treating AS with CsA (Non Patent Literature 1). Thereafter, an attempt was made to elucidate the mechanism, and the action on the hemodynamics of the glomerulus, and the like were also reported (Non Patent Literatures 2 and 3). In 2008, Faul et al. reported that in podocytes, CsA maintains phosphorylation of synaptopodin by inhibiting calcineurin and protects synaptopodin -which is an actin-interacting protein- from degradation by cathepsin L (Non Patent Literature 4). Based on this report, the mechanism of action by CsA has been considered to be the stabilization of the actin skeleton in podocytes (Non Patent Literatures 5 and 6) .

On the other hand, however, it has been reported that CsA also has side effects such as nephrotoxicity associated with inhibition of the calcineurin-NFAT pathway (Non Patent Literature 7).

Alisporivir is a compound reported as a cyclosporine derivative (Patent Literature 1), and has been developed as a therapeutic agent for hepatitis C virus.

As widely used AS models, COL4A3 deficient (Col4a3 KO) mouse (Non Patent Literature 8) and COL4A5 nonsense mutant (Col4a5-G5X) mouse (Non Patent Literature 9) can be mentioned. Both of them are models in which the expression itself of causative proteins α3 (IV) and α5 (IV) has been lost. Col4a5 G5X mutant mouse was produced by Dr. Michelle Rheault et al. of University of Minnesota, based on the mutant type of a boy who shows renal pathology from the age of 6. This mouse has a mutation at the fifth codon of Exon1 converted from Glycine to a stop codon, and shows almost no expression of α5(IV) variant since the mutation probably accompanies a nonsense mutationdependent mRNA degradation mechanism, etc., and α345(IV) on the renal glomerular basement membrane (GBM) is lost (Non Patent Literature 9). This mouse is a model that exhibits from continuous proteinuria leakage to inflammatory cell infiltration, focal segmental glomerulosclerosis (FSGS), interstitial fibrosis, etc., and reflects well the progressive clinical pathology of AS. Using this model mouse, the present inventors have also explored for pathological control factors and evaluated the efficacy of therapeutic drugs, and identified several therapeutic targets and therapeutic drug candidates. However, all of them target factors involved in the progression of pathological conditions and the effect of improving the renal pathology was limited. Therefore, a need for a model animal that more accurately reflects the pathology of α345(IV), which causes AS, has been recognized.

### [Citation List]

### [Patent Literature]

[PTL 1]
WO 2000/001715

### [Non Patent Literatures]

[NPL 1]
   L. Callis et al., Pediatric Nephrology (1992) 6: 140-144
[NPL 2]
   L. Callis et al., Kidney International (1999) 55: 1051-1056
[NPL 3]
   Dilys Chen et al., J. Am. Soc. Nephrol (2003) 14: 690-698
[NPL 4]
   Christian Faul et al., Nat. Med. (2008) 14(9): 931-938
[NPL 5]
   Massela, L et al., Pediatric Nephrology (2010) 25(7): 1269-1275
[NPL 6]
   Sugimoto, K et al., Clin. Exp. Nephrol. (2014) 18: 492-498
[NPL 7]
   Charbit, M et al., Pediatric Nephrology (2007) 22: 57-63
[NPL 8]
   Miner JH et al., J. Cell Biol. (1996) 135: 1403-1413.
[NPL 9]
   Rheault MN et al., J. Am. Soc. Nephrol. (2004) 15: 1466-1474.

### [Summary of Invention]

### [Technical Problem]

The present invention aims to provide a method for treating AS, that is safer and directly acts on the onset mechanism of the disease.

### [Solution to Problem]

The present inventors took note of a decrease in the formation and extracellular secretion of a trimer of the causative proteins COL4A3/A4/A5 that previously decreased in AS renal tissues, studied the possibility of a radical treatment method for AS by improving them, and successfully constructed a system (evaluation system by split NanoLuc) that can evaluate them with high throughput (Omachi K. et al., Cell Chem. Biol. (2018) 25: 634). Using the constructed system, they screened an independently collected microbial extract library, and clarified that CsA having the following structure promotes extracellular secretion of the trimer of type-IV collagen, which is the causative protein of AS.

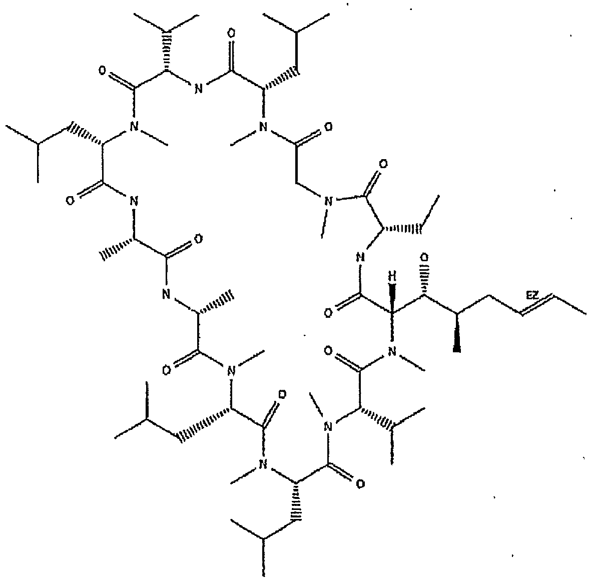

Until now, it has been considered that the efficacy of CsA on AS is achieved by the calcineurin inhibitory activity of CsA. Thus, PSC-833-which is a derivative of CsA but does not inhibit calcineurin and has no immunosuppressive action- was tested, and no effect of promoting the secretion of mutant collagen trimer was confirmed. Therefore, even if CsA is effective for AS, it could not be inferred that a derivative thereof has the same effect.

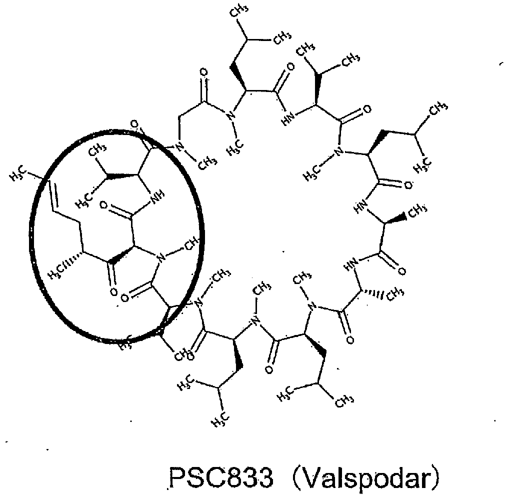

The present inventors conducted further studies of the action of CsA derivatives on AS, and unexpectedly found that Alisporivir -which does not inhibit calcineurin and has no immunosuppressive action like PSC833- has an action to promote the secretion of AS mutant collagen trimer.

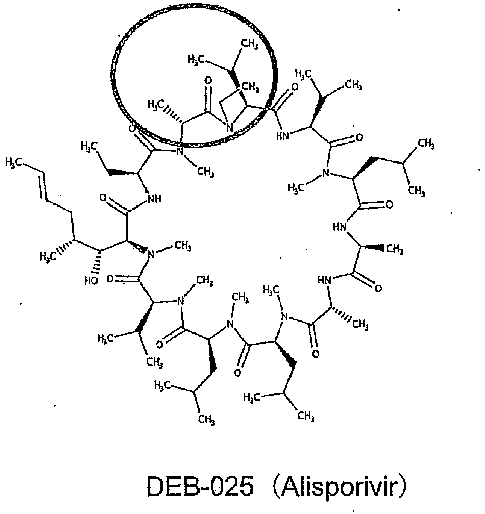

Furthermore, the present inventors have also investigated other compounds, and found that NIM258-which does not inhibit calcineurin and has no immunosuppressive action-also has an action to promote the secretion of AS mutant collagen trimer.

Therefrom the present inventors have found that CsA, Alisporivir, and NIM258 afford the formation and extracellular secretion of type-IV collagen trimer based on a new mechanism completely different from the calcineurin pathway, and enable the radical treatment of AS, particularly that activities other than the calcineurin pathway that Alisporivir and NIM258 have achieve an AS therapeutic effect by promoting formation/secretion of type-IV collagen trimer.

Until now, it has been considered difficult to clinically apply CsA as a therapeutic drug for AS because of the nephrotoxicity associated with its calcineurin inhibitory activity. That Alisporivir, which has already been confirmed to have no nephrotoxicity in clinical trials (S. Zeuzem et al., Aliment Pharmacol. Ther. (2015) 42: 829-844; M. Buti et al., J. of Viral Hepatitis (2015) 22: 596-606), and NIM258, which similarly has no immunosuppressive action, show a secretagogue action on type-IV collagen and are effective in treating AS means that the problem of nephrotoxicity that has prevented the clinical application of CsA can be solved, whereby the present inventors have succeeded for the first time in providing a radical therapeutic drug for AS with low nephrotoxicity.

Furthermore, the present inventors elucidated the mechanism by which CsA and Alisporivir promote the formation and secretion of trimers of type-IV collagen, and found that this mechanism is due to the involvement of cyclophilin D. Specifically, in cells lacking cyclophilin D, the promoting action of CsA and Alisporivir on the formation and secretion of trimers of type-IV collagen is not sufficiently exhibited. Therefore, they have found that CsA and Alisporivir promote the formation and secretion of trimers of type-IV collagen by acting on cyclophilin D. In addition, the present inventors investigated the role of cyclophilin D in the formation and secretion of trimers of type-IV collagen, and found that knockdown of cyclophilin D promotes the formation and secretion of trimers of type-IV collagen.

From the above, the present inventors have found that Alisporivir promotes extracellular secretion of trimers of type-IV collagen, which is abnormal in AS, and becomes a curative drug for AS, that the mechanism of promoting the formation and secretion of trimers of type-IV collagen by CsA and Alisporivir is based on the inhibition of cyclophilin D, and that cyclophilin D is a new target for the formation and secretion of trimers of type-IV collagen.

On the other hand, the present inventors also analyzed the mutations of type-IV collagen for which promotion of the formation of trimers by inhibition of cyclophilin D can be effective. As a result, it was found that the inhibition is particularly effective for deficiency in the formation and secretion of trimers of type-IV collagen due to amino acid mutations in or around the region of Exon 41 in COL4A5.

### [Advantageous Effects of Invention]

The present invention promotes the formation and extracellular secretion of trimers of type-IV collagen. Therefore, it can contribute to the radical treatment and prevention of diseases caused by deficiency in the formation and secretion of trimers of type-IV collagen represented by AS. Particularly, the present invention is effective for diseases caused by deficiency in the formation and secretion of trimers of type-IV collagen due to mutations in or around the region of Exon 41 in COL4A5.

### [Brief Description of Drawings]

[Fig. 1]

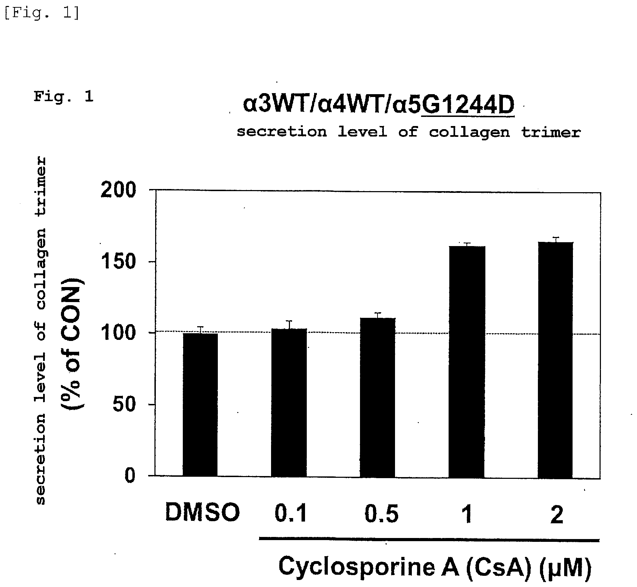

   Fig. 1 is a graph showing the secretion level of collagen trimer containing COL4A5 (G1244D) when treated with CsA. In the graph, the numerical value on the horizontal axis shows the concentration (µM) of CsA, and the vertical axis shows the secretion level of collagen trimer as a ratio (% of control) to the control (dimethyl sulfoxide (DMSO)-treated group). The "DMSO" on the horizontal axis shows the DMSO treatment group. The secretion level of collagen trimer increases depending on the concentration of CsA.
[Fig. 2]

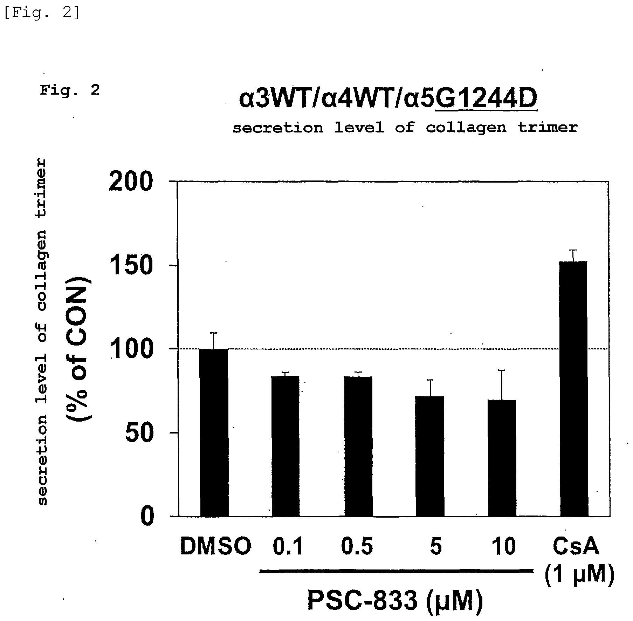

   Fig. 2 is a graph showing the secretion level of collagen trimer containing COL4A5 (G1244D) when treated with PSC-833. In the graph, the numerical value on the horizontal axis shows the concentration (µM) of PSC-833, and the vertical axis shows the secretion level of collagen trimer as a ratio (% of control) to the control (dimethyl sulfoxide (DMSO)-treated group). The "DMSO" on the horizontal axis shows the DMSO treatment group, and "CsA" shows cyclosporine A-treated group (1 µM) (positive control). The secretion level of collagen trimer does not increase even when the amount of PSC-833 is increased to 10 µM.
[Fig. 3]

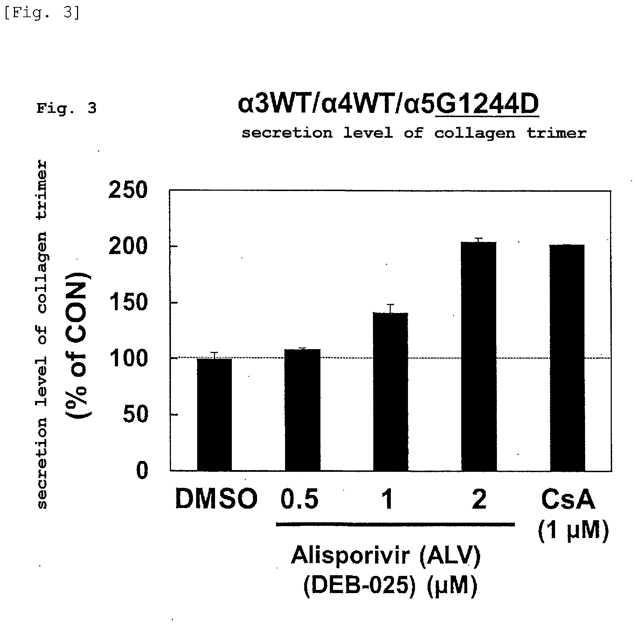

   Fig. 3 is a graph showing the secretion level of collagen trimer containing COL4A5 (G1244D) when treated with Alisporivir. In the graph, the numerical value on the horizontal axis shows the concentration (µM) of Alisporivir, and the vertical axis shows the secretion level of collagen trimer as a ratio (% of control) to the control (DMSO-treated group). The "DMSO" on the horizontal axis shows the DMSO treatment group, and "CsA" shows CsA-treated group (positive control). The secretion level of collagen trimer increases depending on the concentration of Alisporivir.
[Fig. 4]

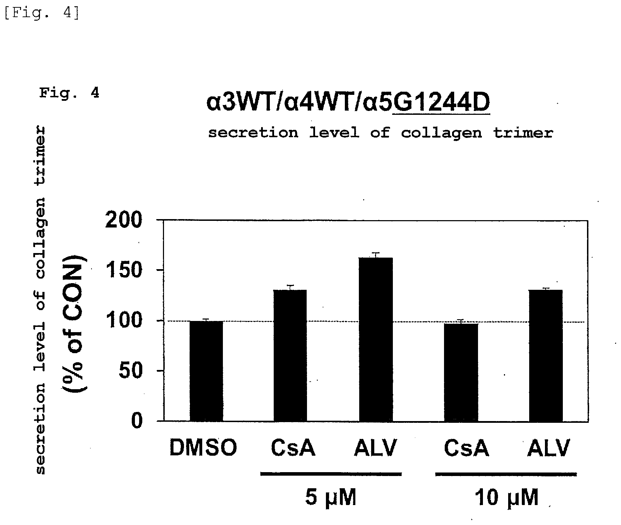

   Fig. 4 is a graph showing the results of the experiments of Fig. 1 and Fig. 3 performed under treatment conditions with higher concentrations of CsA (5, 10 µM) and Alisporivir (5, 10 µM). In the graph, the horizontal axis shows the concentrations of cyclosporine A (CsA) and Alisporivir (ALV), and the vertical axis shows the secretion level of collagen trimer as a ratio (% of control) to the control (DMSO-treated group). The "DMSO" on the horizontal axis shows the DMSO treatment group, "ALV" shows the Alisporivir-treated group, and "CsA" shows the CsA-treated group (positive control). At higher concentrations, the secretion level of collagen trimer tended to be high for Alisporivir as compared with CsA.
[Fig. 5]

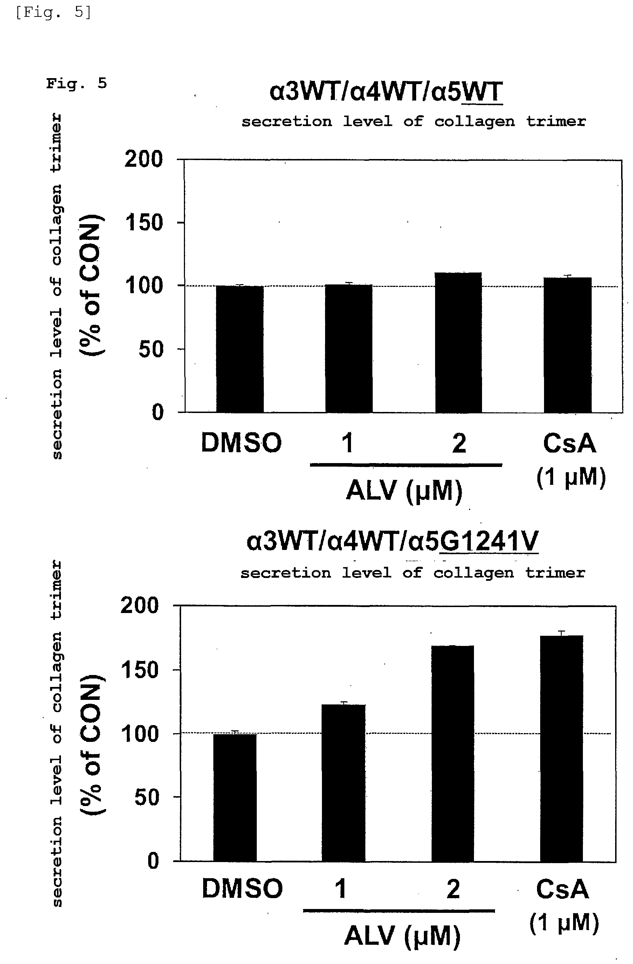

   Fig. 5 includes graphs showing the secretion level of collagen trimer when wild-type (α3WT/α4WT/α5WT: upper graph) and a cell line with G1241V amino acid mutation in COL4A5 (α3 and α4 are wild-type) (α3WT/α4WT/α5G1241V: lower graph) were each treated with Alisporivir. In the graph, the horizontal axis shows the concentration (µM) of Alisporivir, and the vertical axis shows the secretion level of collagen trimer as a ratio (% of control) to the control (DMSO-treated group). The "DMSO" on the horizontal axis shows the DMSO treatment group, "ALV" shows the Alisporivir-treated group, and "CsA" shows the CsA-treated group (positive control). It was shown that the trimer formation and secretion promoting effect by CsA and Alisporivir is higher in the mutant type than in the wild type.
[Fig. 6]

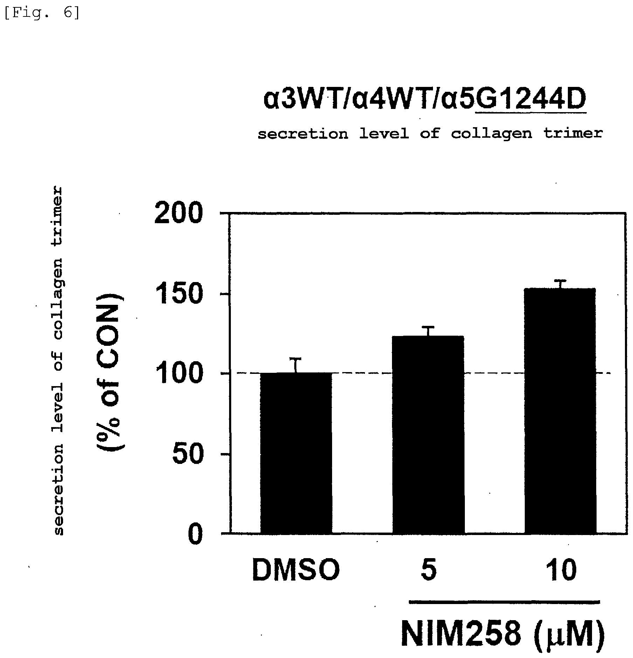

   Fig. 6 is a graph showing the secretion level of collagen trimer when a cell line with G1241V amino acid mutation in COL4A5 (α3 and α4 are wild-type) (α3WT/α4WT/α5G1241V: lower graph) was treated with NIM258. In the graph, the horizontal axis shows the concentration (µM) of NIM258, and the vertical axis shows the secretion level of collagen trimer as a ratio (% of control) to the control (DMSO-treated group). The "DMSO" on the horizontal axis shows the DMSO treatment group, and "NIM258" shows the NIM258-treated group. A trimer formation and secretion promoting effect by NIM258 was shown.
[Fig. 7]

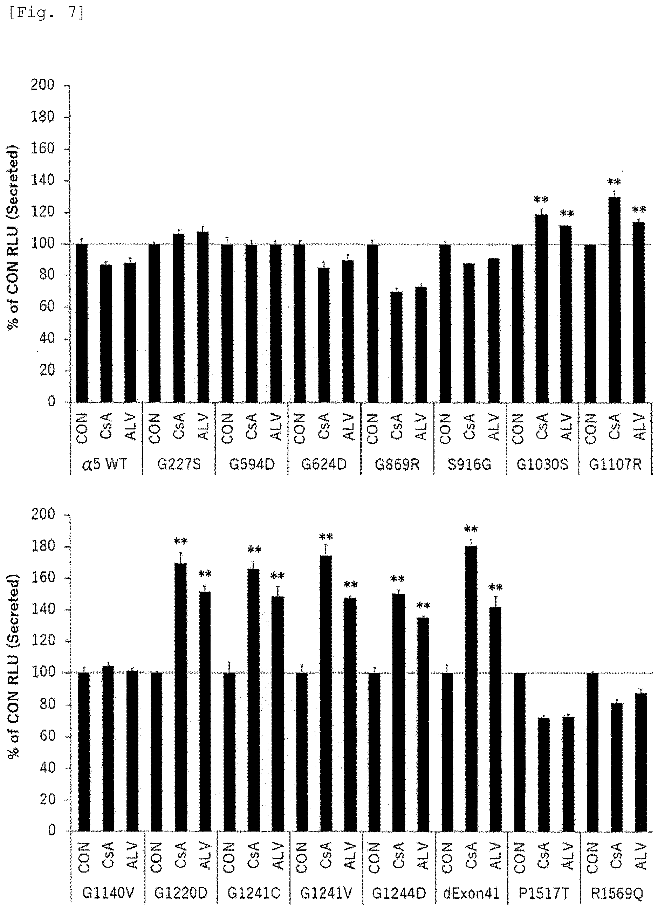

   Fig. 7 is a graph showing the measurement results of the trimer formation and secretion promoting ability of CsA and Alisporivir in cells of a wide range of clinically reported AS variants (G227S, G594D, G624D, G869R, S916G, G1030S, G1107R, G1140V, G1220D, G1241C, G1241V, G1244D, P1517T, and R1569Q of COL4A5), and Exon41 variants (1202nd - 1263rd amino acids of COL4a5)-deleted (dExon41) variant). The vertical axis shows the secretion level of collagen trimer as a ratio (% of control) of each cell to the control (DMSO-treated group). α5WT shows wild-type COL4a5. The "CON" on the horizontal axis shows the control (DMSO-treated group), "CsA" shows the CsA-treated group (positive control), and "ALV" shows the Alisporivir-treated group. It was shown that the trimer formation and secretion promoting effect by CsA and Alisporivir was particularly high in G1030S, G1107R, G1220D, G1241C, G1241V, G1244D, and Exon41-deleted cells.
[Fig. 8]

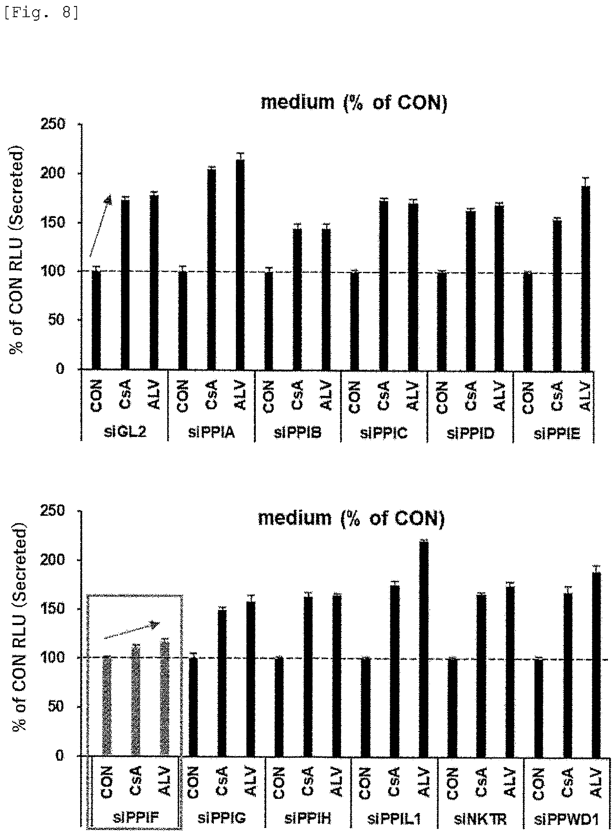

   Fig. 8 is a graph showing the examination results of the collagen trimer formation promoting effect by adding CsA and Alisporivir to G1244D mutation-transfected HEK293T cells in which cyclophilins (PPIA (cyclophilin A), PPIB, PPIC, PPID, PPIE, PPIF (cyclophilin D), PPIG, PPIH, PPIL1, NKTR, PPWD1) were knocked down with siRNA, in order to elucidate the collagen trimer formation promoting mechanism by CsA and Alisporivir. The vertical axis shows the secretion level of collagen trimer as a ratio (% of control) of each cell to the control (DMSO-treated group). In the horizontal axis, "CON" shows the control (DMSO-treated group), "CsA" shows the CsA-treated group (positive control), and "ALV" shows the Alisporivir-treated group. A trimer formation and secretion promoting ability by CsA and Alisporivir was particularly inhibited in PPIF (cyclophilin D) cells.
[Fig. 9]

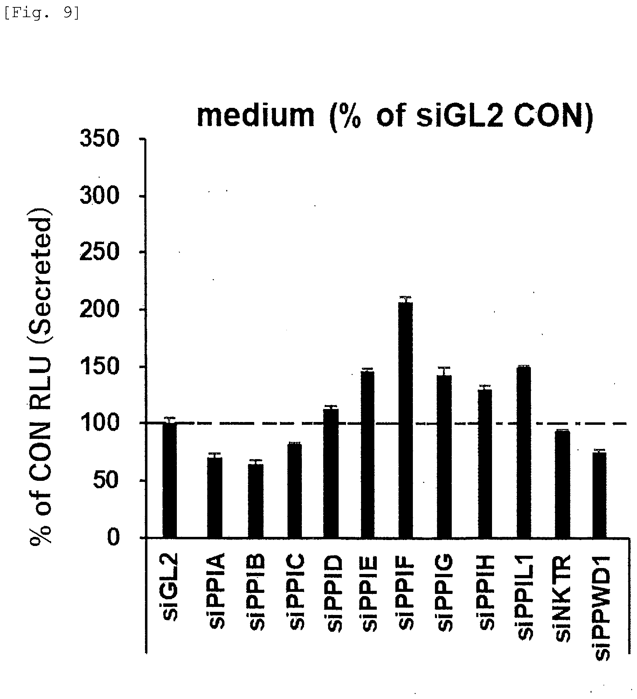

   Fig. 9 is a graph showing the results of investigating collagen trimer forming ability in cells in which various cyclophilins (PPIA (cyclophilin A), PPIB, PPIC, PPID, PPIE, PPIF (cyclophilin D), PPIG, PPIH, PPIL1, NKTR, PPWD1) were knocked down with siRNA, using G1244D mutant-transfected HEK293T cells. The vertical axis shows the secretion level of collagen trimer as a ratio (% of control) to the control (siGL2-treated group). The horizontal axis shows the type of siRNA used. Particularly, promotion of collagen trimer formation and secretion was confirmed in the cells treated with siPPIF.
[Fig. 10]

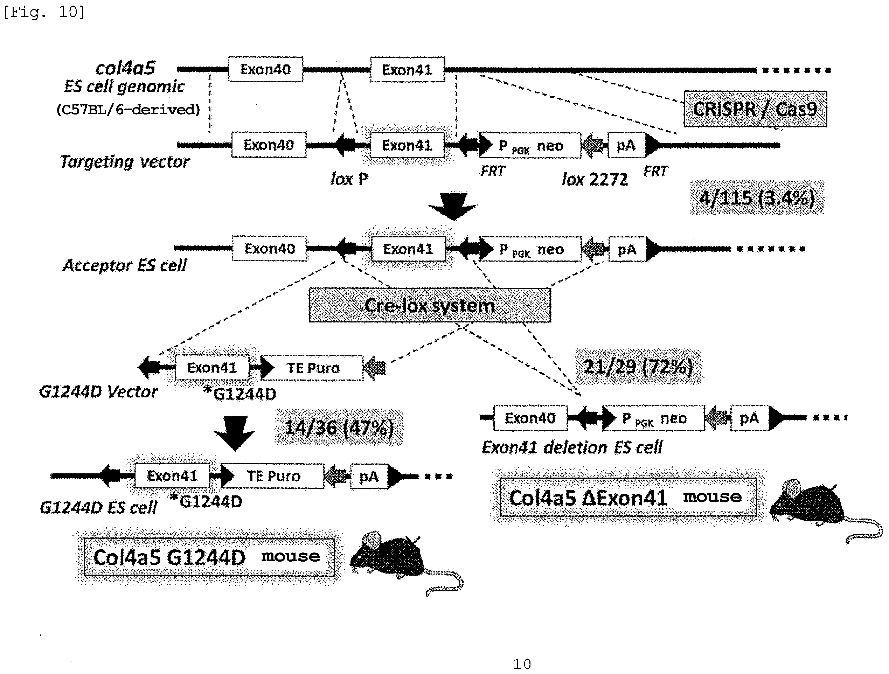

   Fig. 10 is a schematic diagram showing the nucleic acids used in producing Col4a5G1244D mouse and Col4a5ΔExon41 mouse.
[Fig. 11]
   Photographs showing expression of Laminin in ΔExon41 mouse, and Western blot (WB) photograph (inside of frame) of cell/basement membrane lysate.
[Fig. 12]

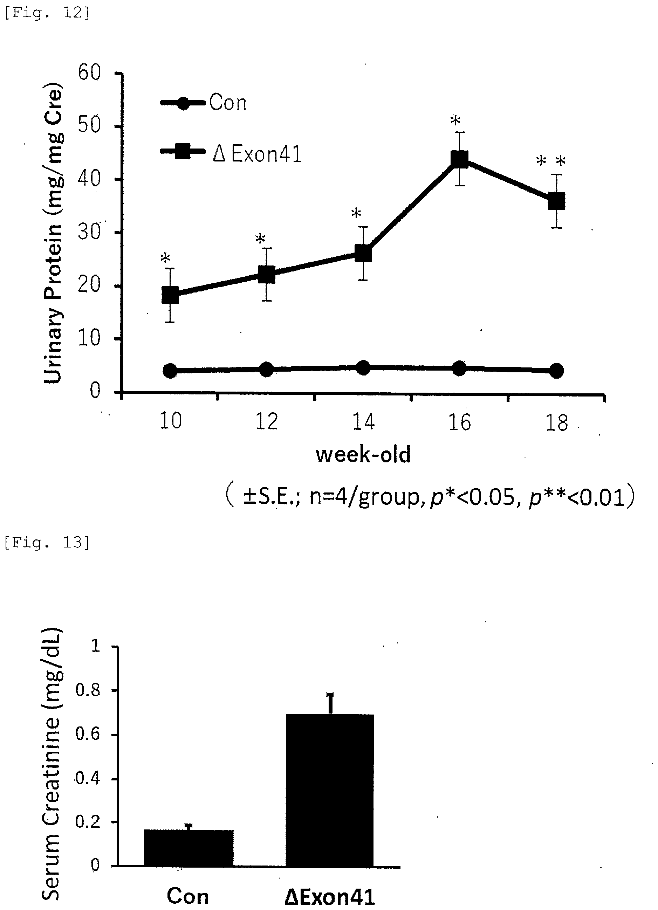

   Fig. 12 is a graph showing the urinary protein level of ΔExon41mouse. The vertical axis shows urinary protein level (mg/mg Cre), and the horizontal axis shows age in weeks (week-old). Black circles show wild-type mouse, and black squares show ΔExon41 mouse.
[Fig. 13]
   Fig. 13 is a graph showing the serum creatinine value of ΔExon41mouse. The vertical axis shows serum creatinine value (mg/dL). Con in the horizontal axis shows wild-type mouse, and ΔExon41shows ΔExon41mouse.
[Fig. 14]

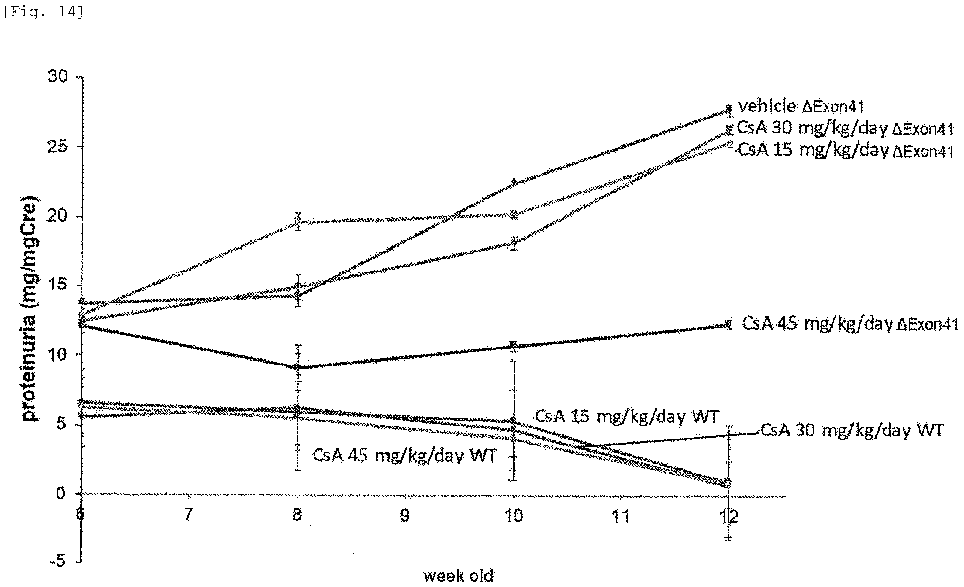

   Fig. 14 is a graph showing changes in the urinary protein after administration of CsA to ΔExon41mouse. The vertical axis shows urinary protein level (mg/mg Cre), and the horizontal axis shows age in weeks (week-old). Administration of CsA suppressed an increase in the urinary protein.

### [Description of Embodiments]

In one embodiment, the present invention relates to a composition for promoting the formation and/or secretion of collagen trimers in cells having a type-IV collagen gene having a mutation, which composition contains a cyclophilin D inhibitor as an active ingredient, or an agent for promoting the formation and/or secretion of collagen trimers.

As the cyclophilin D inhibitor, Alisporivir, as well as, for example, the compounds described in WO2009/018179; WO2012/103520; US9,132,138B; WO2014/093632; JP2016-124821A; JP2017-513490A; WO2015/200725; US9,738,615B; WO2016/112321; US10,179,161B; WO2019/173382 can be mentioned. The cyclophilin D inhibitor may also be a substance described as a cyclophilin D selective inhibitor in these documents.

Among these substances, a substance that does not show a calcineurin inhibitory action is preferred as the cyclophilin D inhibitor. Whether or not the candidate substance exhibits a calcineurin inhibitory action can be determined, for example, using a system in which a reporter gene is inserted downstream of the NFAT binding site because calcineurin is transferred into the nucleus by dephosphorylating the activated T cell nuclear factor (NF-AT) (Blood (2000) 96(2): 459-466.). When the expression of the reporter gene does not decrease in the presence of a candidate molecule, the candidate molecule can be determined to show no calcineurin inhibitory action. In the present specification, showing no calcineurin inhibitory action means that a calcineurin inhibitory action may be completely absent but generally, the degree of calcineurin inhibition is sufficiently low and does not cause problems such as toxicity when used for the purpose of the present invention. As one example, the absence of calcineurin inhibitory action may mean that the calcineurin inhibitory action is lower than that of CsA. In addition, since calcineurin inhibition appears as immunosuppressive activity as a biological activity, the cyclophilin D inhibitor may be a substance that does not exhibit immunosuppressive activity.

For example, a cyclosporine derivative represented by the following formula, or a salt thereof, or a hydrate or solvate thereof can be used as the cyclophilin D inhibitor: 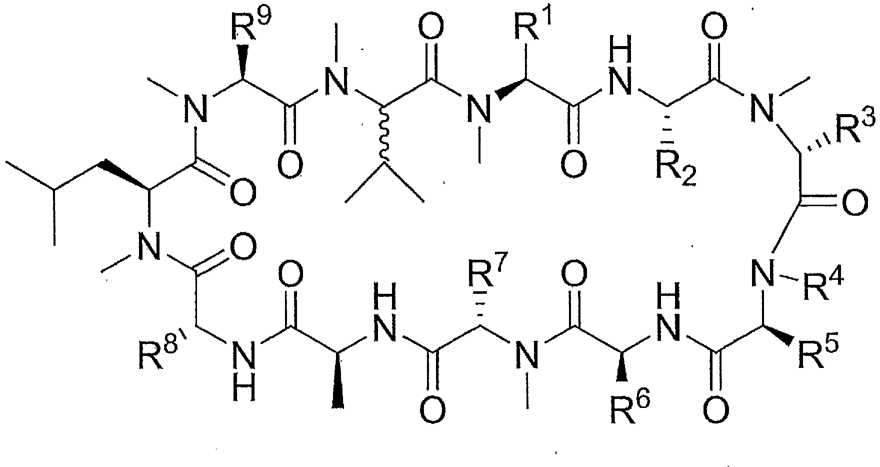 wherein
R¹ is a C1-10 alkyl group or C2-10 alkenyl group substituted by a hydroxyl group; or a group represented by the following formula, 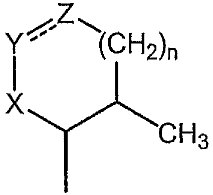 wherein
   X is O or NR¹¹
      R¹¹ is H or a C1-6 alkyl group,
   Y is CR¹²R¹³, CR¹⁴ or >C=O,
      R¹² is H, a C1-6 alkyl group, or an OH group,
      R¹³ is H, a C1-6 alkyl group, or an OH group,
      R¹⁴ is H or a C1-6 alkyl group,
   Z is (CH₂)ₘ, CR¹⁵, NR¹⁶, or O,
      R¹⁵ is H or a C1-6 alkyl group,
      R¹⁶ is H or a C1-6 alkyl group,
      m is an integer of 1 - 3,
   n is 0 or 1, and
   a bond between Y and Z shown by a solid line and a broken line is a single bond or a double bond, when the bond between Y and Z is a single bond, Y is CR¹²R¹³ or C=O, and Z is (CH₂)ₘ, NR¹⁶, or O; when the bond between Y and Z is a double bond, Y is CR¹⁴, and Z is CR¹⁵,
R² is a C1-4 alkyl group optionally substituted by a hydroxyl group,
R³ is a hydrogen atom; a C1-6 alkyl group, a C1-6 alkoxy group, or a C1-6 alkylthio group, each optionally substituted by a hydroxyl group and/or a halogen atom; or a group selected from the following formulas: 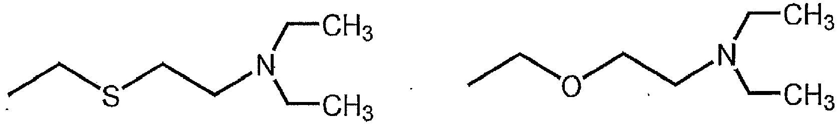 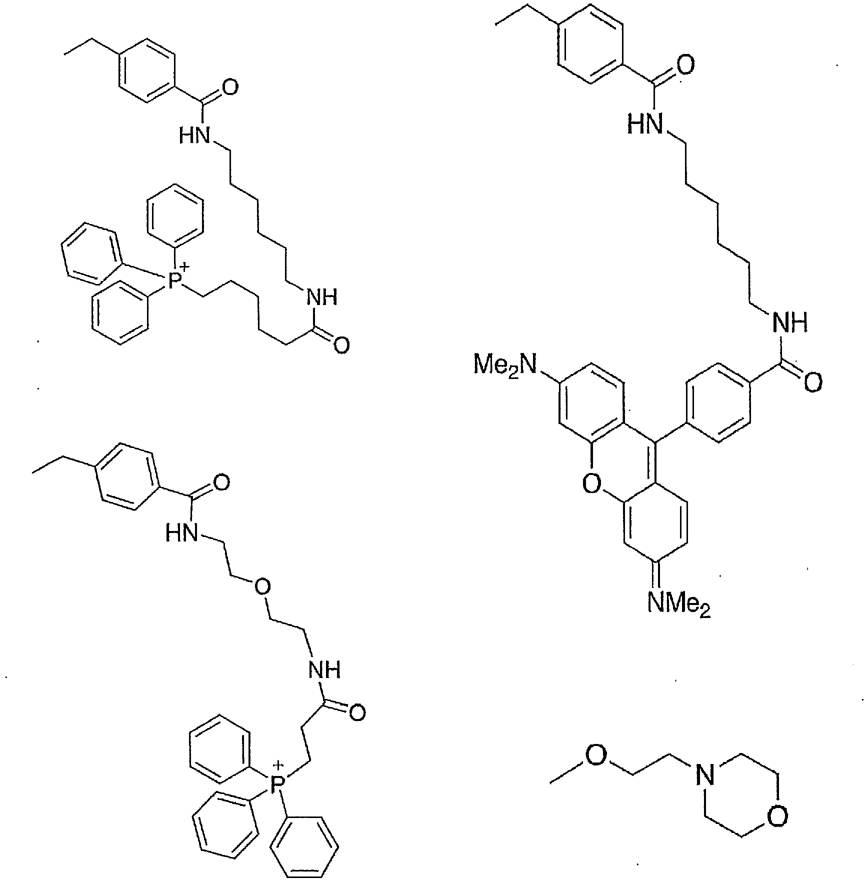
R⁴ is a C1-4 alkyl group,
R⁵ is a C1-6 alkyl group optionally substituted by a hydroxyl group and/or a C1-6 alkoxy group, or a group represented by the following: 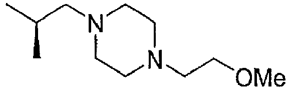
R⁶ is an n-propyl group or an isopropyl group,
R⁷ is a C1-4 alkyl group,
R⁸ is a C1-4 alkyl group optionally substituted by a hydroxyl group,
R⁹ is a branched C3-4 alkyl group, and
a bond shown by a wavy line means that a steric structure of a chiral carbon atom to be bonded is an R form, an S form, or a mixture thereof.

In the above, the "C1-10 alkyl group" means a straight chain or branched saturated hydrocarbon group having 1-10 carbon atoms, the "C1-6 alkyl group" means a straight chain or branched saturated hydrocarbon group having 1-6 carbon atoms, and the "C1-4 alkyl group" means a straight chain or branched saturated hydrocarbon group having 1-4 carbon atoms. As the alkyl group, for example, methyl group, ethyl group, n-propyl group, i-propyl group, n-butyl group, sec-butyl group, tert-butyl group, isobutyl group, pentyl group, isopentyl group, 2,3-dimethylpropyl group, hexyl group, 2-methylpentyl group, heptyl group, 2-methylhexyl group, octyl group, 2-methylheptyl group, nonyl group, 2-methyloctyl group, decyl group, and 2-methylnonyl group can be mentioned. The "branched C3-4 alkyl group" means isopropyl group, isobutyl group, sec-butyl group, or tert-butyl group.

The "C2-10 alkenyl group" means a monovalent group having 2-10 carbon atoms which is obtained by removing one hydrogen atom from any carbon atom of a straight chain or branched unsaturated hydrocarbon having one or more carbon-carbon double bonds. As the C2-10 alkenyl group, for example, vinyl group, propenyl group, isopropenyl group, 1-butenyl group, 2-butenyl group, 3-butenyl group, 1-methyl-1-propenyl group, 2-methyl-1-propenyl group, 1-methyl-2-propenyl group, 2-methyl-2-propenyl group, 1-methylidene-1-propane group, 1-pentenyl group, 1-pentenyl group, 3-pentenyl group, 4-pentenyl group, 1-methyl-1-butenyl group, 1-methyl-2-butenyl group, 1-methyl-3-butenyl group, 1-methylidenebutyl group, 2-methyl-1-butenyl group, 2-methyl-2-butenyl group, 2-methyl-3-butenyl group, 2-methylidenebutyl group, 3-methyl-1-butenyl group, 3-methyl-2-butenyl group, 3-methyl-3-butenyl group, 1-ethyl-1-propenyl group, 1-ethyl-2-propenyl group, 1-hexenyl group, 2-hexenyl group, 3-hexenyl group, 4-hexenyl group, 5-hexenyl group, 1-methyl-1-pentenyl group, 1-methyl-2-pentenyl group, 1-methyl-3-pentenyl group, 1-methyl-4-pentenyl group, 1-methylidenepentyl group, 2-methyl-1-pentenyl group, 2-methyl-2-pentenyl group, 2-methyl-3-pentenyl group, 2-methyl-4-pentenyl group, 2-methylidenepentyl group, 3-methyl-1-pentenyl group, 3-methyl-2-pentenyl group, 3-methyl-3-pentenyl group, 3-methyl-4-pentenyl group, 3-methylidenepentyl group, 4-methyl-1-pentenyl group, 4-methyl-2-pentenyl group, 4-methyl-3-pentenyl group, 4-methyl-4-pentenyl group, 1-ethyl-1-butenyl group, 1-ethyl-2-butenyl group, 1-ethyl-3-butenyl group, 2-ethyl-1-butenyl group, 2-ethyl-2-butenyl group, 2-ethyl-3-butenyl group, 1-(1-methylethyl)-1-propenyl group, 1-(1-methylethyl)-2-propenyl group, 1-ethyl-2-methyl-1-propenyl group, 1-ethyl-2-methyl-2-propenyl group, 4-hexenyl group, 2-methyl-4-pentenyl group, 4-heptenyl group, 2-methyl-4-hexenyl group, 4-octenyl group, 2-methyl-4-heptenyl group, 4-nonenyl group, 2-methyl-4-octenyl group, 4-decenyl group, and 2-methyl-4-nonenyl group can be mentioned.

The "Cl-6 alkoxy group" is a group bonded to the aforementioned C1-6 alkyl group via an oxygen atom ((C1-6 alkyl group)-O-group), and the alkyl group moiety may be linear or branched. The C1-6 alkoxy group means that the number of carbon atoms in the alkyl group moiety is 1 to 6. As the alkoxy group, for example, methoxy group, ethoxy group, 1-propyloxy group, 2-propyloxy group, 2-methyl-1-propyloxy group, 2-methyl-2-propyloxy group, 2,2-dimethyl-1-propyloxy group, 1-butyloxy group, 2-butyloxy group, 2-methyl-1-butyloxy group, 3-methyl-1-butyloxy group, 2-methyl-2-butyloxy group, 3-methyl-2-butyloxy group, 1-pentyloxy group, 2-pentyloxy group, 3-pentyloxy group, 2-methyl-1-pentyloxy group, 3-methyl-1-pentyloxy group, 2-methyl-2-pentyloxy group, 3-methyl-2-pentyloxy group, 1-hexyloxy group, 2-hexyloxy group, and 3-hexyloxy group can be mentioned. The C1-6 alkoxy group is preferably C1-5 alkoxy group, more preferably methoxy group, ethoxy group, n-propyloxy group, i-propyloxy group, n-butyloxy group, sec-butyloxy group, t-butyloxy group, isobutyloxy group, pentyloxy group, isopentyloxy group, or 2,3-dimethylpropyloxy group, further preferably C1-3 alkoxy group (methoxy group, ethoxy group, and propyloxy group), further more preferably methoxy group or ethoxy group.

The "C1-6 alkylthio group" refers to a group bonded to the aforementioned C1-6 alkyl group via a sulfur atom ((C1-6 alkyl group)-S-group), and the alkyl group moiety may be linear or branched. The C1-6 alkylthio group means that the number of carbon atoms in the alkyl group moiety is 1 to 6. As the alkylthio group, for example, methylthio group, ethylthio group, 1-propylthio group, 2-propylthio group, 2-methyl-1-propylthio group, 2-methyl-2-propylthio group, 2,2-dimethyl-1-propylthio group, 1-butylthio group, 2-butylthio group, 2-methyl-1-butylthio group, 3-methyl-1-butylthio group, 2-methyl-2-butylthio group, 3-methyl-2-butylthio group, 1-pentylthio group, 2-pentylthio group, 3-pentylthio group, 2-methyl-1-pentylthio group, 3-methyl-1-pentylthio group, 2-methyl-2-pentylthio group, 3-methyl-2-pentylthio group, 1-hexylthio group, 2-hexylthio group, and 3-hexylthio group can be mentioned. The C1-6 alkylthio group is preferably C1-5 alkylthio group, more preferably methylthio group, ethylthio group, n-propylthio group, i-propylthio group, n-butylthio group, sec-butylthio group, t-butylthio group, isobutylthio group, pentylthio group, isopentylthio group, or 2,3-dimethylpropylthio group, further preferably C1-3 alkylthio group (methylthio group, ethylthio group, and propylthio group), further more preferably methylthio group or ethylthio group.

R¹ in the aforementioned formula is preferably 1-hydroxy-2-methyl-hexyl group, 1-hydroxy-2-methyl-4-hexenyl group, or a group represented by the following formula: 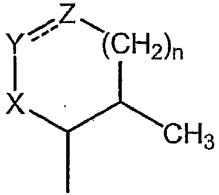 wherein
X is O or NR¹¹
   R¹¹ is H or a C1-6 alkyl group,
Y is CR¹²R¹³, CR¹⁴ or >C=O,
   R¹² is H, a C1-6 alkyl group, or an OH group,
   R¹³ is H, a C1-6 alkyl group, or an OH group,
   R¹⁴ is H or a C1-6 alkyl group,
Z is (CH₂)ₘ, CR¹⁵, NR¹⁶, or O,
   R¹⁵ is H or a C1-6 alkyl group,
   R¹⁶ is H or a C1-6 alkyl group,
   m is an integer of 1 - 3,
n is 0 or 1, and
a bond between Y and Z shown by a solid line and a broken line is a single bond or a double bond, when the bond between Y and Z is a single bond, Y is CR¹²R¹³ or C=O, and Z is (CH₂)ₘ, NR¹⁶, or O; when the bond between Y and Z is a double bond, Y is CR¹⁴, and Z is CR¹⁵.

R¹ may be, for example, a 1-hydroxy-2-methyl-hexyl group or a 1-hydroxy-2-methyl-4-hexenyl group.

R² is preferably a C1-3 alkyl group optionally substituted by a hydroxyl group, and may be, for example, a methyl group, an ethyl group, a 1-hydroxyethyl group, an isopropyl group, or an n-propyl group.

R³ is preferably a hydrogen atom or a C1-4 alkyl group optionally substituted by a hydroxyl group and/or a halogen atom, and may be, for example, a hydrogen atom or a methyl group.

R⁴ is preferably a methyl group.

R⁵ is preferably a C3-4 branched alkyl group, for example, an isobutyl group or a sec-butyl group.

R⁶ is preferably a C3 alkyl group, for example, an isopropyl group.

R⁷ is preferably a C4 alkyl group, for example, an isobutyl group.

R⁸ is preferably a methyl group or a hydroxymethyl group, more preferably a methyl group.

R⁹ is preferably a C3-4 branched alkyl group, for example, an isobutyl group or an isopropyl group.

More specifically, as the cyclophilin D inhibitor, cyclosporine A, cyclosporine D, NIM258, NIM811, Alisporivir, PKF220-384, SCY-635, mtCsA1, mtCsA2, mtCsA3, cyclosporine C, cyclosporine G, cyclosporine M, cyclosporine H, dihydrocyclosporine D, [(D)Ser]8-cyclosporine, [MeIle]8-cyclosporine, [MeAla]6-cyclosporine, [(D)Pro]3-cyclosporine, and SCY-641 can be mentioned. Among these, a substance that does not show a calcineurin inhibitory/immunity suppressive activity is preferred. It is specifically NIM258 (J. Fu et al., J Med Chem (2014) 57: 8503?16.), NIM811 (R. Traber et al., Antiviral Chemistry&Chemotherapy (1994) 5: 331-9), Alisporivir (J. Paeshuyse et al., Hepatology (2006) 43: 761-70.), PKF220-384 (Molecular pharmacology 62(1): 22-9), SCY-635 (S. Hopkins et al., Antimicrob Agents Chemother (2010) 54: 660-72.), mtCsA1, mtCsA2, mtCsA3 (the above from Biochem J. 2012; 441(Pt3): 901-7.), cyclosporine H (J Immunol 1991; 147: 1940-1946), or [MeIle]8-cyclosporine (SciFinder), more preferably, derivative compound that shares the same skeleton as Alisporivir (NIM258, NIM811, SCY-635) (Jiping Fu et al., J. Med. Chem. (2014) 57: 8503-8516), most preferably Alisporivir or NIM258.

In one embodiment, the present invention may be a composition for promoting secretion of collagen trimers in cells having a type-IV collagen gene having a mutation, wherein the composition contains a cyclophilin D inhibitor as an active ingredient.

As the cyclophilin D inhibitor, siRNA, shRNA, or miRNA against cyclophilin D, an anti-cyclophilin antibody, an aptamer against cyclophilin D, or an antisense nucleic acid molecule against cyclophilin D can also be used. The cyclophilin D inhibitor may be a peptide having the following sequence:
EFGGVMCVESVNREMSPLVD (SEQ ID NO: 53)
REMSPLVDNIALWMTEYLNR (SEQ ID NO: 54)
MCVESVNREMSPLVDNIALW (SEQ ID NO: 55)
LLSLALVGACITLGAYLGHK (SEQ ID NO: 56)

As the nucleic acid molecule such as siRNA and antisense, for example, nucleic acid molecules having the following sequences can be mentioned (left is 5'-terminus, right is 3'terminus):
GGCAGAUGUCGUCCCAAAG (SEQ ID NO: 27)
CUUUGGGACGACAUCUGCC (SEQ ID NO: 28)
GTCCTCCCACTCTTAGAGCC (SEQ ID NO: 57)
GTCCTCCCACTCTTAGAGCC (SEQ ID NO: 58)
CTTCCCGCCTGTGCCATTGT (SEQ ID NO: 59)
GATGTCCTCCCACTCTTAGA (SEQ ID NO: 60)
TGTCCTCCCACTCTTAGAGCC (SEQ ID NO: 61)
GGAGGACAUCCAAGAAGAUUGUCAT (SEQ ID NO: 62)
AUGACAAUCUUCUUGGAUGUCCUCCCA (SEQ ID NO: 63)
CCCAAAGACAGCUGAGAACUUCAGA (SEQ ID NO: 64)
UCUGAAGUUCUCAGCUGUCUUUGGGAC (SEQ ID NO: 65)
GCUCCACCUUCCACAGGGUGAUCCC (SEQ ID NO: 66)
GGGAUCACCCUGUGGAAGGUGGAGCCU (SEQ ID NO: 67)
CAGACUGGUUGGAUGGCAAGCAUG (SEQ ID NO: 68)
ACAUGCUUGCCAUCCAACCAGUCUGUC (SEQ ID NO: 69)
GGCUAAUGCUGGUCCUAACACCAAC (SEQ ID NO: 70)
GUUGGUGUUAGGACCAGCAUUAGCCAU (SEQ ID NO: 71)

These cyclophilin D inhibitors can be obtained, for example, by the methods described in WO2009/018179; WO2012/103520; US9,132,138B; WO2014/093632; JP2016-124821A; JP2017-51349OA; WO2015/200725; US9,738,615B; WO2016/112321; US10,179,161B; WO2019/173382.

The cyclophilin D inhibitor is a compound represented by the aforementioned formula, or the like. In the present specification, the cyclophilin D inhibitor may be in the form of, where necessary, a pharmacologically acceptable salt and/or hydrate or solvate thereof. In the present specification, the "pharmacologically acceptable salt" is a salt formed by combining the compound of the present invention with an inorganic or organic acid, and is acceptable for administration to the body as a medicament. Such salt is described in, for example, Berge et al., J. Pharm. Sci. 66: 1-19 (1977), and the like. As the salt, salts with mineral acids such as hydrochloric acid, hydrobromic acid, sulfuric acid, nitric acid, phosphoric acid, and the like; salts with organic acids such as methanesulfonic acid, benzenesulfonic acid, p-toluenesulfonic acid, acetic acid, propionic acid, tartaric acid, fumaric acid, maleic acid, malic acid, oxalic acid, succinic acid, citric acid, benzoic acid, mandelic acid, cinnamic acid, lactic acid, glycolic acid, glucuronic acid, ascorbic acid, nicotinic acid, salicylic acid, and the like; and salts with acidic amino acids such as aspartic acid, glutamic acid, and the like; can be mentioned. A hydrate or solvate of a cyclophilin D inhibitor, and a hydrate or solvate of a pharmacologically acceptable salt of a cyclophilin D inhibitor may also be encompassed in the cyclophilin D inhibitor. In the present specification, unless it is clearly unsuitable, the "cyclophilin D inhibitor" also includes, even if it is not explicitly indicated, a pharmacologically acceptable salt, a hydrate and a solvate of a cyclophilin D inhibitor, as well as a hydrate or solvate of a pharmacologically acceptable salt of a cyclophilin D inhibitor.

Type-IV collagen is a major constituent component of the basement membrane, and includes 6 types (α1, 2, 3, 4, 5, and 6) (SEQ ID NO: 2, 4, 6, 8, 10, and 12, respectively), each of which is encoded by the corresponding gene (COL4A1, 2, 3, 4, 5, and 6) (SEQ ID NO: 1, 3, 5, 7, 9, and 11, respectively). In collagen, three α chains are associated to form a triple helix structure. In the glomerular basement membrane (GBM) of the kidney, α3, α4, and α5 are mainly expressed, and mutations in these are considered to be often involved particularly in the onset of AS. In the present specification, the "collagen" optionally means a type-IV collagen. In the present specification, moreover, the "type-IV collagen" optionally means a type-IV collagen having a mutation. In the present specification, the type-IV collagen gene having a mutation is preferably a type-IV collagen gene (COL4A3, COL4A4, or COL4A5), most preferably COL4A5.

In the present specification, the "mutation" means, unless inconsistent to understand as such, a mutation due to substitution, deletion, and/or insertion of an amino acid, preferably substitution. In one embodiment, the "mutation" of collagen is a mutation that reduces the amount of collagen trimer secreted, or a mutation that results in AS.

The mutation in the type-IV collagen gene is not particularly limited as long as it is a mutation that contributes to the formation and secretion of collagen trimers, and is preferably a mutation that reduces the secretion of collagen trimers. For example, mutation selected from G129E, G227S, G230C, G325R, G426R, G475S, G521D, G573D, G594D, G594S, G624D, G650D, L664N, G675S, G796R, G869R, G911E, S916G, G953V, G1030S, G1107R, G1140V, G1143D, G1220D, G1241C, G1241V, G1244D, G1448R, P1517T, C1567R, R1569Q, M1607I, L1649R, and R1683Q in COL4A5 (preferably, G1241V and/or G1244D), or amino acid mutation in Exon41 can be mentioned. In the present specification, AS having such mutation is preferred.

"Promoting the secretion of collagen trimer" means that the extracellular secretion level of collagen consequently increases as compared with the case of non-administration of the drug. It may include any or two or three kinds of actions from promotion of collagen trimer secretion itself, increase of collagen gene production, and promotion of collagen trimer formation. The composition of the present invention enables a radical treatment of AS caused by insufficient secretion levels of collagen trimer by promoting the secretion of collagen trimer.

The composition of the present invention can be a therapeutic or prophylactic agent for a disease or condition, represented by AS, which is based on the deficiency in the formation and/or secretion of collagen trimers. In the present specification, AS may be read as appropriate as a disease or condition based on the deficiency in the formation and/or secretion of collagen trimers. AS is typically a hereditary disease, and representative clinical symptoms thereof include renal lesions such as glomerulonephritis, chronic nephritis, hematuria (particularly, persistent hematuria), proteinuria, wide range of irregular thickening of the glomerular basement membrane, and changes in the reticulation of dense layer of the glomerular basement membrane; ear lesions such as deafness, particularly, sensorineural (neurotic) deafness, hearing loss and progressive exacerbation in high frequency region; eye lesions such as anterior lenticonus, posterior subcapsular cataract, posterior polymorphous dystrophy, and blind spot retina; complications of diffuse leiomyomatosis; and the like.

The patients to be treated/prevented with the therapeutic or prophylactic drug of the present invention may have an amino acid mutation G1030S, G1107R of COL4A5, or in the region of Exon41, where the mutation in the region of Exon41 may be G1220D, G1241C, G1241V, or G1244D.

Therefore, in another embodiment, the present invention relates to the above-mentioned composition (medical composition) which is a therapeutic drug, a prophylactic drug, or an improving agent for AS. The medical composition of the present invention can be formulated as a medical composition for oral or parenteral administration, for example, tablet, powder, granule, capsule, internal liquid, syrup, ointment, lotion, or injection (e.g., injection for intravenous injection, injection for subcutaneous administration, injection for muscle injection, drip). In the present specification, the medical composition can be prepared according to a conventional method.

The medical composition may contain a pharmacologically acceptable carrier (additive for preparation). The type of preparation additive used in the production of the medical composition, the ratio of the preparation additive to the active ingredient, or the method for producing the medical composition can be appropriately selected by those skilled in the art according to the form of the composition. As the preparation additive, an inorganic or organic substance, or a solid or liquid substance can be used, and generally, it can be added in a proportion of 1 wt% to 90 wt% based on the weight of the active ingredient.

The medical composition may be further administered with other medicaments. The medical composition of the present invention and other medicament may be contained in a single preparation or may be administered together as separate preparations. As such other medicament, a medicament that does not inhibit the therapeutic effect of a cyclophilin D inhibitor on AS, but is known to have a therapeutic effect on AS, or a medicament that enhances the therapeutic effect of a cyclophilin D inhibitor can be mentioned. As the medicament that enhances the therapeutic effect of a cyclophilin D inhibitor, rennin-angiotensin-aldosterone system inhibitors: angiotensin converting enzyme inhibitors (e.g., enalapril, ramipril, benazepril, and the like), angiotensin II receptor antagonists (e.g., valsartan, losartan, candesartan, and the like); statin (e.g., fluvastatin and the like); aldosteron antagonists (e.g., spironolactone and the like); activated vitamin D2 analogues (e.g., paricalcitol and the like); and the like can be mentioned.

The present invention relates to a method for treating, preventing, or improving AS, including administering an effective amount of a cyclophilin D inhibitor to a patient in need thereof. The present invention relates to a method for improving deficiency in forming and/or secreting collagen trimers in a patient, or an agent for promoting formation and/or secretion of collagen trimers in a patient, each including administering an effective amount of a cyclophilin D inhibitor to a patient in need thereof. The "patient in need thereof" is specifically a patient affected with AS. The patient is preferably a patient having an amino acid mutation G1030S, G1107R, or in the region of Exon41 in COL4A5, where the mutation in the region of Exon41 may be G1220D, G1241C, G1241V, or G1244D. For example, the therapeutic or prophylactic method of the present invention may include a step of examining whether the patient to be the subject of treatment or prophylaxis has such a mutation. Specifically, the present invention may be directed to a method for the treatment or prophylaxis of AS, including examining a gene mutation in COL4A5 of a patient, and when the aforementioned gene mutation is an amino acid mutation G1030S, G1107R, or in the region of Exon41, administering an effective amount of the composition of the present invention to the patient. In this method, the amino acid mutation in the region of Exon41 may be G1220D, G1241C, G1241V, or G1244D. The "effective amount" means an amount that can improve a symptom or condition to be treated, prevented, or improved. As the symptom or condition to be treated, prevented, or improved, for example, disappearance of α5(IV) expression on the basement membrane; widespread expression of α2(IV) in GBM; increased ectopic expression of laminin; proteinurina; leakage of urinary albumin; elevated serum creatinine levels; and other progressive renal conditions can be mentioned.

In the present specification, the patient to be the subject of treatment, prophylaxis, or improvement includes mammals such as human, cow, horse, dog, cat, pig, sheep and the like, and is preferably human.

The medical composition of the present invention can be administered in vivo, ex vivo, or in vitro.

Furthermore, the present invention relates to the use of a cyclophilin D inhibitor for the production of a therapeutic, prophylactic, or improving drug for AS. The present invention relates to the use of a cyclophilin D inhibitor for the production of a medicament that improves deficiency in the formation and/or secretion of collagen trimers. The present invention relates to the use of a cyclophilin D inhibitor for the production of an agent for promoting formation and/or secretion of collagen trimers. Also, the present invention relates to a cyclophilin D inhibitor for the treatment, prophylaxis, or improvement of AS. The present invention relates to a cyclophilin D inhibitor for improving deficiency in the formation and/or secretion of collagen trimers. The present invention relates to a cyclophilin D inhibitor for promoting deficiency in the formation and secretion of collagen trimers. For example, when a cyclophilin D inhibitor is used for the purpose of treatment, prophylaxis, or improvement, a medical composition containing the cyclophilin D inhibitor as an active ingredient can be administered in an oral administration form or a parenteral administration form, such as injection, drip transfusion and the like. When a cyclophilin D inhibitor is administered to a mammal or the like, the aforementioned preparation may be orally administered, or may be administered parenterally as an injection or a drip transfusion. The dose varies depending on symptoms, age, gender, body weight, administration form, and the like. For example, when orally administered to an adult, the daily dose is generally 0.1-1000 mg.

In another embodiment, the present invention relates to a non-human model animal having a G1220D mutation in the COL4A5 gene, or a non-human model animal in which Exon41 in the COL4A5 gene is deleted or disrupted. The deletion of Exon 41 is not necessarily in the entire region and may be a partial deletion of Exon 41 as long as it exhibits symptoms based on the deficiency in the formation and/or secretion of collagen trimers. Alternatively, the gene in Exon 41 may be disrupted. For example, a non-human model animal may be characterized by having one or more of the following phenotypes selected from the following, as compared with the corresponding wild type animal: disappearance of α5(IV) expression on the basement membrane; widespread expression of α2(IV) in GBM; increased ectopic expression of laminin; proteinurina; leakage of urinary albumin; elevated serum creatinine levels; and other progressive renal conditions. The symptoms based on the deficiency in the formation and/or secretion of collagen trimers may be of the phenotypes listed above.

Alternatively, in another embodiment, the present invention may be directed to a nucleic acid construct in which Exon 41 is deleted or disrupted. The construct can be a nucleic acid construct for producing a model animal of the present invention or an agent for generating a model animal of the present invention.

The non-human model animal does not need to be an adult and may be its cell, tissue, or embryo. The non-human model animal may be a mouse, rat, guinea pig, hamster, rabbit, dog, pig, sheep, cow, or monkey, and is preferably a mouse.

The present invention includes an evaluation method or a screening method for the therapeutic effect of the aforementioned drug on AS using the non-human model animal. Specifically, this method may be a method including administering a test drug to the aforementioned non-human animal model, observing or measuring symptoms or conditions based on the deficiency in the formation and/or secretion of collagen trimers, or levels (disappearance of α5(IV) expression on the basement membrane; widespread expression of α2(IV) in GBM; increased ectopic expression of laminin; proteinurina; leakage of urinary albumin; elevated serum creatinine levels; and other progressive renal conditions) to be the indices thereof in the aforementioned non-human model animal, and, when the administration of the test drug improves the symptoms or conditions based on the deficiency in the formation and/or secretion of collagen trimers, or levels to be the indices thereof, determining that the test drug may have a therapeutic effect on AS. That it "improves the symptoms or conditions based on the deficiency in the formation and/or secretion of collagen trimers, or levels to be the indices thereof" means that the condition becomes closer to that in the wild type than in the model animal, and does not have to mean that the symptom or condition is completely eliminated.

The present invention is explained in more detail in the following by referring to Examples which do not limit the scope of the present invention. Documents cited throughout the present specification are incorporated in full in the present specification by reference.

### [Example]

### (Example 1) Synthesis of NIM258

### (1) Synthesis of tert-butyl(furan-2-yl(tosyl)methyl)carbamate (4)

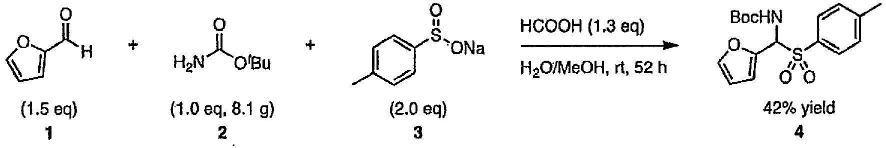

To a suspension prepared from carbamate (2) (8.13 g, 69.4 mmol) and sulfinic acid 3 (35.2 g, 140 mmol, 2.0 eq), and a mixed solvent (2/1, 168 mL) of methanol and water were added aldehyde 1 (8.65 mL, 104.1 mmol, 1.5 eq) and formic acid (3.4 mL, 90.2 mmol, 1.3 eq). The mixture was stirred at room temperature for 52 hr, and a precipitated white solid was collected by filtration. The solid was washed with water and diethyl ether, and dried by heating under reduced pressure to give carbamate (4) (10.2 g, 42% yield).

### (2) Synthesis of tert-butyl tert-butyl((1S,2S)-1-(furan-2-yl)-2-methyl-3-oxopropyl)carbamate (7)

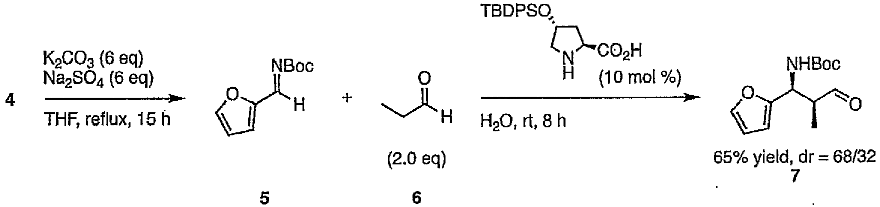

Anhydrous sodium sulfate (53.0 g) and calcium carbonate (15.4 g) were measured into a two-neck flask, and dried under reduced pressure with a heat gun. After cooling to room temperature, anhydrous THF (100 mL) was added, to the suspension was added carbamate 4 (6.6 g, 18.6 mmol), and the mixture was heated under reflux for 15 hr. After cooling to room temperature, the suspension was passed through celite to remove solids, and the filtrate was evaporated under reduced pressure to give imine 5 (3.9 g) as a yellow liquid. The obtained product was used for the next step without further purification.

Under argon atmosphere and at room temperature, water (11 mL) was added to imine 5 (3.6 g, 18.6 mmol), aldehyde 6 (2.16 g, 37.2 mmol, 2.0 eq) and (2S,4R)-4-((tertbutyldiphenylsilyl)oxy)pyrrolidine-2-carboxylic acid (689.7 mg, 1.86 mmol, 10 mol%) were added, and the reaction solution was stirred for 8 hr. The reaction solution was diluted with water, and the aqueous layer was extracted three times with ethyl acetate (30 mL). The combined organic layer was washed with saturated brine, and dried over anhydrous sodium sulfate. The solvent was evaporated under reduced pressure, and the obtained crude product was purified by silica gel column chromatography (hexane/ethyl acetate=12/1) to give the desired aldehyde 7 (3.07 g, 65% yield, dr 68/32) as a diastereomer mixture.

### (3) Synthesis of tert-butyl((1S,2S)-1-(furan-2-yl)-3-hydroxy-2-methylpropyl)carbamate (8)

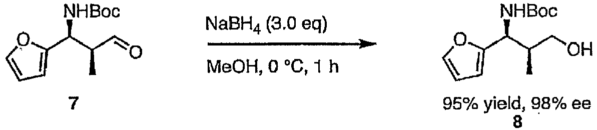

To a methanol solution (40 mL) of aldehyde 7 (3.07 g, 12.1 mmol) was slowly added under ice-cooling sodium borohydride (1.38 g, 36.4 mmol, 3.0 eq). After stirring for 1 hr, a saturated ammonium chloride aqueous solution was added to discontinue the reaction. The reaction solution was extracted three times with ethyl acetate. The combined organic layer was washed with saturated brine, and dried over anhydrous sodium sulfate. The solvent was evaporated and the obtained crude product was purified by silica gel column chromatography (hexane/ethyl acetate=7/1) to give alcohol 8 (2.96 g, 95% yield).

### (4) Synthesis of tert-butyl((1S,2S)-3-((tertbutyldiphenylsilyl)oxy)-1-(furan-2-yl)-2-methylpropyl)carbamate (9)

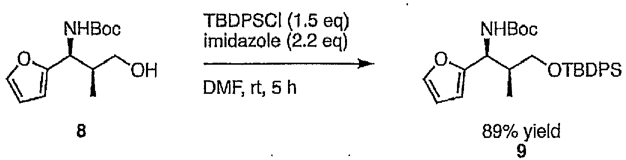

To N,N-dimethylformamide solution (45 mL) in which alcohol 8 (2.96 g, 11.6 mmol) was dissolved were successively added, under argon atmosphere and at room temperature, tertbutyldiphenylsilyl chloride (4.7 mL, 17.1 mmol, 1.5 eq) and imidazole (1.75 g, 25.5 mmol, 2.2 eq), and the mixture was stirred at room temperature for 5 hr. The reaction solution was ice-cooled, and 1N aqueous hydrochloric acid solution (15 mL) was slowly added to discontinue the reaction. The mixture was heated to room temperature, further stirred for 30 min, and extracted three times with ethyl acetate/hexane mixed solution (2/3, 30 mL). The combined organic layer was washed with saturated aqueous sodium hydrogen carbonate and saturated brine in this order, dried over anhydrous sodium sulfate, and the solvent was evaporated under reduced pressure. The obtained crude product was purified by silica gel column chromatography (hexane/ethyl acetate=19/1) to give carbamate 9 (5.11 g 89% yield) as a diastereomer mixture.

### (5) Synthesis of tert-butyl((1S,2S)-3-((tertbutyldiphenylsilyl)oxy)-1-(furan-2-yl)-2-methylpropyl)(methyl)carbamate (10)

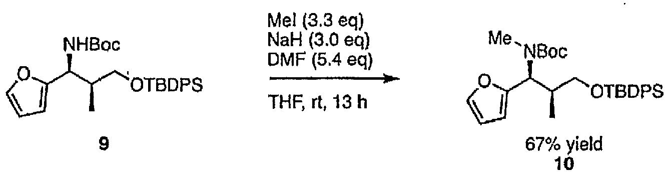

Under an argon atmosphere, anhydrous THF (30 mL) and sodium hydride (60%, 1.24 g, 31.0 mmol) were successively added to a 200 mL eggplant flask. After cooling to 0°C, to the reaction mixture was added a THF solution (10 mL) of carbamate 9 (5.11 g, 10.3 mmol) with a cannula. After stirring at 0°C for 15 min, methyl iodide (2.13 mL, 34.2 mmol, 3.3 eq) and N,N-dimethylformamide (4.33 mL, 55.9 mmol, 5.4 eq) were successively added. The reaction solution was returned to room temperature and stirred for 13 hr. A saturated ammonium chloride aqueous solution was added to discontinue the reaction, and the aqueous layer was extracted three times with ethyl acetate (20 mL). The combined organic layer was washed with saturated brine, dried over anhydrous sodium sulfate, and the solvent was evaporated under reduced pressure to give a crude product. This was purified by silica gel column chromatography (hexane/ether=12/1) to give carbamate 10 (3.53 g, 67% yield).

### (6) Synthesis of (1S,2S)-3-((tert-butyldiphenylsilyl)oxy)-1-(furan-2-yl)-N,2-dimethylpropane-1-amine (11)

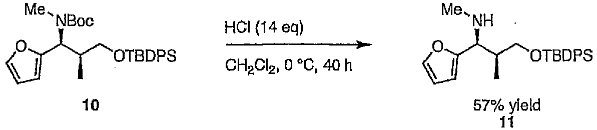

Under an argon atmosphere and at 0°C, to a dichloromethane solution (20 mL) of carbamate 10 (2.91 g, 5.73 mmol) was added hydrogen chloride/dioxane solution (4.0 M, 20 mL), and the mixture was stirred for 45 hr. Saturated aqueous sodium hydrogen carbonate (20 mL) was added to discontinue the reaction, and the aqueous layer was extracted 6 times with dichloromethane (15 mL). The combined organic layer was washed with saturated brine, dried over anhydrous sodium sulfate, and the solvent was evaporated under reduced pressure to give a crude product. This was purified by silica gel column chromatography (hexane/ethyl acetate=5/1 - 4/1) to give amine 11 (1.33 g, 57% yield).

### (7) Synthesis of tert-butyl(R)-1-((((1S,2S)-3-((tertbutyldiphenylsilyl)oxy)-1-(furan-2-yl)-2-methylpropyl)(methyl)amino)-1-oxopropan-2-yl)(methyl)carbamate (13)

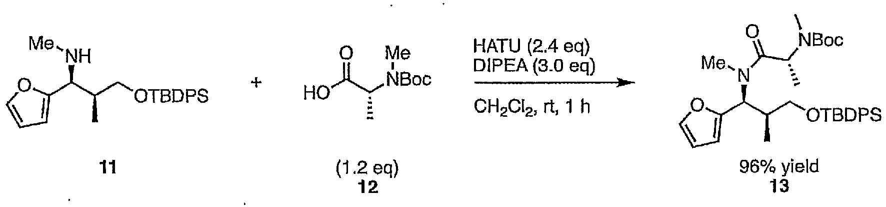

Under an argon atmosphere, amine 11 (781 mg, 1.92 mmol) and carboxylic acid 12 (469 mg, 2.30 mmol) were dissolved in anhydrous dichloromethane (18 mL). After cooling to 0°C, HATU (1.75 g, 4.60 mmol) and N,N-diisopropylethylamine (1.0 mL, 5.75 mmol) were added. The mixture was heated to room temperature, and further stirred for 1 hr. The solution was diluted with ethyl acetate (20 mL) and water (5 mL), and 1N hydrochloric acid (10 mL) was added to discontinue the reaction. The aqueous layer was extracted three times with ethyl acetate (15 mL), and the combined organic layer was washed with saturated aqueous sodium hydrogen carbonate and saturated brine, and dried over anhydrous sodium sulfate. The solvent was evaporated under reduced pressure and the obtained crude product was purified by silica gel column chromatography (hexane/ethyl acetate=9/1 - 8/1) to give amide 13 (1.09 g, 96% yield).

### (8) Synthesis of (2S, 3S)-2-((R)-2-((tertbutoxycarbonyl)(methyl)amino)-N-methylpropanamide)-4-((tertbutyldiphenylsilyl)oxy)-3-methylbutanic acid (14)

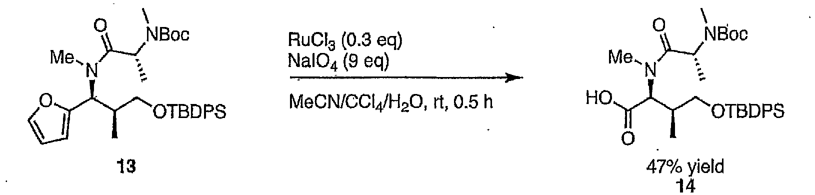

To an acetonitrile/carbon tetrachloride/water suspension (22 mL/10 mL/22 mL) of sodium periodate (3.50 g, 16.4 mmol, 9.0 eq) was added chloride ruthenium (III) (113.5 mg, 0.55 mmol) with vigorous stirring, and the mixture was further stirred for 15 min. To the reaction mixture was added a carbon tetrachloride solution (5 mL) of amide 13 (1.08 g, 1.82 mmol) with a cannula. After stirring for 30 min, water and 10% sodium thiosulfate aqueous solution were added to discontinue the reaction. The aqueous layer was extracted four times with ethyl acetate (30 mL), and the combined organic layer was washed with saturated brine and dried over anhydrous sodium sulfate. The solvent was evaporated and the obtained crude product was purified by silica gel column chromatography (hexane/ethyl acetate) to give carboxylic acid 14 (533.4 mg, purity 90%). The obtained desired product was used for the next step without further purification.

### (9) Synthesis of benzyl(2S,3S)-2-((R)-2-((tertbutoxycarbonyl)(methyl)amino)-N-methylpropanamide)-4-((tertbutyldiphenylsilyl)oxy)-3-methylbutanoate (15)

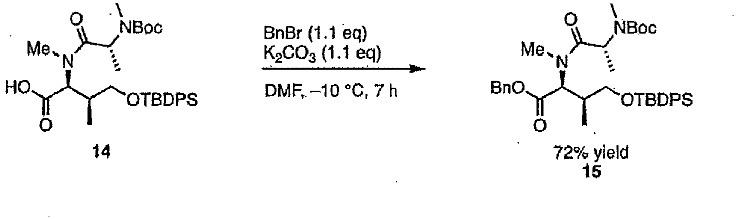

Under an argon atmosphere and at -10°C, to an N,N-dimethylformamide solution (10 mL) of carboxylic acid 14 (528.9 mg, 90% purity, 0.93 mmol) were slowly added potassium carbonate (141.1 mg, 1.02 mmol) and benzyl bromide (0.12 ml, 1.02 mmol). After 7 hr, water was added to discontinue the reaction, and the aqueous layer was extracted three times with ethyl acetate (20 mL). The combined organic layer was washed with saturated brine, dried over anhydrous sodium sulfate, and the solvent was evaporated. The obtained crude product was purified by silica gel column chromatography (hexane/dichloromethane=1/2) to give benzyl ester 15 (442.1 mg, 72% yield).

### (10) Synthesis of benzyl(2S,3S)-2-((R)-2-((tertbutoxycarbonyl)(methyl)amino)-N-methylpropanamide)-4-hydroxy-3-methylbutanoate (16)

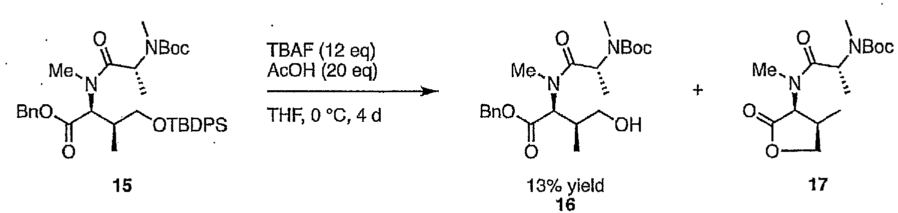

Under an argon atmosphere and at 0°C, to a THF solution (25 mL) of ester 15 (304.8 mg, 0.46 mmol) was added acetic acid (0.53 mL, 9.2 mmol, 20 eq). To this solution was added tetrabutylammonium fluoride (5.5 mL, 1.0 M in THF, 12 eq), and the mixture was stirred for 4 days. The mixture was diluted with ethyl acetate and saturated brine, and the aqueous layer was extracted four times with ethyl acetate (15 mL). The combined organic layer was washed with saturated brine, dried over anhydrous sodium sulfate, and the solvent was evaporated under reduced pressure to give a crude product. The obtained compound was purified by silica gel column chromatography (hexane/ethyl acetate = 5/1 - 1/1) to give alcohol 16 (48.5 mg, purity 50%, containing lactone 17, 13% yield).

(11) Synthesis of benzyl(2S,3S)-2-((R)-2-((tertbutoxycarbonyl)(methyl)amino)-N-methylpropanamide)-3-methyl-4-oxobutanoic acid (18).

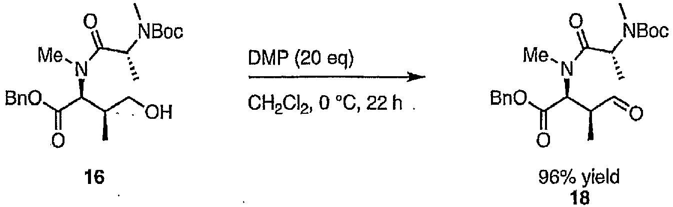

Under an argon atmosphere and at 0°C, to a dichloromethane solution (10 mL) of alcohol 16 (48.5 mg, 50% purity, 0.115 mmol) was added a Dess-Martin reagent (1.01 g, 2.3 mmol), and the mixture was stirred for 22 hr. A 10% sodium thiosulfate aqueous solution (8 mL) and saturated aqueous sodium hydrogen carbonate (8 mL) were added to discontinue the reaction, and the mixture was diluted with ethyl acetate (20 mL). After stirring for 20 min, the aqueous layer was extracted three times with ethyl acetate (10 mL), and the combined organic layer was washed with saturated brine, and dried over anhydrous sodium sulfate. The solvent was evaporated and the obtained crude product was purified by silica gel column chromatography (hexane/ethyl acetate=4/1) to give aldehyde 18 (23.3 mg, 96% yield).

### (12) Synthesis of benzyl(2S,3R)-2-((R)-2-((tertbutoxycarbonyl)(methyl)amino)-N-methylpropanamide)-4-(4-(2-methoxyethyl)piperazin-1-yl)-3-methylbutanoate (19)

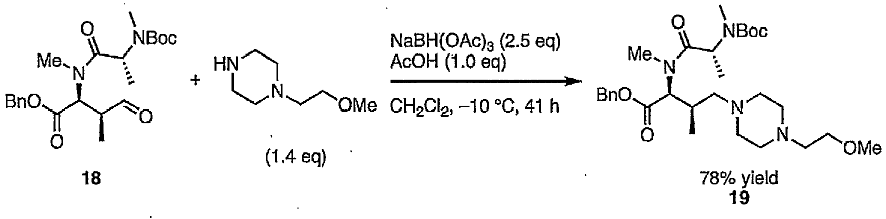

Under an argon atmosphere and at -20°C, to a dichloromethane solution (2 mL) of aldehyde 18 (23.6 mg, 0.056 mmol) were successively added acetic acid (3.5 mg, 0.056 mmol) and 1-(2-methoxyethyl)piperazine (11.4 mg, 0.079 mmol). To this solution was slowly added sodium triacetoxyborohydride (29.9 mg, 0.14 mmol) and the mixture was stirred at -10°C for 41 hr. The reaction was discontinued with saturated ammonium chloride aqueous solution, and the aqueous layer was extracted 5 times with ethyl acetate (10 mL). The combined organic layer was washed with saturated brine, dried over anhydrous sodium sulfate, and the solvent was evaporated under reduced pressure to give a crude product. This was purified by silica gel column chromatography (2 - 7% methanol/dichloromethane) to give amine 19 (24.0 mg, 78% yield).

### (13) Synthesis of (2S,3R)-2-((R)-2-((tertbutoxycarbonyl)(methyl)amino)-N-methylpropanamide)-4-(4-(2-methoxyethyl)piperazin-1-yl)-3-methylbutanoic acid

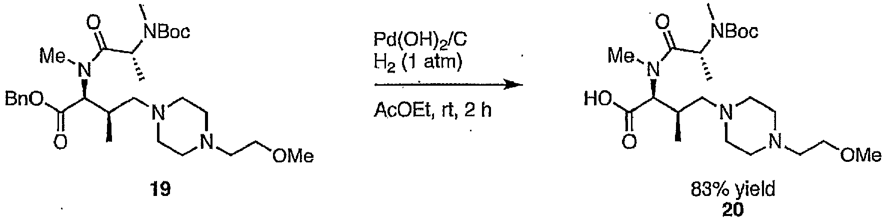

To an ethyl acetate solution (5 mL) of amine 19 (30.8 mg, 0.056 mmol) was added palladium hydroxide (10 wt%, 1.3 mg), and the mixture was stirred under 1 atm hydrogen atmosphere for 2 hr. The reaction solution was passed through celite, the solid was filtered off, and the solvent was evaporated under reduced pressure to give a crude product. This was purified by silica gel column chromatography (7 - 10% methanol/dichloromethane) to give carboxylic acid 20 (21.4 mg, 83% yield).

### (14) Synthesis of acetyl-CsA (22)

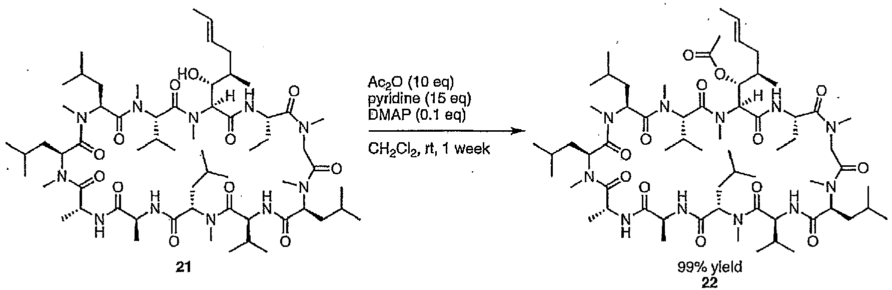

Under an argon atmosphere, cyclosporine A (21) (4.0 g, 3.33 mmol) was dissolved in dichloromethane (24 mL), acetic anhydride (3.2 mL, 33.3 mmol) and pyridine (4.0 mL, 50.0 mmol), and DMAP (40.6 mg, 0.33 mmol, 10 mol%) were successively added. The mixture was heated to room temperature and further stirred for 1 week. Saturated aqueous sodium hydrogen carbonate (100 mL) was slowly poured into the reaction solution. After stirring for 1 hr, the aqueous layer was extracted three times with dichloromethane (20 mL). The combined organic layer was washed two times with 1N hydrogen chloride aqueous solution (48 mL), and with saturated aqueous sodium hydrogen carbonate (48 mL) and saturated brine, and dried over anhydrous sodium sulfate. The solvent was evaporated under reduced pressure to give a crude product (4.1 g). The obtained product was used for the next step without further purification.

### (15) Synthesis of compound 23

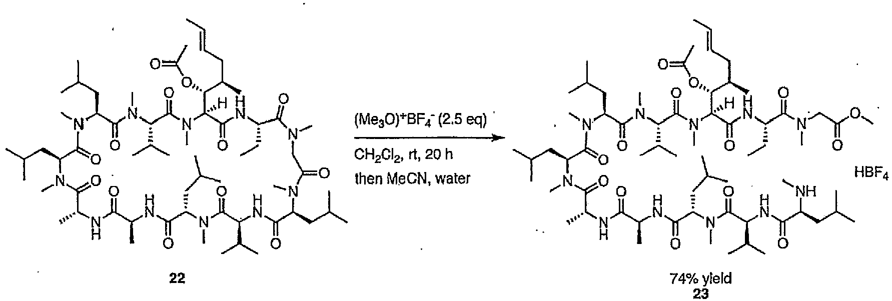

Under an argon atmosphere, to a dichloromethane solution (4 mL) of acetylated compound 22 (2.16 g, 1.74 mmol) was added trioxonium tetrafluoroborate (642.6 mg, 4.34 mmol), and the mixture was stirred at room temperature for 20 hr. To this reaction solution were added acetonitrile (4 mL) and water (13.5 mL), and the mixture was further stirred for 48 hr. The organic layer was extracted and dried over anhydrous sodium sulfate. The solvent was evaporated under reduced pressure to give a crude product. The obtained compound was purified by silica gel flash column chromatography (1 - 9% methanol/dichloromethane) to give ring-opened compound 23 (1.75 g, 74% yield).

### (16) Synthesis of compound 24

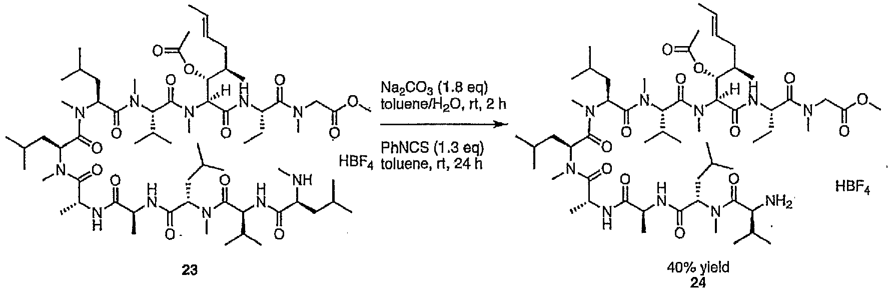

To a mixture of ring-opened compound 23 (1.74 g, 1.28 mmol) in toluene (4.2 mL) and water (2.5 mL) was added sodium carbonate (243 mg, 2.3 mmol) at room temperature. 2-Methyltetrahydrofuran (20 mL) was added to completely dissolve the solid. After stirring for 2 hr, the organic layer was separated and evaporated under reduced pressure. The obtained residue was dissolved in toluene (4.2 mL), and phenylisothiocyanate (224 mg, 1.66 mmol) was added at room temperature. After stirring for 24 hr, methanol (2.4 mL) was added, 50% tetrafluoroboric acid (0.8 mL) was successively added, and the mixture was further stirred for 1 hr. Water (2.4 mL) was added, and the organic layer was recovered by partitioning and washed with water and saturated brine. By drying over anhydrous sodium sulfate and evaporation under reduced pressure, a crude product was obtained and purified by flash column chromatography to give amine 24 (580.6 mg, 40% yield).

### (17) Synthesis of compound 25

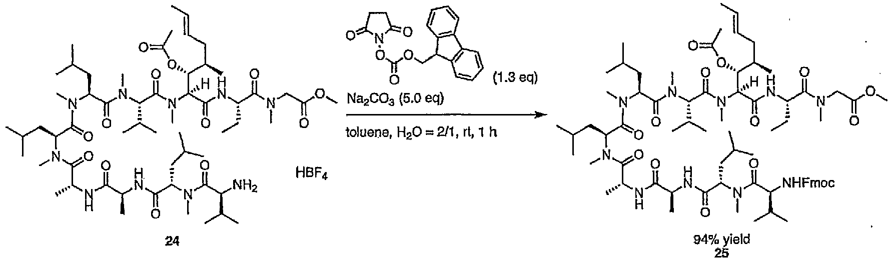

Under an argon atmosphere, to amine 24 (486 mg, 0.39 mmol) were added toluene (2.8 mL) and water (1.5 mL) to prepare a suspension. Sodium carbonate (210 mg, 1.96 mmol) and Fmocsuccinimide (174 mg, 0.51 mmol) were slowly added at room temperature. After stirring for 1 hr, methanol (0.2 mL) was added, and the mixture was further stirred at 50°C for 1 hr. The organic layer was separated, and the solvent was evaporated under reduced pressure to give a crude product. The obtained compound was purified by silica gel column chromatography (3% methanol/dichloromethane) to give carbamate 25 (508 mg, 94% yield).

### (18) Synthesis of compound 26

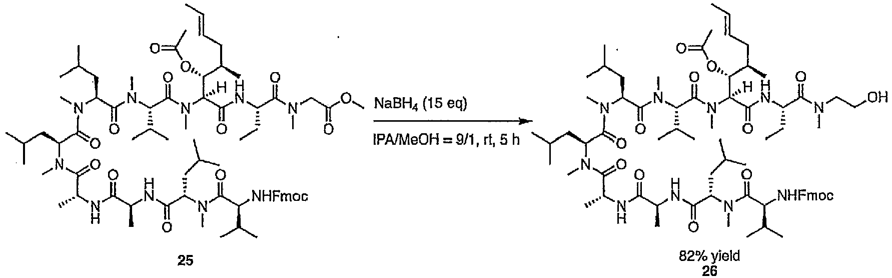

Under an argon atmosphere, to a solution of carbamate 25 (477.1 mg, 0.35 mmol) in isopropyl alcohol (10 mL) and methanol (1.1 mL) was added sodium borohydride (202.3 mg, 5.22 mmol) at 0°C. The mixture was heated to room temperature and further stirred for 5 hr, and water (5 mL) was added to discontinue the reaction. The aqueous layer was extracted 5 times with dichloromethane (10 mL), and the combined organic layer was washed with saturated aqueous sodium hydrogen carbonate and saturated brine, and dried over anhydrous sodium sulfate. The solvent was evaporated under reduced pressure and the obtained crude product was purified by silica gel column chromatography (3% methanol/dichloromethane) to give alcohol 26 (383.7 mg, 82% yield).

### (19) Synthesis of compound 27

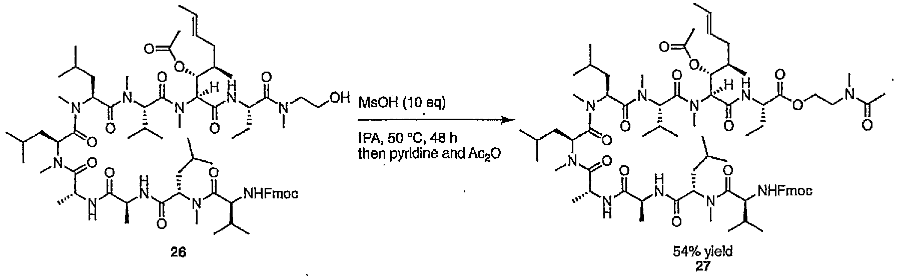

Under an argon atmosphere, alcohol 26 (320.9 mg, 0.24 mmol) was dissolved in isopropyl alcohol (15 mL), methanesulfonic acid (0.16 mL, 2.39 mmol) was added at 50°C, and the mixture was stirred for 48 hr. The reaction solution was cooled to room temperature, and acetic anhydride (0.11 mL, 1.19 mmol) and pyridine (0.48 mL, 5.97 mmol) were successively added. After stirring for 1 hr, saturated aqueous sodium hydrogen carbonate (3 mL) was added to discontinue the reaction. The aqueous layer was extracted four times with dichloromethane (10 mL), and the combined organic layer was washed twice with 5% aqueous hydrochloric acid solution (10 mL), once with saturated aqueous sodium hydrogen carbonate (10 mL), and once with saturated brine. By drying over anhydrous sodium sulfate and evaporation, a crude product was obtained and purified by silica gel column chromatography (1 - 4% methanol/dichloromethane) to give compound 27 (179.5 mg, 54% yield).

### (20) Synthesis of compound 28

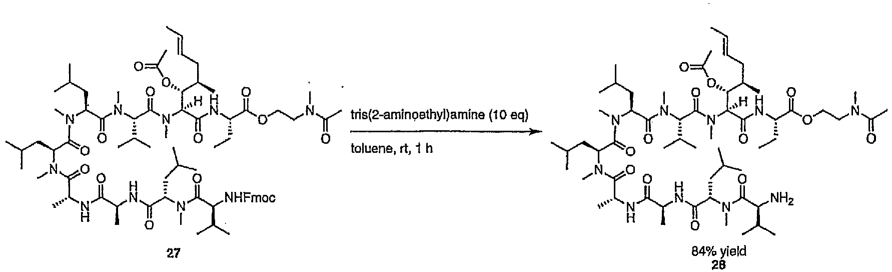

Under an argon atmosphere, to a toluene solution (3 mL) of compound 27 (72.6 mg, 0.052 mmol) was added tris (2-aminoethyl)amine (0.078 mL, 0.52 mmol) at room temperature, and the mixture was stirred for 1 hr. Water (5 mL) was added to discontinue the reaction, and the aqueous layer was extracted three times with dichloromethane (10 mL). The combined organic layer was washed with saturated brine, and dried over anhydrous sodium sulfate. The solvent was evaporated under reduced pressure and the obtained crude product was purified by silica gel column chromatography (5 - 7% methanol/dichloromethane) to give amine 28 (51.0 mg, 84% yield).

### (21) Synthesis of compound 29

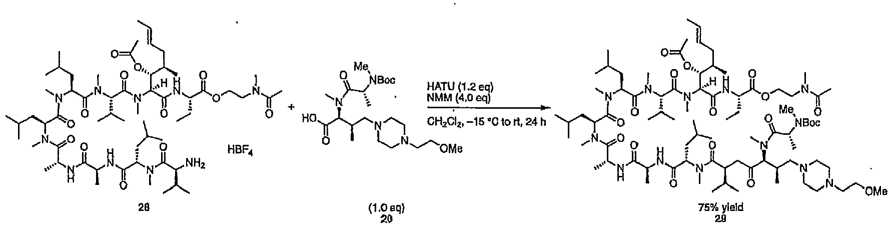

Under an argon atmosphere, amine 28 (26.8 mg, 0.023 mmol) and carboxylic acid 20 (10.6 mg, 0.023 mmol) were dissolved in dichloromethane (3 mL), and a dichloromethane solution of N-methylmorpholine (0.92 mM, 0.1 mL) was added at 0°C. This solution was cooled to -15°C, a DMF solution of HATU (0.28 mM, 0.1 mL) was added, and the mixture was stirred at -15°C for 24 hr. Saturated ammonium chloride (3 mL) was added to discontinue the reaction, and the aqueous layer was extracted three times with dichloromethane (10 mL). The combined organic layer was washed with saturated brine, and dried over anhydrous sodium sulfate. The solvent was evaporated under reduced pressure and the obtained crude product was purified by silica gel column chromatography (5 - 10% methanol/dichloromethane) to give a desired compound 29 (27.6 mg, 75% yield).

### (22) Synthesis of NIM258

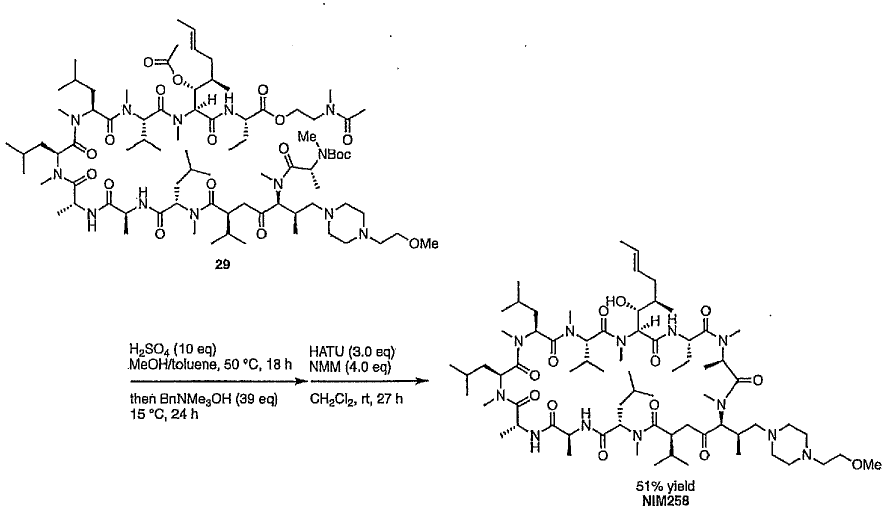

Under an argon atmosphere, compound 29 (27.6 mg, 0.017 mmol) was dissolved in methanol (3 mL) and toluene (1.5 mL), and a methanol solution of sulfuric acid (1.7 mM, 0.2 mL) was added at room temperature. This reaction solution was stirred at 50°C for 18 hr and cooled to 15°C. A methanol solution of benzyltrimethylammonium hydroxide (6.7 mM, 0.1 mL) was added and the mixture was further stirred for 24 hr. Water (3 mL) was added to discontinue the reaction. After stirring for 4 hr, the aqueous layer was extracted 5 times with dichloromethane (10 mL) and the combined organic layer was washed with water and saturated brine in this order, and dried over anhydrous sodium sulfate. The solvent was evaporated under reduced pressure and the obtained crude product was used for the next step without purification.

Under an argon atmosphere, the crude product (24.8 mg) was dissolved in dichloromethane (180 mL). To this reaction solution was added a dichloromethane solution of HATU (19.8 mg, 0.052 mmol) and N-methylmorpholine (0.23 mM, 0.3 mL) at room temperature, and the mixture was stirred for 27 hr. Thereafter, a saturated ammonium chloride aqueous solution (3 mL) was added to discontinue the reaction, and the aqueous layer was extracted four times with dichloromethane (10 mL). The combined organic layer was washed with saturated brine, and dried over anhydrous sodium sulfate. The solvent was evaporated under reduced pressure and the obtained crudely purified product was purified by silica gel column chromatography (3 - 10% methanol/dichloromethane) to give a desired NIM258 (11.8 mg, 51% yield).

### (Example 2) Promotion of collagen trimer secretion by CsA, PSC-833, and Alisporivir

### (1) Generation of a cell line stably expressing split nano luciferase-fused α345

HEK293T cells (Human Embryonic Kidney 293) were obtained from the RIKEN Cell Bank, and thereafter addition of virus concentration solution for COL4A4-3FLAG (Puro), COL4A3-SmBiT (Hyg), COL4A5-LgBiT (BSD) (Omachi K. et al., Cell Chemical Biology 2018, 25, 634-643) in this order, exchange of drug resistance containing medium, passage and preservation of surviving cells were repeated whereby a cell line stably expressing α345 (IV) trimer was generated. COL4A5 variant G1244D was prepared using pLVSIN EF1α BSD COL4A5-LgBiT as a template and using Quikchange (registered trade mark) II Sitedirected Mutagenesis Kit (STRATAGENE (registered trade mark)). The primers used were follows. PCR reactions (95°C for 1 min, one cycle; 95°C for 50 sec, 60°C for 50 sec, 68°C for 90 sec/1 kb, 18 cycles; 68°C for 7 sec) were performed with each Primer. The base sequence of DNA was confirmed by the cycle sequence method (Sigma-Aldrich JAPAN).
[G1244D mutation primer]
   Sense primer: 5'-ccctcctggttctccggatccagctctggaaggacc-3' (SEQ ID NO: 13)
   Antisense primer: 5'-ggtccttccagagctggatccggagaaccaggaggg-3' (SEQ ID NO: 14)
[G1241V mutation primer]
   Sense primer: 5'-gtcccccaggccctcctgtttctccgggtccagctctg-3' (SEQ ID NO: 15)
   Antisense primer: 5'-cagagctggacccggagaaacaggagggcctgggggac-3' (SEQ ID NO: 16)

### (2) Luminescence of Type IV collagen α345 trimer by Nano Luciferase

A cell line stably expressing split nanoluciferase-fused α345 required for the NanoLuc α345 trimer assay was seeded on LumiNuc 96 well white plate (Thermo). After 24 hr, the cells were cultured in phenol red-free DMEM complete medium containing 2-phosphate ascorbic acid (200 µM), substrates were added to the culture supernatant and cells according to the standard method of NanoGlo live cell assay (Promega) reagent, and luminescence was confirmed with luminometer GloMax Navigator (Promega).

### (3) Type IV collagen α345 trimer assay by Nano Luciferase

The cell line stably expressingα345 (COL4A5-G1244D) was detached and seeded on LumiNuc 96 well white plate (Thermo) at a density of 2×10⁴ cells/well. 24 hr after seeding, the medium was exchanged with a phenol red-free DMEM complete medium containing 2-phosphate ascorbic acid (200 µM) and each concentration of cyclosporin A (CsA) (0.1, 0.5, 1, 2, 5, 10 µM), or each concentration of PSC-833 (0.5, 1, 2, 5, 10 µM), or each concentration of Alisporivir (0.1, 0.5, 5, 10 µM) and the cells were cultured. A substrate was added 24 hr later to the culture supernatant according to the standard method of NanoGlo live cell assay (Promega) reagent, and luminescence (index of trimer secretion) was measured by luminometer GloMax Navigator (Promega). The control solvent (CON) used was dimethyl sulfoxide (DMSO).

### (4) Results

The results are shown in Fig. 1 - Fig. 4. The proportion of luminescence (trimer secretion) in the drug-treated group compared to the DMSO-treated group (control: CON) was calculated and expressed in %. CsA was used as the positive control in Fig. 2 and Fig. 3. As a result, CsA promoted collagen trimer secretion, but PSC-833 did not affect collagen trimer secretion or intracellular expression level. On the other hand, Alisporivir showed an action of promoting collagen trimer secretion. In addition, under the treatment conditions of concentrations of 5 µM and 10 µM, Alisporivir showed a stronger action of promoting collagen trimer secretion than CsA.

### (Example 3) Relationship between collagen mutation and promotion of secretion level by drug

AS has been reported to involve various gene mutations. It has been reported that COL4a5/G1241V, like G1244D, maintains relatively high intracellular formation but shows reduced trimer secretion. On the other hand, COL4a5/WT in healthy subjects who do not develop AS is normal in both intracellular formation and trimer secretion. Therefore, the influence of CsA and Alisporivir on the secretion of trimers of COL4a5/WT and COL4a5/G1241V-containing collagens was investigated by an evaluation system using a homeostatic expression cell line.

As a result, CsA and Alisporivir did not show an influence on COL4a5/WT. On the other hand, CsA and Alisporivir promoted extracellular secretion of collagen trimers for COL4a5/G1241V, like G1244D. Therefore, it was shown that CsA and Alisporivir have no action on normal cells and have an effect of promoting the secretion of trimers of mutant COL4a5-containing collagen.

### (Example 4) Collagen trimer secretion when NIM258 is administered

By a method similar to that in Example 1 and Example 2, the extracellular secretion of collagen trimer when 5 µM or 10 µM of NIM258 was administered was measured. As a result, the extracellular secretion of collagen trimers was promoted in the NIM258-administered group as compared with the control DMSOadministered group (Fig. 6). Therefore, it was shown that NIM258 has an effect of promoting the secretion of trimers of mutant COL4a5-containing collagen, like CsA and Alisporivir.

### (Example 5) Relationship between various collagen mutations in COL4A5 and effectiveness of CsA and Alisporivir

A wide range of AS mutants (G227S, G594D, G624D, G869R, S916G, G1030S, G1107R, G1140V, G1220D, G1241C, G1241V, G1244D, P1517T, and R1569Q of COL4A5) with clinical reports, and Exon41 (1202nd to 1263rd amino acids of COL4a5)-deleted variant were measured for the trimer formation promoting ability of CsA and Alisporivir by a method similar to that in Example 1. As the control, wild-type COL4A5 was used.

As a result, collagen trimer formation afforded by CsA and Alisporivir exhibited a significant effect in G1030S, G1107R, G1220D, G1241C, G1241V, G1244D, and Exon41-deleted variants (Fig. 7). In particular, Exon41 deficiency itself and all the mutations existing in Exon41 promoted trimer formation by CsA and Alisporivir. Therefore, the trimer formation promoting effect by CsA and Alisporivir is considered to be particularly effective for collagen dysplasia based on the mutations existing in Exon41 and therearound.

### (Example 6) Elucidation of mechanism of collagen trimer formation promotion by CsA and Alisporivir

To elucidate the mechanism of collagen trimer formation promotion by CsA and Alisporivir, cells in which cyclophilins to which CsA and Alisporivir can bind were knocked down with siRNA were produced, and the collagen trimer formation promoting effect by CsA and Alisporivir in the cells was investigated comprehensively.

As the cells, G1244D mutation-introduced HEK293T cells - for which the effect of CsA and Alisporivir were confirmed in previous experiments- were used.

An cell line stably expressing split nanoluciferase-fused α345 required for the NanoLuc α345 trimer assay was seeded on a 6 well plate (Corning) at 3×10⁵ cells/well, and the siRNAs shown in Table 1 were introduced by lipofection at the time of subconfluence. The target genes are all cyclophilins, and the abbreviations in Table 1 indicate the following substances.
PPIA: Peptidylprolyl isomerase A (cyclophilin A)
PPIB: Peptidylprolyl isomerase B
PPIC: Peptidylprolyl isomerase C
PPID: Peptidylprolyl isomerase D
PPIE: Peptidylprolyl isomerase E
PPIF: Peptidylprolyl isomerase F (cyclophilin D)
PPIG: Peptidylprolyl isomerase G
PPIH: Peptidylprolyl isomerase H
PPIL1: Peptidylprolyl isomerase like 1
NKTR: Natural Killer Cell Triggering Receptor (Peptidyl-Prolyl Cis-Trans Isomerase NKTR)
PPWD1: Peptidylprolyl Isomerase Domain and WD Repeat Containing 1

Lipofectamine RNAi Max was used for transfection of siRNA. The final siRNA concentration was adjusted to 15 nM, and transfection was performed according to the standard protocol. 24 hr after siRNA introduction, the cells were detached and reseeded on a LumiNuc 96 well white plate (Thermo) at 4×10⁴ cells/well. 24 hr thereafter, the medium was exchanged with a phenol red-free DMEM complete medium containing 2-phosphate ascorbic acid (200 µM) and Cyclosporin A (CsA) (1 µM), or Alisporivir (2 µM) and the cells were cultured. Substrates were added 24 hr later to the culture supernatant and cells according to the standard method of NanoGlo live cell assay (Promega) reagent, and luminescence (index of trimer secretion) was measured by luminometer GloMax Navigator (Promega). The control solvent (CON) used was DMSO.

**[Table 1]**

| target gene (human) | siRNA sequence | | | | manufacturer |
|---|---|---|---|---|---|
| | SEQ NO: | sense (5' to 3') | SEQ NO: | antisense (5' to 3') | |
| PPIA | 17 | CAAGAUGACUAAUGUCAAA | 18 | UUUGACAUUAGUCAUCUUG | Sigma-Aldrlch |
| PPIB | 19 | GGUCUCUUCGGAAAGACUGUUCCA A | 20 | UUGGAACAGUCUUUCCGAAGAGAC C | invitrogen |
| PPIC | 21 | GCAACAGGAGAGAAAGGAUAUGGAU | 22 | AUCCAUAUCCUUUCUCUCCUGUUG C | invitrogen |
| PPID | 23 | GCCAAGUAAUUAAAGGAAUAGGAGU | 24 | ACUCCUAUUCCUUUAAUUACUUGG C | Invitrogen |
| PPIE | 25 | CCAAUGAGAAUUAAGGAAGGCUCUU | 26 | AAGAGCCUUCCUUAAUUCUCAUUG G | invitrogen |
| PPIF | 27 | GGCAGAUGUCGUCCCAAAG | 28 | CUUUGGGACGACAUCUGCC | Sigma-Aldrich |
| PPIG | 29 | GGGAUAAGAGUGAGUUGAAUGAAA U | 30 | AUUUCAUUCAACUCACUCUUAUCCC | invitrogen |
| PPIH | 31 | CGUGGUGUUCUUUGAUGUCAGUAU U | 32 | AAUACUGACAUCAAAGAACACCACG | invitrogen |
| PPIL1 | 33 | GAACUUUGCUGAGUUGGCUCGUCG A | 34 | UCGACQAGCCAACUCAGCAAAGUUC | invitrogen |
| NKTR | 35 | GGAAAGCAGCAUGUCCGAAAGUAAA | 36 | UUUACUUUCGGACAUGCUGCUUUC C | invitrogen |
| PPWD1 | 37 | GACCCAACAAUAGUCUGUACAUCAU | 38 | AUGAUGUACAGACUAUUGUUGGGU C | Invitrogen |

As a result, the effect of promoting the formation of collagen trimers by CsA and Alisporivir decreased only in the cells knocked down with PPIF (cyclophilin D) (Fig. 8). Therefrom it was shown that CsA and Alisporivir promote the formation of collagen trimers through binding to cyclophilin D.

### (Example 7) Influence of cyclophilin D on collagen trimer formation

In the above-mentioned experiment of Example 4, it was confirmed that the promotion of collagen trimer formation by CsA and Alisporivir is performed via cyclophilin D. Thus, an influence of cyclophilin D on collagen trimer formation was investigated.

Using G1244D mutation-introduced HEK293T cells in the same manner as in Example 5, the collagen trimer forming ability in cells in which various cyclophilins were knocked down with siRNA was investigated comprehensively.

As a result, remarkable formation of collagen trimers was confirmed in the cyclophilin D-knocked down cells (Fig. 9). Therefrom it was confirmed that inhibition of cyclophilin D promotes formation of collagen trimers.

### (Example 8) Generation and evaluation of AS model mouse

Since the existing AS model animal lacks the gene of collagen protein itself, evaluation of the pathological condition based on the dysfunction of collagen protein could not be performed. Now that the research achievements by the present inventors revealed that an important gene mutation of AS exists in Exon 41 of COL4A5, attempts were made to create a model mouse by which the pathological condition based on the dysfunction of collagen protein can be evaluated, by creating a G1244D or Exon41-deleted mutant mouse as a typical COL4A5 missense mutation (Glycine substitution).

In typical Alport syndrome patients and the existing model mice (Col4a3 KO, Col4a5-G5X), α345(IV) on GBM disappears. Since switching to α345(IV) after birth is not established, fragile α112(IV) in the fetal stage is persistently present in GBM. Furthermore, it has been reported that laminin -which is a basement membrane molecule- accumulates ectopically in GBM (Kashtan CE et al., J Am Soc Nephrol 2001; 12: 252-260.; Cosgrove D et al., Am J Pathol 2000; 157: 1649-1659). Thus, the expression of α5(IV), α2(IV), and Laminin was evaluated in the kidneys of the prepared G1244D mouse and ΔExon41 mouse.

The kick-in method is a method developed by Prof. Kimi Araki et al. of the Institute of Resource Development and Analysis, Kumamoto University, and includes production of an acceptor ES cell in which the target exon into which the mutation is to be inserted is sandwiched between loxP sequences, followed by high probability recombination, using the Cre-loxP system, with the target exon having the target mutation (Tomonoh Y et al., PLoS One 2014; 9: e88549.). To generate a G1244D mutant mouse, a targeting vector into which Exon41 sandwiched between loxPs, a drug selection cassette, and homologous sequences before and after Exon41 are inserted was produced, and ES cells (acceptor ES cells) having a targeting sequence allele were constructed by homologous recombination using CRISPR/Cas9. Then, a vector having Exon41 with G1244D mutation, a drug selection cassette, and loxKMR3 and lox2272 on both ends of the cassette was produced, and the sequence between loxps were exchanged with that of the acceptor ES cells by recombination using Cre recombinase. Since loxKMR3 prevents re-cleavage after recombination by substituting 3 bases on the loxP3' side, and recombination occurs only between lox2272 and lox2272 by substitution of the spacer sequence of loxP, the orientation of the inserted sequence can be specified. Finally, the drug selection cassette was removed in ES cells with the G1244D mutation, and Col4a5-G1244D mouse was established (Fig. 10). mRNA was extracted from the kidney of the produced mouse and the sequence of RT-PCR product was analyzed. As a result, it was confirmed that the desired mutation (G>A) was inserted in the G1244D mouse (not shown).

Acceptor ES cells prepared by the kick-in method have a sequence in which both ends of Exon 41 are sandwiched between loxPs. Thus, Exon41 was deleted by Cre recombinase in the acceptor ES cells, and the drug resistance cassette was removed to establish Col4a5-Exon41-deleted (ΔExon41) mouse (Fig. 10). When mRNA was extracted from the kidney of the produced mouse and the RT-PCR product was electrophoresed, a band shorter by 186 bp than Exon 41 was confirmed in ΔExon41 mouse (not shown). Furthermore, in sequence analysis, it was confirmed that Exon 41 was removed and a transcription product of continuous Exon 40 and Exon 42 was formed (not shown). In addition, even when Eon41 was deleted, the repeat of Glycine-X-Y in the collagen domain was maintained, suggesting that the ability of the α5(IV) mutant to form partial trimers was preserved. Specific experiment methods are described in the following.

### (1) Production of targeting vector

The kidney of C57BL/6 mouse (Charles river, 000664, Black6) was shredded, and genomic DNA was purified with DNeasy Blood & Tissue Kit (QIAGEN, 69504) and used as a template. A short arm of 1.9 kb (Arm1) on the 5'-side and 1.7 kb (Arm3) on the 3'-side were set around 0.9 kb (Arm2) including the target Exon 41. A deleted 3'-side region (147 bp) for inserting double-stranded break (DSB) was formed between Arm2 and Arm3. Arm1 and Arm2 were inserted into the both ends of loxP in the pBluescript II SK(+)loxP vector (Tomonoh Y et al., PLoS One 2014; 9: e88549) (vector 1). Arm3 was inserted into the downstream of the FRT-PGK-neo-lox2272-pA-FRT region of the p03 vector (Tomonoh et al., 2014, supra) (vector 2). The Arm1-loxP-Arm2 sequence of vector 1 was inserted before FRT of vector 2 to give a targeting vector.

### (2) Production of mutation vector

After insertion of Arm2 into the downstream of loxKMR3 in the pK-TEPS-2272 vector (Tomonoh et al., 2014, supra), G1244D mutation was inserted. The sequence of each lox is as follows.
loxP: ATAACTTCGTATAGCATACATTATACGAAGTTAT (SEQ ID NO: 39)
loxKMR3: ATAACTTCGTATAGCATACATTATACCTTGTTAT (SEQ ID NO: 40)
lox2272: ATAACTTCGTATAGGATACTTTATACGAAGTTAT (SEQ ID NO: 41)

### (3) Gene transfer into ES cells

The produced targeting vector was purified by phenol/chloroform extraction, washed with 70% ethanol, dehydrated with 100% ethanol, dissolved in sterilized TE, and then 40 µg of the vector was used for one electroporation. ES cells (6NK) cultured in a 10 cm petri dish for culture to a confluent state were washed with PBS, and the cells were detached with trypsin and suspended in a medium. After centrifuging the cell solution (4°C, 800 rpm), the supernatant was removed and the pellets were resuspended in cold PBS. The targeting vector and Cas9 vector were mixed with the cell solution and transfected by electroporation. Thereafter, the cells were incubated at room temperature for 10 min, suspended in a medium, seeded in a 10 cm petri dish for culture, and statically cultured under the conditions of 5% CO₂, 37°C. After 24 hr, the medium was replaced with a medium containing G418 (Nacalai, 16548) (200 µg/mL), and the drug selection was performed over a period of about 1 week. Colonies were picked up, proliferated, and sequence insertion was confirmed by PCR and Southern blotting. Electroporation of the mutation vector and Cre expression vector was similarly performed on the clone in which the target sequence was confirmed, and the drug was selected over a period of about 1 week in a medium containing Puromycin (Nacalai, 14861-84) (2 mg/ml). Colonies were picked up, proliferated, and the sequence was confirmed by PCR.

### (4) Generation of mutant mouse

Mice were generated from ES cell clones having each target sequence. The mouse 2-cell phase of ICR lineage was immersed in an ES cell suspension, incubated overnight, and then the chimeric embryo was transplanted into the uterus of a pseudopregnant female. The obtained littermate chimera was mated with C57BL/6J mouse to obtain F1 mouse. Furthermore, the drug resistance cassette of FRT was removed by FLP recombinase, and the mouse was established.

### (5) Genotyping

Tail was collected from mouse and washed with 70% ethanol. After air drying, 50 mM NaOH was added, the tail was shredded, boiled for 10 min, and then neutralized with 1M-Tris-HCI. After centrifugation (15,000 rpm, 4°C, 10 min), the supernatant was extracted and used as a DNA sample. PCR reaction (94°C-2 min: 1 cycle, 98°C-10 sec-68°C-1 min/kb: 20 cycle, 4°C-hold) of each sample was performed, and the band was confirmed by agarose gel electrophoresis. The sequences of the primers used for each mouse genotyping are shown in Table 2.

### (6) Extraction of RNA

The kidney tissue was homogenized after adding 1 mL of RNAisoPlus (TaKaRa, 9109) to a 1.5 mL tube, and shredding the tissue. Then, 0.2 mL of chloroform was added, and the mixture was stirred well and allowed to stand at room temperature for 2 min. After centrifugation (12,000 rpm, 4°C, 15 min), the aqueous layer was transferred to a new 1.5 mL tube, an equal amount of chloroform was added, and the mixture was stirred and then centrifuged again (12,000 rpm, 4°C, 15 min). The aqueous layer was further transferred to a new 1.5 mL tube, an equal amount of isopropanol was added, and the mixture was stirred and then centrifuged (12,000 rpm, 4°C, 30 min). The obtained pellets were washed twice with 70% ethanol, air dried, and then dissolved in DEPC-treated water. The yield and purity of the obtained total RNA were measured using an Epoch Microplate Spectrophotometer (BioTek). After confirming that it was a high-purity RNA with an OD260/OD280 ratio of not less than 2.0, the RNA was used in various experiments.

### (7) semi-RT-PCR method

A reverse transcriptase reaction (37°C-30 min, 85°C-10 sec) was performed using Prime Script (registered trade mark) RT reagent kit (TaKaRa, RR036A) and the total RNA extracted from the kidney as a template. Thereafter, PCR reaction (94°C-2 min: 1 cycle, 98°C-10 sec-68°C-1 min/kb: 20 cycle, 4°C-hold) was performed using KOD Plus (TaKaRa), and the band was confirmed by agarose gel electrophoresis. The base sequence of the PCR product was confirmed by the cycle sequence method (Sigma-Aldrich JAPAN). The sequences of the primers used in the PCR reaction are shown in Table 2.

### [Table 2]

**Table 2. Primers used in genotyping and Semi-RT-PCR**

| | | | 5' to 3' sequence | SEQ ID NO: |
|---|---|---|---|---|
| Mouse for genotyping | Control | Arm2 check Fw | aagggttccggatccccatcaagcttatcg | 42 |
| | | Arm3 check Rv | gggtagggatggaagttcaccaaagccc | 43 |
| | | Deletion arm2-3 Fw | cctgtgctcactctttagtagatcaattttgcatcc | 44 |
| | G1244D | Arm1 nick Fw | gctacacataatgacatacacctgaggctg | 45 |
| | | Recon check Rv | ccgagctcgtcgacaagctcgaattagcttgg | 46 |
| | | Deletion arm2-3 Rv | gagtcaatttgaccatgctgtgctgctgaag | 47 |
| | ΔExon41 | Outside arm1 Fw | ggaggtgacatgtgccagtctgcgggaactgg | 48 |
| | | Deletion arm2-3 Rv | gagtcaatttgaccatgctgtgctgctgaag | 49 |
| | | FRT-arm3 check Rv | acacagatctgatatcatcgatgaattcgagcttg c | 50 |
| Semi RT-PCR | | Col4a5 Exon37 | ggtcggtgggagagactggtcttcctg | 51 |
| | | Col4a5 Exon46 | tggagcctggtactccaatgggtcc | 52 |

### (8) Preparation of frozen section of kidney tissue

For the mouse kidney frozen block, a fresh mouse kidney after dissection was cut in half, immersed in OCT compound (Sakura Finetech) in Cryomold, and rapidly frozen in liquid nitrogen. The kidney frozen block was sliced in a thickness of 5 µm using a cryostat (Leica) and used as a frozen section. The prepared sections were dried and stored at -80°C until staining.

### (9) Immunofluorescence staining

The kidney frozen section was fixed by immersing in icecooled acetone solution at -20°C for 5 min. Thereafter, it was blocked for 1 hr at room temperature by Serum free protein block (DAKO, X0909) in a moistening box. After washing with PBS, the primary antibody diluted 1:100 with Antibody diluent (DAKO, S0809) was reacted at room temperature for 1 hr. As the primary antibody, anti-Laminin polyclonal antibody (sigma, L9393), Anti-Human Alpha5(IV) Antibody, Clone H53 (Chondrex, 7078), or Anti-Human Alpha2(IV) Antibody, Clone H22 (Chondrex, 7071) was used. Thereafter, washing with PBS was performed three times, and a fluorescently-labeled secondary antibody (Alexa fluor antibody) diluted 1:500 with Antibody diluent (DAKO) was reacted at room temperature for 1 hr. Washing with PBS was performed three times, the section was mounted in Vector shield (Vector, H-1000), and imaging and analysis was performed using BZ-X700 (Keyence).

### (10) Collection of urine

Urine was collected by 24 hr urine pooling using a mouse metabolism cage (AS ONE). The collected urine was centrifuged (12,000 rpm, 4°C, 5 min) to remove contaminants, and the supernatant was collected and stored at -80°C.

### (11) Measurement of proteinuria score

The proteinuria score was calculated by the ratio of urinary total protein concentration/urinary creatinine concentration. The total urinary protein concentration was measured using the Bradford method. The urinary creatinine level was measured according to the protocol and using the creatinine measurement kit (WAKO).

### (12) CBB staining

Urine samples were prepared with 5×sample buffer for reduction (0.25 M Tris-HCl (pH 6.8), 10% SDS, 4.1M β-mercaptoethanol, 50% glycerol, BPB). SDS-PAGE was performed using 12% polyacrylamide gel and the band was detected by staining with CBB (Coomassie Brilliant Blue).

### (13) Results

G1244D mouse showed the same pattern of α5(IV) expression on the basement membrane as the control mouse, and no change was observed in the expression of α2(IV) and laminin on the mesangial matrix (not shown). In addition, the kidney function was evaluated. As a result, G1244D mouse did not show proteinuria even after 18 weeks of age when the existing Col4a5-G5X mouse showed remarkable pathological conditions, and even at 35 weeks of age, no change was observed in urinary albumin leakage (not shown).

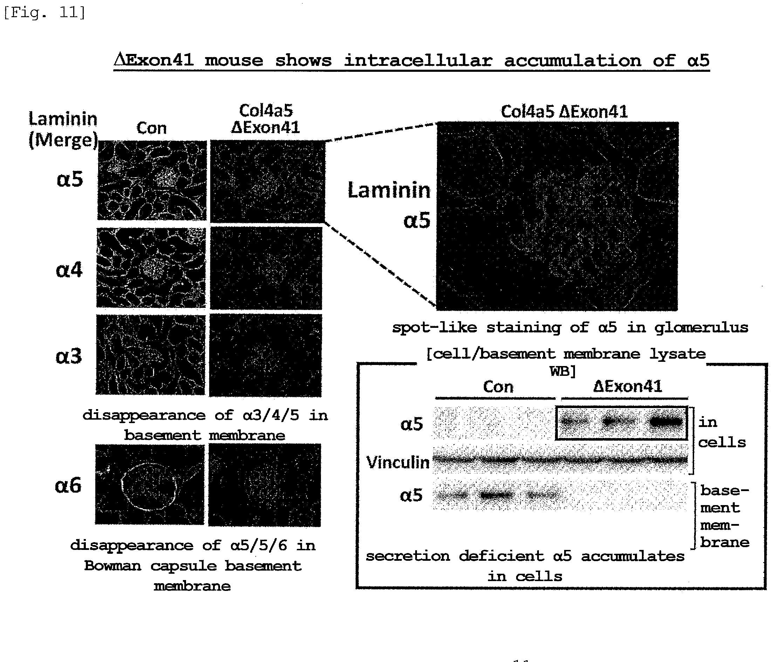

On the other hand, α5(IV) expression on the basement membrane disappeared in ΔExon41 mouse, and α2(IV) was widely expressed instead in GBM. In addition, consistent with previous reports, it was clarified that laminin, which is mainly present as a mesangial matrix in normal glomerulus, shows an increase in ectopic expression in GBM of ΔExon41 mouse (Fig. 11). Furthermore, the kidney function was evaluated. As a result, ΔExon41 mouse exhibited progressive proteinuria (Fig. 12), and significant leakage of urinary albumin and the like already at 10 weeks of age (not shown). In addition, the serum creatinine level increased (Fig. 13). Therefore, ΔExon41 mouse does not undergo normal GBM formation due to abnormal trimer formation of α5(IV) variant, and shows progressive renal pathology such as leakage of albumin and protein into urine. It was therefore shown that the mouse is a model mouse that reflects the dysfunction of AS.

### (Example 9) Administration of cyclosporine A to Alport syndrome model mouse

Whether the AS-like pathology in the ΔExon41 mouse produced in Example 7 is improved by the administration of cyclosporine A was confirmed.

### (1) Administration of cyclosporin A

Cyclosporin A powder was weighed and dissolved in ethanol, and then adjusted to 15, 30, 45 mg/kg/day using 0.5% methylcellulose. After measuring the body weight, the adjusted cyclosporin A was administered intraperitoneally to the mouse once per day. 0.5% Methylcellulose added with ethanol to the final 4% was administered to the control group.

### (2) Collection of urine

Urine was collected once every two weeks from the age of 6 weeks using a metabolic cage (AS ONE) in which mice can freely ingest food and water and can move. The urine accumulated in 24 hr was suspended well and 1 mL was collected in a 1.5 mL tube. The urine was centrifuged (4°C, 12,000 rpm, 5 min) to remove contaminants in the collected urine, and the supernatant was stored at -80°C as a urine sample.

### (3) Calculation of proteinuria score

The proteinuria score was calculated by the ratio of urinary total protein concentration/urinary creatinine concentration. The total urinary protein concentration was measured using the Bradford method. The urinary creatinine concentration was measured according to the protocol and using the creatinine measurement kit.

As a result, it was shown that proteinuria in ΔExon41 mouse was improved by the administration of cyclosporine A at 45 mg/kg/day (Fig. 14). Therefore, it was shown that this model mouse can be used for the evaluation of AS treatment effect via cyclophilin D.

## Claims

1. A composition for promoting secretion of a collagen trimer in a cell having a type-IV collagen gene having a mutation, which composition comprises a cyclophilin D inhibitor as an active ingredient.

2. The composition according to claim 1, wherein the cyclophilin D inhibitor is a cyclosporine derivative represented by the following formula, or a salt thereof, or a hydrate or a solvate thereof: 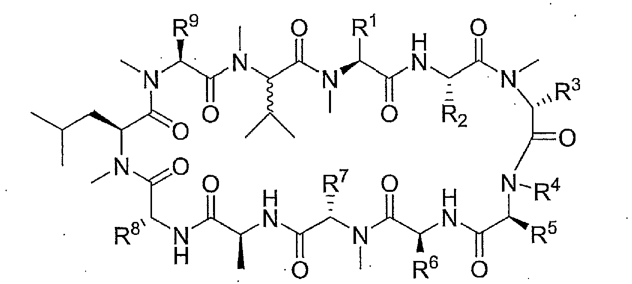 wherein
R¹ is a C5-9 alkyl group or C5-9 alkenyl group substituted by a hydroxyl group; or a group represented by the following formula 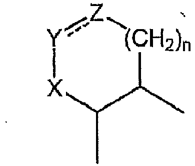 wherein
X is O or NR¹¹
R¹¹ is H or a C1-6 alkyl group,
Y is CR¹²R¹³, CR¹⁴ or >C=O,
R¹² is H, a C1-6 alkyl group, or an OH group,
R¹³ is H, a C1-6 alkyl group, or an OH group,
R¹⁴ is H or a C1-6 alkyl group,
Z is (CH₂)ₘ, CR¹⁵, NR¹⁶, or O,
R¹⁵ is H or a C1-6 alkyl group,
R¹⁶ is H or a C1-6 alkyl group,
m is an integer of 1 - 3,
n is 0 or 1, and
a bond between Y and Z shown by a solid line and a broken line is a single bond or a double bond, when the bond between Y and Z is a single bond, Y is CR¹²R¹³ or C=O, and Z is (CH₂)ₘ, NR¹⁶, or O; when the bond between Y and Z is a double bond, Y is CR¹⁴, and Z is CR¹⁵,
R² is a C1-4 alkyl group optionally substituted by a hydroxyl group,
R³ is a hydrogen atom; a C1-6 alkyl group, a C1-6 alkoxy group, or a C1-6 alkylthio group, each optionally substituted by a hydroxyl group and/or a halogen atom; or a group selected from the following formulas: 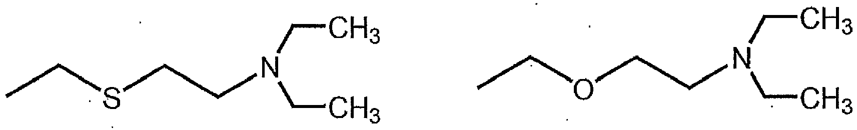 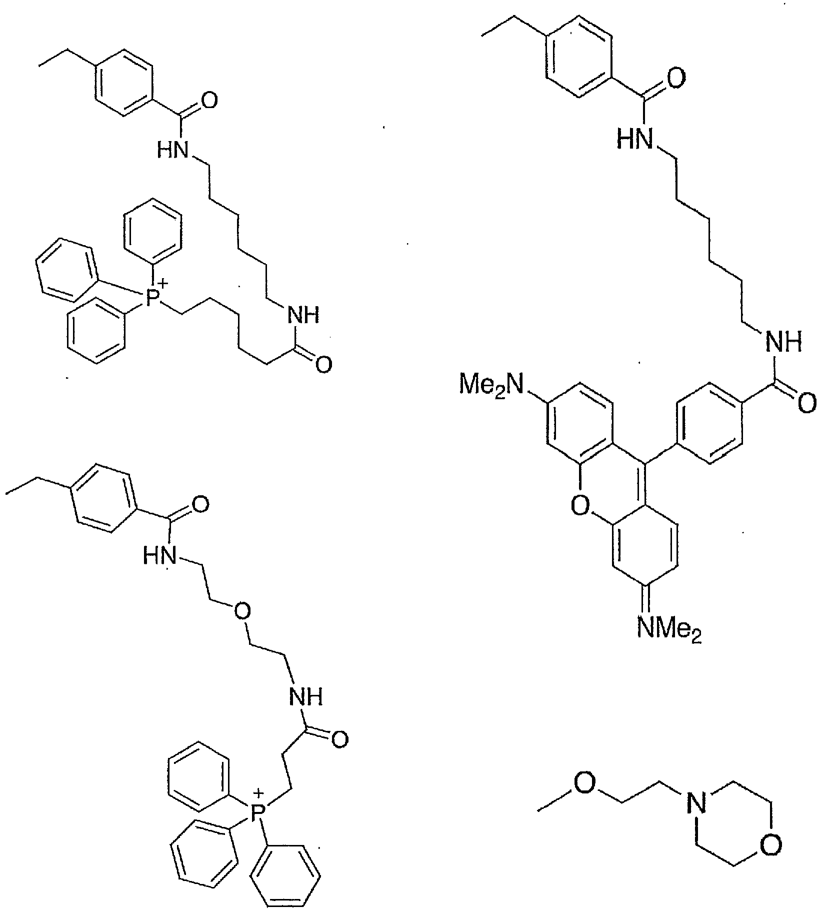
R⁴ is a C1-4 alkyl group,
R⁵ is a C1-6 alkyl group optionally substituted by a hydroxyl group and/or a C1-6 alkoxy group, or a group represented by the following: 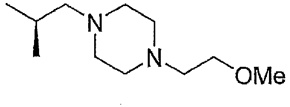
R⁶ is an n-propyl group or an isopropyl group,
R⁷ is a C1-4 alkyl group,
R⁸ is a C1-4 alkyl group optionally substituted by a hydroxyl group,
R⁹ is a branched C3-4 alkyl group, and
a bond shown by a wavy line means that a steric structure of a chiral carbon atom to be bonded is an R form, an S form, or a mixture thereof.

3. The composition according to claim 2, wherein the cyclosporine derivative is a compound selected from the following:

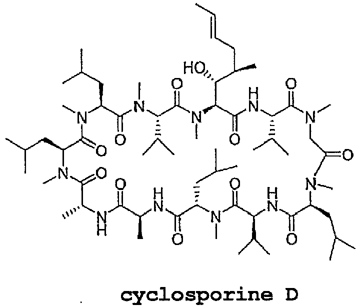

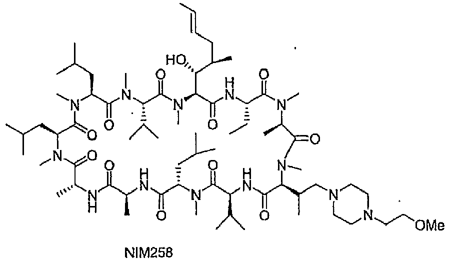

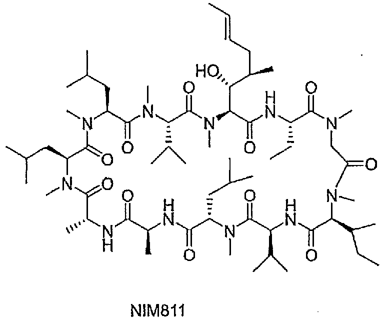

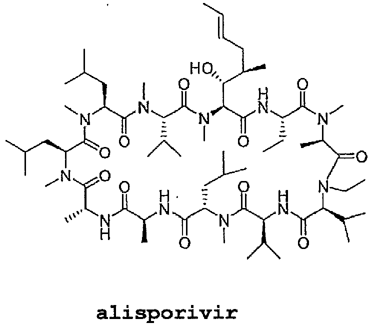

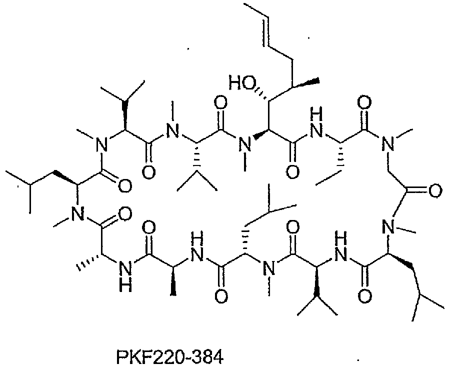

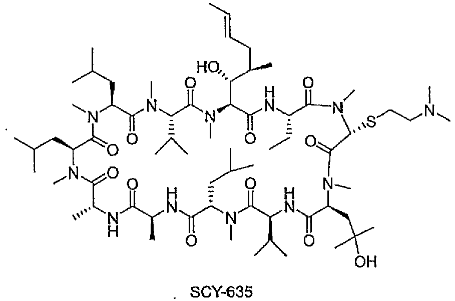

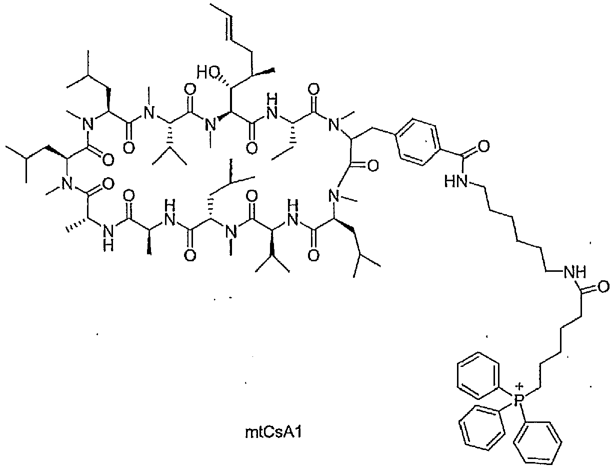

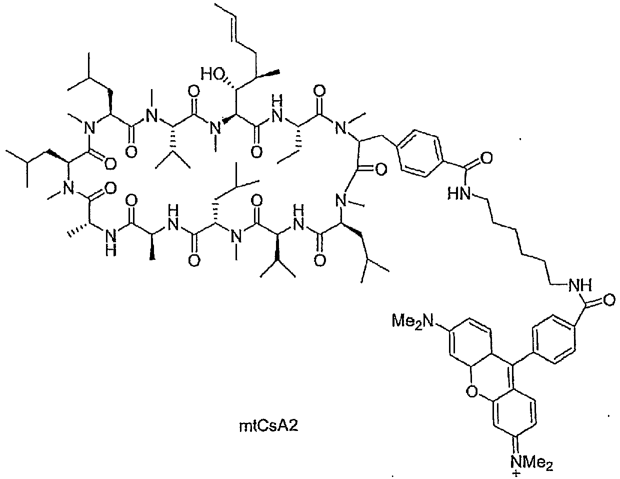

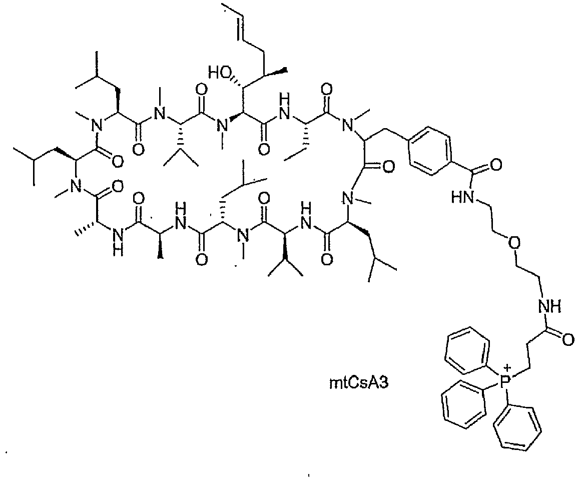

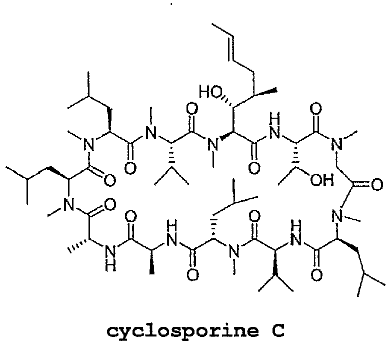

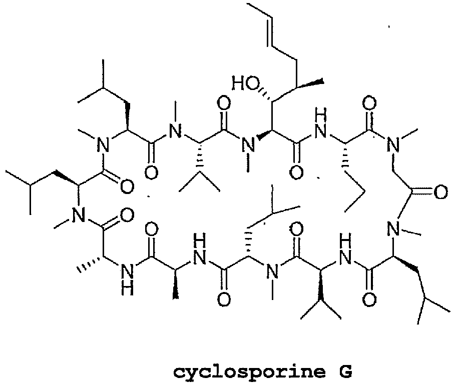

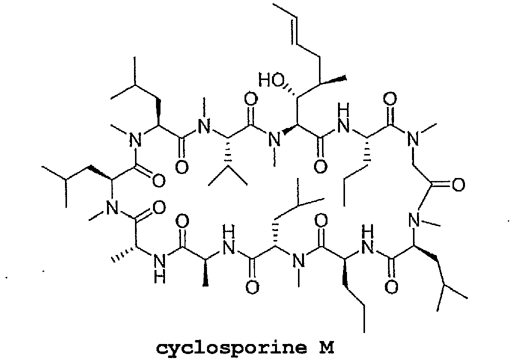

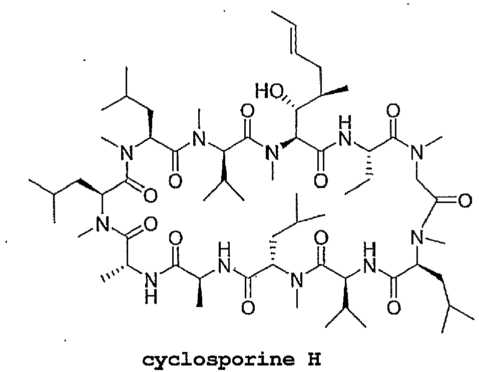

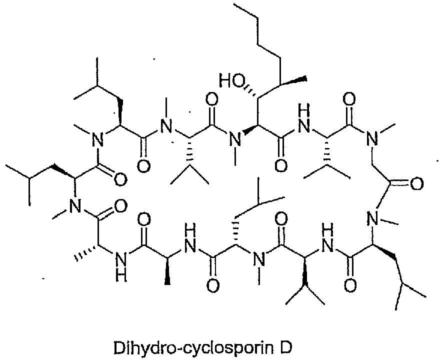

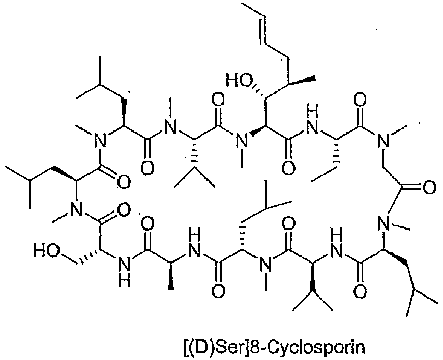

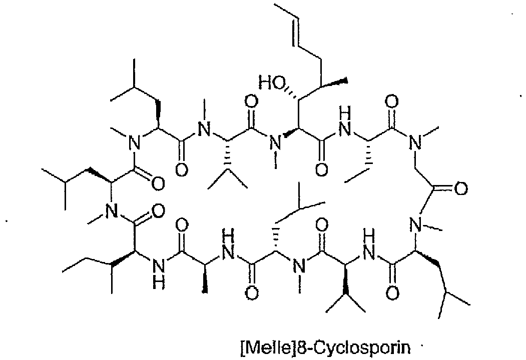

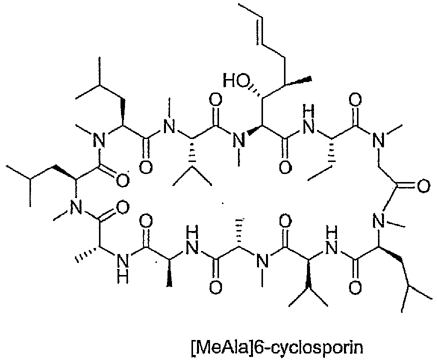

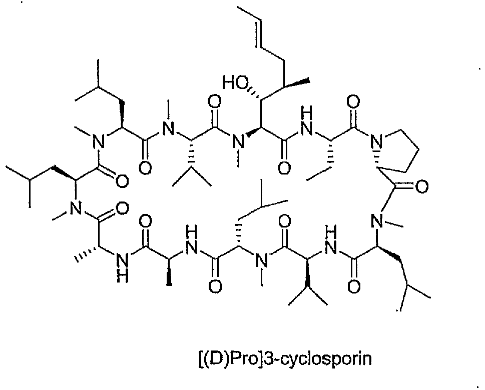

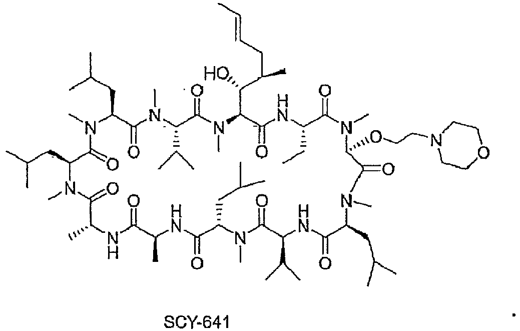

4. The composition according to any one of claims 1 to 3, wherein the cyclophilin D inhibitor does not show a calcineurin inhibitory activity.

5. The composition according to claim 1, wherein the cyclophilin D inhibitor is Alisporivir or NIM258.

6. The composition according to claim 1, wherein the cyclophilin D inhibitor is siRNA, shRNA, miRNA against cyclophilin D, an anti-cyclophilin antibody, an aptamer against cyclophilin D, or an antisense nucleic acid molecule against cyclophilin D.

7. The composition according to any one of claims 1 to 6, wherein the type-IV collagen gene having a mutation is COL4A3, COL4A4, or COL4A5.

8. The composition according to claim 7, wherein the type-IV collagen gene having a mutation is COL4A5.

9. The composition according to any one of claims 1 to 8, wherein the mutation in the type-IV collagen gene causes Alport syndrome.

10. The composition according to claim 8, wherein the mutation in the type-IV collagen gene is a mutation that reduces the secretion of collagen trimer in COL4A5.

11. The composition according to claim 8, wherein the mutation in the type-IV collagen gene is G1030S, G1107R, or a mutation in a region of Exon41 in COL4A5.

12. The composition according to claim 11, wherein the mutation in a region of Exon41 in COL4A5 is G1220D, G1241C, G1241V, or G1244D.

13. A therapeutic drug, prophylactic drug, or improving drug for Alport syndrome, comprising the composition according to any one of claims 1 to 12.

14. The therapeutic drug, prophylactic drug, or improving drug according to claim 13, for administration to a patient judged to have a mutation that decreases secretion of collagen trimer in COL4A5.

15. The therapeutic drug, prophylactic drug, or improving drug according to claim 13, wherein the mutation that decreases secretion of collagen trimer is G1030S, G1107R, or a mutation in a region of Exon41 in COL4A5.

16. The therapeutic drug, prophylactic drug, or improving drug according to claim 15, wherein the mutation in the region of Exon41 in COL4A5 is G1220D, G1241C, G1241V, or G1244D.

17. A non-human animal model having a G1220D mutation in the COL4A5 gene, or a non-human animal model having deletion of Exon41 of the COL4A5 gene.

18. The non-human animal model according to claim 17, wherein the animal is a mouse.

19. A method for evaluating a therapeutic effect of a test drug on Alport syndrome, comprising
administering the test drug to the non-human animal model according to claim 17 or 18,
observing or measuring the Alport syndrome or a level to be an index thereof in the non-human animal model, and
when the administration of the test drug improves the symptoms or conditions based on the deficiency in the formation and/or secretion of collagen trimers, or levels to be the indices thereof, determining that the test drug may have a therapeutic effect on the Alport syndrome.

20. The method according to claim 19, wherein the symptoms or conditions based on the deficiency in the formation and/or secretion of collagen trimers, or levels to be the indices thereof in the non-human animal model is at least one or more kinds selected from the following:
(i) disappearance of α5(IV) expression on the basement membrane
(ii) widespread expression of α2(IV) in GBM
(iii) increased ectopic expression of laminin
(iv) proteinurina
(v) leakage of urinary albumin
(vi) elevated serum creatinine levels
(vii) progressive renal condition.


SEQUENCE LISTING

<110>  NATIONAL UNIVERSITY CORPORATION KUMAMOTO UNIVERSITY

<120>  Agent for radical treatment of intractable hereditary kidney
       disease Alport syndrome

<130>  KMD-1-PCT

<150>  JP2019-165583
<151>  2019-09-11

<160>  71

<170>  PatentIn version 3.5

<210>  1
<211>  6540
<212>  DNA
<213>  Homo sapiens



<220>
<221>  CDS
<222>  (131)..(5140)

<220>
<221>  sig_peptide
<222>  (131)..(211)

<400>  1
aggtctccgc ttggagccgc cgcacccggg acggtgcgta gcgctggaag tccggccttc      60

cgagagctag ctgtccgccg cggcccccgc acgccgggca gccgtccctc gccgcctcgg     120

gcgcgccacc atg ggg ccc cgg ctc agc gtc tgg ctg ctg ctg ctg ccc        169
           Met Gly Pro Arg Leu Ser Val Trp Leu Leu Leu Leu Pro
           1               5                   10

gcc gcc ctt ctg ctc cac gag gag cac agc cgg gcc gct gcg aag ggt       217
Ala Ala Leu Leu Leu His Glu Glu His Ser Arg Ala Ala Ala Lys Gly
    15                  20                  25

ggc tgt gct ggc tct ggc tgt ggc aaa tgt gac tgc cat gga gtg aag       265
Gly Cys Ala Gly Ser Gly Cys Gly Lys Cys Asp Cys His Gly Val Lys
30                  35                  40                  45

gga caa aag ggt gaa aga ggc ctc ccg ggg tta caa ggt gtc att ggg       313
Gly Gln Lys Gly Glu Arg Gly Leu Pro Gly Leu Gln Gly Val Ile Gly
                50                  55                  60

ttt cct gga atg caa gga cct gag ggg cca cag gga cca cca gga caa       361
Phe Pro Gly Met Gln Gly Pro Glu Gly Pro Gln Gly Pro Pro Gly Gln
            65                  70                  75

aag ggt gat act gga gaa cca gga cta cct gga aca aaa ggg aca aga       409
Lys Gly Asp Thr Gly Glu Pro Gly Leu Pro Gly Thr Lys Gly Thr Arg
        80                  85                  90

gga cct ccg gga gca tct ggc tac cct gga aac cca gga ctt ccc gga       457
Gly Pro Pro Gly Ala Ser Gly Tyr Pro Gly Asn Pro Gly Leu Pro Gly
    95                  100                 105

att cct ggc caa gac ggc ccg cca ggc ccc cca ggt att cca gga tgc 505
Ile Pro Gly Gln Asp Gly Pro Pro Gly Pro Pro Gly Ile Pro Gly Cys
110                 115                 120                 125

aat ggc aca aag ggg gag aga ggg ccg ctc ggg cct cct ggc ttg cct 553
Asn Gly Thr Lys Gly Glu Arg Gly Pro Leu Gly Pro Pro Gly Leu Pro
                130                 135                 140

ggt ttc gct gga aat ccc gga cca cca ggc tta cca ggg atg aag ggt 601
Gly Phe Ala Gly Asn Pro Gly Pro Pro Gly Leu Pro Gly Met Lys Gly
            145                 150                 155

gat cca ggt gag ata ctt ggc cat gtg ccc ggg atg ctg ttg aaa ggt 649
Asp Pro Gly Glu Ile Leu Gly His Val Pro Gly Met Leu Leu Lys Gly
        160                 165                 170

gaa aga gga ttt ccc gga atc cca ggg act cca ggc cca cca gga ctg 697
Glu Arg Gly Phe Pro Gly Ile Pro Gly Thr Pro Gly Pro Pro Gly Leu
    175                 180                 185

cca ggg ctt caa ggt cct gtt ggg cct cca gga ttt acc gga cca cca 745
Pro Gly Leu Gln Gly Pro Val Gly Pro Pro Gly Phe Thr Gly Pro Pro
190                 195                 200                 205

ggt ccc cca ggc cct ccc ggc cct cca ggt gaa aag gga caa atg ggc 793
Gly Pro Pro Gly Pro Pro Gly Pro Pro Gly Glu Lys Gly Gln Met Gly
                210                 215                 220

tta agt ttt caa gga cca aaa ggt gac aag ggt gac caa ggg gtc agt 841
Leu Ser Phe Gln Gly Pro Lys Gly Asp Lys Gly Asp Gln Gly Val Ser
            225                 230                 235

ggg cct cca gga gta cca gga caa gct caa gtt caa gaa aaa gga gac 889
Gly Pro Pro Gly Val Pro Gly Gln Ala Gln Val Gln Glu Lys Gly Asp
        240                 245                 250

ttc gcc acc aag gga gaa aag ggc caa aaa ggt gaa cct gga ttt cag 937
Phe Ala Thr Lys Gly Glu Lys Gly Gln Lys Gly Glu Pro Gly Phe Gln
    255                 260                 265

ggg atg cca ggg gtc gga gag aaa ggt gaa ccc gga aaa cca gga ccc 985
Gly Met Pro Gly Val Gly Glu Lys Gly Glu Pro Gly Lys Pro Gly Pro
270                 275                 280                 285

aga ggc aaa ccc gga aaa gat ggt gac aaa ggg gaa aaa ggg agt ccc 1033
Arg Gly Lys Pro Gly Lys Asp Gly Asp Lys Gly Glu Lys Gly Ser Pro
                290                 295                 300

ggt ttt cct ggt gaa ccc ggg tac cca gga ctc ata ggc cgc cag ggc 1081
Gly Phe Pro Gly Glu Pro Gly Tyr Pro Gly Leu Ile Gly Arg Gln Gly
            305                 310                 315

ccg cag gga gaa aag ggt gaa gca ggt cct cct ggc cca cct gga att 1129
Pro Gln Gly Glu Lys Gly Glu Ala Gly Pro Pro Gly Pro Pro Gly Ile
        320                 325                 330

gtt ata ggc aca gga cct ttg gga gaa aaa gga gag agg ggc tac cct 1177
Val Ile Gly Thr Gly Pro Leu Gly Glu Lys Gly Glu Arg Gly Tyr Pro
    335                 340                 345

gga act ccg ggg cca aga gga gag cca ggc cca aaa ggt ttc cca gga 1225
Gly Thr Pro Gly Pro Arg Gly Glu Pro Gly Pro Lys Gly Phe Pro Gly
350                 355                 360                 365

cta cca ggc caa ccc gga cct cca ggc ctc cct gta cct ggg cag gct 1273
Leu Pro Gly Gln Pro Gly Pro Pro Gly Leu Pro Val Pro Gly Gln Ala
                370                 375                 380

ggt gcc cct ggc ttc cct ggt gaa aga gga gaa aaa ggt gac cga gga 1321
Gly Ala Pro Gly Phe Pro Gly Glu Arg Gly Glu Lys Gly Asp Arg Gly
            385                 390                 395

ttt cct ggt aca tct ctg cca gga cca agt gga aga gat ggg ctc ccg 1369
Phe Pro Gly Thr Ser Leu Pro Gly Pro Ser Gly Arg Asp Gly Leu Pro
        400                 405                 410

ggt cct cct ggt tcc cct ggg ccc cct ggg cag cct ggc tac aca aat 1417
Gly Pro Pro Gly Ser Pro Gly Pro Pro Gly Gln Pro Gly Tyr Thr Asn
    415                 420                 425

gga att gtg gaa tgt cag ccc gga cct cca ggt gac cag ggt cct cct 1465
Gly Ile Val Glu Cys Gln Pro Gly Pro Pro Gly Asp Gln Gly Pro Pro
430                 435                 440                 445

gga att cca ggg cag cca gga ttt ata ggc gaa att gga gag aaa ggt 1513
Gly Ile Pro Gly Gln Pro Gly Phe Ile Gly Glu Ile Gly Glu Lys Gly
                450                 455                 460

caa aaa gga gag agt tgc ctc atc tgt gat ata gac gga tat cgg ggg 1561
Gln Lys Gly Glu Ser Cys Leu Ile Cys Asp Ile Asp Gly Tyr Arg Gly
            465                 470                 475

cct ccc ggg cca cag gga ccc ccg gga gaa ata ggt ttc cca ggg cag 1609
Pro Pro Gly Pro Gln Gly Pro Pro Gly Glu Ile Gly Phe Pro Gly Gln
        480                 485                 490

cca ggg gcc aag ggc gac aga ggt ttg cct ggc aga gat ggt gtt gca 1657
Pro Gly Ala Lys Gly Asp Arg Gly Leu Pro Gly Arg Asp Gly Val Ala
    495                 500                 505

gga gtg cca ggc cct caa ggt aca cca ggg ctg ata ggc cag cca gga 1705
Gly Val Pro Gly Pro Gln Gly Thr Pro Gly Leu Ile Gly Gln Pro Gly
510                 515                 520                 525

gcc aag ggg gag cct ggt gag ttt tat ttc gac ttg cgg ctc aaa ggt 1753
Ala Lys Gly Glu Pro Gly Glu Phe Tyr Phe Asp Leu Arg Leu Lys Gly
                530                 535                 540

gac aaa gga gac cca ggc ttt cca gga cag ccc ggc atg cca ggg aga 1801
Asp Lys Gly Asp Pro Gly Phe Pro Gly Gln Pro Gly Met Pro Gly Arg
            545                 550                 555

gcg ggt tct cct gga aga gat ggc cat ccg ggt ctt cct ggc ccc aag 1849
Ala Gly Ser Pro Gly Arg Asp Gly His Pro Gly Leu Pro Gly Pro Lys
        560                 565                 570

ggc tcg ccg ggt tct gta gga ttg aaa gga gag cgt ggc ccc cct gga 1897
Gly Ser Pro Gly Ser Val Gly Leu Lys Gly Glu Arg Gly Pro Pro Gly
    575                 580                 585

gga gtt gga ttc cca ggc agt cgt ggt gac acc ggc ccc cct ggg cct 1945
Gly Val Gly Phe Pro Gly Ser Arg Gly Asp Thr Gly Pro Pro Gly Pro
590                 595                 600                 605

cca gga tat ggt cct gct ggt ccc att ggt gac aaa gga caa gca ggc 1993
Pro Gly Tyr Gly Pro Ala Gly Pro Ile Gly Asp Lys Gly Gln Ala Gly

610                 615                 620

ttt cct gga ggc cct gga tcc cca ggc ctg cca ggt cca aag ggt gaa        2041
Phe Pro Gly Gly Pro Gly Ser Pro Gly Leu Pro Gly Pro Lys Gly Glu
            625                 630                 635

cca gga aaa att gtt cct tta cca ggc ccc cct gga gca gaa gga ctg        2089
Pro Gly Lys Ile Val Pro Leu Pro Gly Pro Pro Gly Ala Glu Gly Leu
        640                 645                 650

ccg ggg tcc cca ggc ttc cca ggt ccc caa gga gac cga ggc ttt ccc        2137
Pro Gly Ser Pro Gly Phe Pro Gly Pro Gln Gly Asp Arg Gly Phe Pro
    655                 660                 665

gga acc cca gga agg cca ggc ctg cca gga gag aag ggc gct gtg ggc        2185
Gly Thr Pro Gly Arg Pro Gly Leu Pro Gly Glu Lys Gly Ala Val Gly
670                 675                 680                 685

cag cca ggc att gga ttt cca ggg ccc ccc ggc ccc aaa ggt gtt gac        2233
Gln Pro Gly Ile Gly Phe Pro Gly Pro Pro Gly Pro Lys Gly Val Asp
                690                 695                 700

ggc tta cct gga gac atg ggg cca ccg ggg act cca ggt cgc ccg gga        2281
Gly Leu Pro Gly Asp Met Gly Pro Pro Gly Thr Pro Gly Arg Pro Gly
            705                 710                 715

ttt aat ggc tta cct ggg aac cca ggt gtg cag ggc cag aag gga gag        2329
Phe Asn Gly Leu Pro Gly Asn Pro Gly Val Gln Gly Gln Lys Gly Glu
        720                 725                 730

cct gga gtt ggt cta ccg gga ctc aaa ggt ttg cca ggt ctt ccc ggc        2377
Pro Gly Val Gly Leu Pro Gly Leu Lys Gly Leu Pro Gly Leu Pro Gly
    735                 740                 745

att cct ggc aca ccc ggg gag aag ggg agc att ggg gta cca ggc gtt        2425
Ile Pro Gly Thr Pro Gly Glu Lys Gly Ser Ile Gly Val Pro Gly Val
750                 755                 760                 765

cct gga gaa cat gga gcg atc gga ccc cct ggg ctt cag ggg atc aga        2473
Pro Gly Glu His Gly Ala Ile Gly Pro Pro Gly Leu Gln Gly Ile Arg
                770                 775                 780

ggt gaa ccg gga cct cct gga ttg cca ggc tcc gtg ggg tct cca gga        2521
Gly Glu Pro Gly Pro Pro Gly Leu Pro Gly Ser Val Gly Ser Pro Gly
            785                 790                 795

gtt cca gga ata ggc ccc cct gga gct agg ggt ccc cct gga gga cag        2569
Val Pro Gly Ile Gly Pro Pro Gly Ala Arg Gly Pro Pro Gly Gly Gln
        800                 805                 810

gga cca ccg ggg ttg tca ggc cct cct gga ata aaa gga gag aag ggt        2617
Gly Pro Pro Gly Leu Ser Gly Pro Pro Gly Ile Lys Gly Glu Lys Gly
    815                 820                 825

ttc ccc gga ttc cct gga ctg gac atg ccg ggc cct aaa gga gat aaa        2665
Phe Pro Gly Phe Pro Gly Leu Asp Met Pro Gly Pro Lys Gly Asp Lys
830                 835                 840                 845

ggg gct caa gga ctc cct ggc ata acg gga cag tcg ggg ctc cct ggc        2713
Gly Ala Gln Gly Leu Pro Gly Ile Thr Gly Gln Ser Gly Leu Pro Gly
                850                 855                 860

ctt cct gga cag cag ggg gct cct ggg att cct ggg ttt cca ggt tcc        2761

Leu Pro Gly Gln Gln Gly Ala Pro Gly Ile Pro Gly Phe Pro Gly Ser
            865                 870                 875

aag gga gaa atg ggc gtc atg ggg acc ccc ggg cag ccg ggc tca cca     2809
Lys Gly Glu Met Gly Val Met Gly Thr Pro Gly Gln Pro Gly Ser Pro
        880                 885                 890

gga cca gtg ggt gct cct gga tta ccg ggt gaa aaa ggg gac cat ggc     2857
Gly Pro Val Gly Ala Pro Gly Leu Pro Gly Glu Lys Gly Asp His Gly
    895                 900                 905

ttt ccg ggc tcc tca gga ccc agg gga gac cct ggc ttg aaa ggt gat     2905
Phe Pro Gly Ser Ser Gly Pro Arg Gly Asp Pro Gly Leu Lys Gly Asp
910                 915                 920                 925

aag ggg gat gtc ggt ctc cct ggc aag cct ggc tcc atg gat aag gtg     2953
Lys Gly Asp Val Gly Leu Pro Gly Lys Pro Gly Ser Met Asp Lys Val
                930                 935                 940

gac atg ggc agc atg aag ggc cag aaa gga gac caa gga gag aaa gga     3001
Asp Met Gly Ser Met Lys Gly Gln Lys Gly Asp Gln Gly Glu Lys Gly
            945                 950                 955

caa att gga cca att ggt gag aag gga tcc cga gga gac cct ggg acc     3049
Gln Ile Gly Pro Ile Gly Glu Lys Gly Ser Arg Gly Asp Pro Gly Thr
        960                 965                 970

cca gga gtg cct gga aag gac ggg cag gca gga cag cct ggg cag cca     3097
Pro Gly Val Pro Gly Lys Asp Gly Gln Ala Gly Gln Pro Gly Gln Pro
    975                 980                 985

gga cct aaa ggt gat cca ggt ata agt gga acc  cca ggt gct cca gga     3145
Gly Pro Lys Gly Asp Pro Gly Ile Ser Gly Thr  Pro Gly Ala Pro Gly
990                 995                 1000                1005

ctt ccg gga cca aaa  gga tct gtt ggt gga  atg ggc ttg cca gga     3190
Leu Pro Gly Pro Lys  Gly Ser Val Gly Gly  Met Gly Leu Pro Gly
                1010                 1015                 1020

aca cct gga gag aaa  ggt gtg cct ggc atc  cct ggc cca caa ggt     3235
Thr Pro Gly Glu Lys  Gly Val Pro Gly Ile  Pro Gly Pro Gln Gly
                1025                 1030                 1035

tca cct ggc tta cct  gga gac aaa ggt gca  aaa gga gag aaa ggg     3280
Ser Pro Gly Leu Pro  Gly Asp Lys Gly Ala  Lys Gly Glu Lys Gly
                1040                 1045                 1050

cag gca ggc cca cct  ggc ata ggc atc cca  ggg ctg cga ggt gaa     3325
Gln Ala Gly Pro Pro  Gly Ile Gly Ile Pro  Gly Leu Arg Gly Glu
                1055                 1060                 1065

aag gga gat caa ggg  ata gcg ggt ttc cca  gga agc cct gga gag     3370
Lys Gly Asp Gln Gly  Ile Ala Gly Phe Pro  Gly Ser Pro Gly Glu
                1070                 1075                 1080

aag gga gaa aaa gga  agc att ggg atc cca  gga atg cca ggg tcc     3415
Lys Gly Glu Lys Gly  Ser Ile Gly Ile Pro  Gly Met Pro Gly Ser
                1085                 1090                 1095

cca ggc ctt aaa ggg  tct ccc ggg agt gtt  ggc tat cca gga agt     3460
Pro Gly Leu Lys Gly  Ser Pro Gly Ser Val  Gly Tyr Pro Gly Ser
                1100                 1105                 1110

cct ggg cta cct gga gaa aaa ggt gac aaa ggc ctc cca gga ttg 3505
Pro Gly Leu Pro Gly Glu Lys Gly Asp Lys Gly Leu Pro Gly Leu
                1115                1120                1125

gat ggc atc cct ggt gtc aaa gga gaa gca ggt ctt cct ggg act 3550
Asp Gly Ile Pro Gly Val Lys Gly Glu Ala Gly Leu Pro Gly Thr
                1130                1135                1140

cct ggc ccc aca ggc cca gct ggc cag aaa ggg gag cca ggc agt 3595
Pro Gly Pro Thr Gly Pro Ala Gly Gln Lys Gly Glu Pro Gly Ser
                1145                1150                1155

gat gga atc ccg ggg tca gca gga gag aag ggt gaa cca ggt cta 3640
Asp Gly Ile Pro Gly Ser Ala Gly Glu Lys Gly Glu Pro Gly Leu
                1160                1165                1170

cca gga aga gga ttc cca ggg ttt cca ggg gcc aaa gga gac aaa 3685
Pro Gly Arg Gly Phe Pro Gly Phe Pro Gly Ala Lys Gly Asp Lys
                1175                1180                1185

ggt tca aag ggt gag gtg ggt ttc cca gga tta gcc ggg agc cca 3730
Gly Ser Lys Gly Glu Val Gly Phe Pro Gly Leu Ala Gly Ser Pro
                1190                1195                1200

gga att cct gga tcc aaa gga gag caa gga ttc atg ggt cct ccg 3775
Gly Ile Pro Gly Ser Lys Gly Glu Gln Gly Phe Met Gly Pro Pro
                1205                1210                1215

ggg ccc cag gga cag ccg ggg tta ccg gga tcc cca ggc cat gcc 3820
Gly Pro Gln Gly Gln Pro Gly Leu Pro Gly Ser Pro Gly His Ala
                1220                1225                1230

acg gag ggg ccc aaa gga gac cgc gga cct cag ggc cag cct ggc 3865
Thr Glu Gly Pro Lys Gly Asp Arg Gly Pro Gln Gly Gln Pro Gly
                1235                1240                1245

ctg cca gga ctt ccg gga ccc atg ggg cct cca ggg ctt cct ggg 3910
Leu Pro Gly Leu Pro Gly Pro Met Gly Pro Pro Gly Leu Pro Gly
                1250                1255                1260

att gat gga gtt aaa ggt gac aaa gga aat cca ggc tgg cca gga 3955
Ile Asp Gly Val Lys Gly Asp Lys Gly Asn Pro Gly Trp Pro Gly
                1265                1270                1275

gca ccc ggt gtc cca ggg ccc aag gga gac cct gga ttc cag ggc 4000
Ala Pro Gly Val Pro Gly Pro Lys Gly Asp Pro Gly Phe Gln Gly
                1280                1285                1290

atg cct ggt att ggt ggc tct cca gga atc aca ggc tct aag ggt 4045
Met Pro Gly Ile Gly Gly Ser Pro Gly Ile Thr Gly Ser Lys Gly
                1295                1300                1305

gat atg ggg cct cca gga gtt cca gga ttt caa ggt cca aaa ggt 4090
Asp Met Gly Pro Pro Gly Val Pro Gly Phe Gln Gly Pro Lys Gly
                1310                1315                1320

ctt cct ggc ctc cag gga att aaa ggt gat caa ggc gat caa ggc 4135
Leu Pro Gly Leu Gln Gly Ile Lys Gly Asp Gln Gly Asp Gln Gly
                1325                1330                1335

gtc ccg gga gct aaa ggt ctc ccg ggt cct cct ggc ccc cca ggt 4180
Val Pro Gly Ala Lys Gly Leu Pro Gly Pro Pro Gly Pro Pro Gly
                1340                1345                1350

cct tac gac atc atc aaa ggg gag ccc ggg ctc cct ggt cct gag 4225
Pro Tyr Asp Ile Ile Lys Gly Glu Pro Gly Leu Pro Gly Pro Glu
                1355                1360                1365

ggc ccc cca ggg ctg aaa ggg ctt cag gga ctg cca ggc ccg aaa 4270
Gly Pro Pro Gly Leu Lys Gly Leu Gln Gly Leu Pro Gly Pro Lys
                1370                1375                1380

ggc cag caa ggt gtt aca gga ttg gtg ggt ata cct gga cct cca 4315
Gly Gln Gln Gly Val Thr Gly Leu Val Gly Ile Pro Gly Pro Pro
                1385                1390                1395

ggt att cct ggg ttt gac ggt gcc cct ggc cag aaa gga gag atg 4360
Gly Ile Pro Gly Phe Asp Gly Ala Pro Gly Gln Lys Gly Glu Met
                1400                1405                1410

gga cct gcc ggg cct act ggt cca aga gga ttt cca ggt cca cca 4405
Gly Pro Ala Gly Pro Thr Gly Pro Arg Gly Phe Pro Gly Pro Pro
                1415                1420                1425

ggc ccc gat ggg ttg cca gga tcc atg ggg ccc cca ggc acc cca 4450
Gly Pro Asp Gly Leu Pro Gly Ser Met Gly Pro Pro Gly Thr Pro
                1430                1435                1440

tct gtt gat cac ggc ttc ctt gtg acc agg cat agt caa aca ata 4495
Ser Val Asp His Gly Phe Leu Val Thr Arg His Ser Gln Thr Ile
                1445                1450                1455

gat gac cca cag tgt cct tct ggg acc aaa att ctt tac cac ggg 4540
Asp Asp Pro Gln Cys Pro Ser Gly Thr Lys Ile Leu Tyr His Gly
                1460                1465                1470

tac tct ttg ctc tac gtg caa ggc aat gaa cgg gcc cat ggc cag 4585
Tyr Ser Leu Leu Tyr Val Gln Gly Asn Glu Arg Ala His Gly Gln
                1475                1480                1485

gac ttg ggc acg gcc ggc agc tgc ctg cgc aag ttc agc aca atg 4630
Asp Leu Gly Thr Ala Gly Ser Cys Leu Arg Lys Phe Ser Thr Met
                1490                1495                1500

ccc ttc ctg ttc tgc aat att aac aac gtg tgc aac ttt gca tca 4675
Pro Phe Leu Phe Cys Asn Ile Asn Asn Val Cys Asn Phe Ala Ser
                1505                1510                1515

cga aat gac tac tcg tac tgg ctg tcc acc cct gag ccc atg ccc 4720
Arg Asn Asp Tyr Ser Tyr Trp Leu Ser Thr Pro Glu Pro Met Pro
                1520                1525                1530

atg tca atg gca ccc atc acg ggg gaa aac ata aga cca ttt att 4765
Met Ser Met Ala Pro Ile Thr Gly Glu Asn Ile Arg Pro Phe Ile
                1535                1540                1545

agt agg tgt gct gtg tgt gag gcg cct gcc atg gtg atg gcc gtg 4810
Ser Arg Cys Ala Val Cys Glu Ala Pro Ala Met Val Met Ala Val
                1550                1555                1560

cac agc cag acc att cag atc cca ccg tgc ccc agc ggg tgg tcc 4855
His Ser Gln Thr Ile Gln Ile Pro Pro Cys Pro Ser Gly Trp Ser
                1565                1570                1575

tcg ctg tgg atc ggc tac tct ttt gtg atg cac acc agc gct ggt 4900
Ser Leu Trp Ile Gly Tyr Ser Phe Val Met His Thr Ser Ala Gly

1580                1585                1590

gca gaa ggc tct ggc  caa gcc ctg gcg tcc  ccc ggc tcc tgc ctg        4945
Ala Glu Gly Ser Gly  Gln Ala Leu Ala Ser  Pro Gly Ser Cys Leu
                1595                1600                1605

gag gag ttt aga agt  gcg cca ttc atc gag  tgt cac ggc cgt ggg        4990
Glu Glu Phe Arg Ser  Ala Pro Phe Ile Glu  Cys His Gly Arg Gly
                1610                1615                1620

acc tgc aat tac tac  gca aac gct tac agc  ttt tgg ctc gcc acc        5035
Thr Cys Asn Tyr Tyr  Ala Asn Ala Tyr Ser  Phe Trp Leu Ala Thr
                1625                1630                1635

ata gag agg agc gag  atg ttc aag aag cct  acg ccg tcc acc ttg        5080
Ile Glu Arg Ser Glu  Met Phe Lys Lys Pro  Thr Pro Ser Thr Leu
                1640                1645                1650

aag gca ggg gag ctg  cgc acg cac gtc agc  cgc tgc caa gtc tgt        5125
Lys Ala Gly Glu Leu  Arg Thr His Val Ser  Arg Cys Gln Val Cys
                1655                1660                1665

atg aga aga aca taa tgaagcctga ctcagctaat gtcacaacat ggtgctactt        5180
Met Arg Arg Thr

cttcttcttt ttgttaacag caacgaaccc tagaaatata tcctgtgtac ctcactgtcc        5240

aatatgaaaa ccgtaaagtg ccttatagga atttgcgtaa ctaacacacc ctgcttcatt        5300

gacctctact tgctgaagga gaaaaagaca gcgataagct ttcaatagtg gcataccaaa        5360

tggcactttt gatgaaataa aatatcaata ttttctgcaa tccaatgcac tgatgtgtga        5420

agtgagaact ccatcagaaa accaaagggt gctaggaggt gtgggtgcct tccatactgt        5480

ttgcccattt tcattcttgt attataatta attttctacc cccagagata aatgtttgtt        5540

tatatcactg tctagctgtt tcaaaattta ggtcccttgg tctgtacaaa taatagcaat        5600

gtaaaaatgg ttttttgaac ctccaaatgg aattacagac tcagtagcca tatcttccaa        5660

ccccccagta taaatttctg tctttctgct atgtgtggta ctttgcagct gcttttgcag        5720

aaatcacaat tttcctgtgg aataaagatg gtccaaaaat agtcaaaaat taaatatata        5780

tatatattag taatttatat agatgtcagc aattaggcag atcaaggttt agtttaactt        5840

ccactgttaa aataaagctt acatagtttt cttcctttga aagactgtgc tgtcctttaa        5900

cataggtttt taaagactag gatattgaat gtgaaacatc cgttttcatt gttcacttct        5960

aaaccaaaaa ttatgtgttg ccaaaaccaa acccaggttc atgaatatgg tgtctattat        6020

agtgaaacat gtactttgag cttattgttt ttattctgta ttaaatattt tcagggtttt        6080

aaacactaat cacaaactga atgacttgac ttcaaaagca acaaccttaa aggccgtcat        6140

ttcattagta ttcctcattc tgcatcctgg cttgaaaaac agctctgttg aatcacagta        6200

tcagtatttt cacacgtaag cacattcggg ccatttccgt ggtttctcat gagctgtgtt        6260

cacagacctc agcagggcat cgcatggacc gcaggagggc agattcggac cactaggcct        6320

gaaatgacat ttcactaaaa gtctccaaaa catttctaag actactaagg ccttttatgt 6380

aatttcttta aatgtgtatt tcttaagaat tcaaatttgt aataaaacta tttgtataaa 6440

aattaagctt ttattaattt gttgctagta ttgccacaga cgcattaaaa gaaacttact 6500

gcacaagctg ctaataaatt tgtaagcttt gcatacctta 6540

<210> 2
<211> 1669
<212> PRT
<213> Homo sapiens

<400> 2

Met Gly Pro Arg Leu Ser Val Trp Leu Leu Leu Leu Pro Ala Ala Leu
1               5                   10                  15

Leu Leu His Glu Glu His Ser Arg Ala Ala Ala Lys Gly Gly Cys Ala
            20                  25                  30

Gly Ser Gly Cys Gly Lys Cys Asp Cys His Gly Val Lys Gly Gln Lys
        35                  40                  45

Gly Glu Arg Gly Leu Pro Gly Leu Gln Gly Val Ile Gly Phe Pro Gly
    50                  55                  60

Met Gln Gly Pro Glu Gly Pro Gln Gly Pro Pro Gly Gln Lys Gly Asp
65                  70                  75                  80

Thr Gly Glu Pro Gly Leu Pro Gly Thr Lys Gly Thr Arg Gly Pro Pro
                85                  90                  95

Gly Ala Ser Gly Tyr Pro Gly Asn Pro Gly Leu Pro Gly Ile Pro Gly
            100                 105                 110

Gln Asp Gly Pro Pro Gly Pro Pro Gly Ile Pro Gly Cys Asn Gly Thr
        115                 120                 125

Lys Gly Glu Arg Gly Pro Leu Gly Pro Pro Gly Leu Pro Gly Phe Ala
    130                 135                 140

Gly Asn Pro Gly Pro Pro Gly Leu Pro Gly Met Lys Gly Asp Pro Gly
145                 150                 155                 160

Glu Ile Leu Gly His Val Pro Gly Met Leu Leu Lys Gly Glu Arg Gly
                165                 170                 175

Phe Pro Gly Ile Pro Gly Thr Pro Gly Pro Pro Gly Leu Pro Gly Leu
            180                 185                 190

Gln Gly Pro Val Gly Pro Pro Gly Phe Thr Gly Pro Pro Gly Pro Pro
        195                 200                 205

Gly Pro Pro Gly Pro Pro Gly Glu Lys Gly Gln Met Gly Leu Ser Phe
    210                 215                 220

Gln Gly Pro Lys Gly Asp Lys Gly Asp Gln Gly Val Ser Gly Pro Pro
225                 230                 235                 240

Gly Val Pro Gly Gln Ala Gln Val Gln Glu Lys Gly Asp Phe Ala Thr
                245                 250                 255

Lys Gly Glu Lys Gly Gln Lys Gly Glu Pro Gly Phe Gln Gly Met Pro
            260                 265                 270

Gly Val Gly Glu Lys Gly Glu Pro Gly Lys Pro Gly Pro Arg Gly Lys
        275                 280                 285

Pro Gly Lys Asp Gly Asp Lys Gly Glu Lys Gly Ser Pro Gly Phe Pro
    290                 295                 300

Gly Glu Pro Gly Tyr Pro Gly Leu Ile Gly Arg Gln Gly Pro Gln Gly
305                 310                 315                 320

Glu Lys Gly Glu Ala Gly Pro Pro Gly Pro Pro Gly Ile Val Ile Gly
                325                 330                 335

Thr Gly Pro Leu Gly Glu Lys Gly Glu Arg Gly Tyr Pro Gly Thr Pro
            340                 345                 350

Gly Pro Arg Gly Glu Pro Gly Pro Lys Gly Phe Pro Gly Leu Pro Gly
        355                 360                 365

Gln Pro Gly Pro Pro Gly Leu Pro Val Pro Gly Gln Ala Gly Ala Pro
    370                 375                 380

Gly Phe Pro Gly Glu Arg Gly Glu Lys Gly Asp Arg Gly Phe Pro Gly
385                 390                 395                 400

Thr Ser Leu Pro Gly Pro Ser Gly Arg Asp Gly Leu Pro Gly Pro Pro
                405                 410                 415

Gly Ser Pro Gly Pro Pro Gly Gln Pro Gly Tyr Thr Asn Gly Ile Val
            420                 425                 430

Glu Cys Gln Pro Gly Pro Pro Gly Asp Gln Gly Pro Pro Gly Ile Pro

435                 440                 445

Gly Gln Pro Gly Phe Ile Gly Glu Ile Gly Glu Lys Gly Gln Lys Gly
    450                 455                 460

Glu Ser Cys Leu Ile Cys Asp Ile Asp Gly Tyr Arg Gly Pro Pro Gly
465                 470                 475                 480

Pro Gln Gly Pro Pro Gly Glu Ile Gly Phe Pro Gly Gln Pro Gly Ala
                485                 490                 495

Lys Gly Asp Arg Gly Leu Pro Gly Arg Asp Gly Val Ala Gly Val Pro
            500                 505                 510

Gly Pro Gln Gly Thr Pro Gly Leu Ile Gly Gln Pro Gly Ala Lys Gly
        515                 520                 525

Glu Pro Gly Glu Phe Tyr Phe Asp Leu Arg Leu Lys Gly Asp Lys Gly
    530                 535                 540

Asp Pro Gly Phe Pro Gly Gln Pro Gly Met Pro Gly Arg Ala Gly Ser
545                 550                 555                 560

Pro Gly Arg Asp Gly His Pro Gly Leu Pro Gly Pro Lys Gly Ser Pro
                565                 570                 575

Gly Ser Val Gly Leu Lys Gly Glu Arg Gly Pro Pro Gly Gly Val Gly
            580                 585                 590

Phe Pro Gly Ser Arg Gly Asp Thr Gly Pro Pro Gly Pro Pro Gly Tyr
        595                 600                 605

Gly Pro Ala Gly Pro Ile Gly Asp Lys Gly Gln Ala Gly Phe Pro Gly
    610                 615                 620

Gly Pro Gly Ser Pro Gly Leu Pro Gly Pro Lys Gly Glu Pro Gly Lys
625                 630                 635                 640

Ile Val Pro Leu Pro Gly Pro Pro Gly Ala Glu Gly Leu Pro Gly Ser
                645                 650                 655

Pro Gly Phe Pro Gly Pro Gln Gly Asp Arg Gly Phe Pro Gly Thr Pro
            660                 665                 670

Gly Arg Pro Gly Leu Pro Gly Glu Lys Gly Ala Val Gly Gln Pro Gly
        675                 680                 685

Ile Gly Phe Pro Gly Pro Pro Gly Pro Lys Gly Val Asp Gly Leu Pro
    690                 695                 700

Gly Asp Met Gly Pro Pro Gly Thr Pro Gly Arg Pro Gly Phe Asn Gly
705                 710                 715                 720

Leu Pro Gly Asn Pro Gly Val Gln Gly Gln Lys Gly Glu Pro Gly Val
                725                 730                 735

Gly Leu Pro Gly Leu Lys Gly Leu Pro Gly Leu Pro Gly Ile Pro Gly
            740                 745                 750

Thr Pro Gly Glu Lys Gly Ser Ile Gly Val Pro Gly Val Pro Gly Glu
        755                 760                 765

His Gly Ala Ile Gly Pro Pro Gly Leu Gln Gly Ile Arg Gly Glu Pro
    770                 775                 780

Gly Pro Pro Gly Leu Pro Gly Ser Val Gly Ser Pro Gly Val Pro Gly
785                 790                 795                 800

Ile Gly Pro Pro Gly Ala Arg Gly Pro Pro Gly Gly Gln Gly Pro Pro
                805                 810                 815

Gly Leu Ser Gly Pro Pro Gly Ile Lys Gly Glu Lys Gly Phe Pro Gly
            820                 825                 830

Phe Pro Gly Leu Asp Met Pro Gly Pro Lys Gly Asp Lys Gly Ala Gln
        835                 840                 845

Gly Leu Pro Gly Ile Thr Gly Gln Ser Gly Leu Pro Gly Leu Pro Gly
    850                 855                 860

Gln Gln Gly Ala Pro Gly Ile Pro Gly Phe Pro Gly Ser Lys Gly Glu
865                 870                 875                 880

Met Gly Val Met Gly Thr Pro Gly Gln Pro Gly Ser Pro Gly Pro Val
                885                 890                 895

Gly Ala Pro Gly Leu Pro Gly Glu Lys Gly Asp His Gly Phe Pro Gly
            900                 905                 910

Ser Ser Gly Pro Arg Gly Asp Pro Gly Leu Lys Gly Asp Lys Gly Asp
        915                 920                 925

Val Gly Leu Pro Gly Lys Pro Gly Ser Met Asp Lys Val Asp Met Gly
    930                 935                 940

Ser Met Lys Gly Gln Lys Gly Asp Gln Gly Glu Lys Gly Gln Ile Gly
945                 950                 955                 960

Pro Ile Gly Glu Lys Gly Ser Arg Gly Asp Pro Gly Thr Pro Gly Val
                965                 970                 975

Pro Gly Lys Asp Gly Gln Ala Gly Gln Pro Gly Gln Pro Gly Pro Lys
            980                 985                 990

Gly Asp Pro Gly Ile Ser Gly Thr  Pro Gly Ala Pro Gly  Leu Pro Gly
        995                 1000                 1005

Pro Lys  Gly Ser Val Gly Gly  Met Gly Leu Pro Gly  Thr Pro Gly
    1010                 1015                 1020

Glu Lys  Gly Val Pro Gly Ile  Pro Gly Pro Gln Gly  Ser Pro Gly
    1025                 1030                 1035

Leu Pro  Gly Asp Lys Gly Ala  Lys Gly Glu Lys Gly  Gln Ala Gly
    1040                 1045                 1050

Pro Pro  Gly Ile Gly Ile Pro  Gly Leu Arg Gly Glu  Lys Gly Asp
    1055                 1060                 1065

Gln Gly  Ile Ala Gly Phe Pro  Gly Ser Pro Gly Glu  Lys Gly Glu
    1070                 1075                 1080

Lys Gly  Ser Ile Gly Ile Pro  Gly Met Pro Gly Ser  Pro Gly Leu
    1085                 1090                 1095

Lys Gly  Ser Pro Gly Ser Val  Gly Tyr Pro Gly Ser  Pro Gly Leu
    1100                 1105                 1110

Pro Gly  Glu Lys Gly Asp Lys  Gly Leu Pro Gly Leu  Asp Gly Ile
    1115                 1120                 1125

Pro Gly  Val Lys Gly Glu Ala  Gly Leu Pro Gly Thr  Pro Gly Pro
    1130                 1135                 1140

Thr Gly  Pro Ala Gly Gln Lys  Gly Glu Pro Gly Ser  Asp Gly Ile
    1145                 1150                 1155

Pro Gly  Ser Ala Gly Glu Lys  Gly Glu Pro Gly Leu  Pro Gly Arg
    1160                 1165                 1170

Gly Phe  Pro Gly Phe Pro Gly  Ala Lys Gly Asp Lys  Gly Ser Lys
    1175                 1180                 1185

Gly Glu Val Gly Phe Pro Gly Leu Ala Gly Ser Pro Gly Ile Pro
    1190                1195                1200

Gly Ser Lys Gly Glu Gln Gly Phe Met Gly Pro Pro Gly Pro Gln
    1205                1210                1215

Gly Gln Pro Gly Leu Pro Gly Ser Pro Gly His Ala Thr Glu Gly
    1220                1225                1230

Pro Lys Gly Asp Arg Gly Pro Gln Gly Gln Pro Gly Leu Pro Gly
    1235                1240                1245

Leu Pro Gly Pro Met Gly Pro Pro Gly Leu Pro Gly Ile Asp Gly
    1250                1255                1260

Val Lys Gly Asp Lys Gly Asn Pro Gly Trp Pro Gly Ala Pro Gly
    1265                1270                1275

Val Pro Gly Pro Lys Gly Asp Pro Gly Phe Gln Gly Met Pro Gly
    1280                1285                1290

Ile Gly Gly Ser Pro Gly Ile Thr Gly Ser Lys Gly Asp Met Gly
    1295                1300                1305

Pro Pro Gly Val Pro Gly Phe Gln Gly Pro Lys Gly Leu Pro Gly
    1310                1315                1320

Leu Gln Gly Ile Lys Gly Asp Gln Gly Asp Gln Gly Val Pro Gly
    1325                1330                1335

Ala Lys Gly Leu Pro Gly Pro Pro Gly Pro Pro Gly Pro Tyr Asp
    1340                1345                1350

Ile Ile Lys Gly Glu Pro Gly Leu Pro Gly Pro Glu Gly Pro Pro
    1355                1360                1365

Gly Leu Lys Gly Leu Gln Gly Leu Pro Gly Pro Lys Gly Gln Gln
    1370                1375                1380

Gly Val Thr Gly Leu Val Gly Ile Pro Gly Pro Pro Gly Ile Pro
    1385                1390                1395

Gly Phe Asp Gly Ala Pro Gly Gln Lys Gly Glu Met Gly Pro Ala
    1400                1405                1410

Gly Pro Thr Gly Pro Arg Gly Phe Pro Gly Pro Pro Gly Pro Asp

1415                1420                1425

Gly Leu Pro Gly Ser Met Gly Pro Pro Gly Thr Pro Ser Val Asp
    1430                1435                1440

His Gly Phe Leu Val Thr Arg His Ser Gln Thr Ile Asp Asp Pro
    1445                1450                1455

Gln Cys Pro Ser Gly Thr Lys Ile Leu Tyr His Gly Tyr Ser Leu
    1460                1465                1470

Leu Tyr Val Gln Gly Asn Glu Arg Ala His Gly Gln Asp Leu Gly
    1475                1480                1485

Thr Ala Gly Ser Cys Leu Arg Lys Phe Ser Thr Met Pro Phe Leu
    1490                1495                1500

Phe Cys Asn Ile Asn Asn Val Cys Asn Phe Ala Ser Arg Asn Asp
    1505                1510                1515

Tyr Ser Tyr Trp Leu Ser Thr Pro Glu Pro Met Pro Met Ser Met
    1520                1525                1530

Ala Pro Ile Thr Gly Glu Asn Ile Arg Pro Phe Ile Ser Arg Cys
    1535                1540                1545

Ala Val Cys Glu Ala Pro Ala Met Val Met Ala Val His Ser Gln
    1550                1555                1560

Thr Ile Gln Ile Pro Pro Cys Pro Ser Gly Trp Ser Ser Leu Trp
    1565                1570                1575

Ile Gly Tyr Ser Phe Val Met His Thr Ser Ala Gly Ala Glu Gly
    1580                1585                1590

Ser Gly Gln Ala Leu Ala Ser Pro Gly Ser Cys Leu Glu Glu Phe
    1595                1600                1605

Arg Ser Ala Pro Phe Ile Glu Cys His Gly Arg Gly Thr Cys Asn
    1610                1615                1620

Tyr Tyr Ala Asn Ala Tyr Ser Phe Trp Leu Ala Thr Ile Glu Arg
    1625                1630                1635

Ser Glu Met Phe Lys Lys Pro Thr Pro Ser Thr Leu Lys Ala Gly
    1640                1645                1650

Glu Leu Arg Thr His Val Ser Arg Cys Gln Val Cys Met Arg Arg
    1655                1660                1665

Thr

<210>  3
<211>  6446
<212>  DNA
<213>  Homo sapiens

<220>
<221>  CDS
<222>  (290)..(5428)

<220>
<221>  sig_peptide
<222>  (290)..(388)

<400>  3
gagtgtggct gcagtgcgcc gggacaccag ggctccgcgc tccgcactca agaggctccc      60

gcgtcccaac ccctcgcgcc cgcgcgttcg cggatccagg ccgaggaccg aaaggggccg     120

cccgagcccc cggggccggc gcccagagag cccagcaagg ccggccgccc tgccggtgtg     180

ccgccggcgg gtgcttctgg aaggccaatt gcgttcgggc agcagcccct gaagccgagc     240

ccgaggctaa gtgggactga ccggggccca gagtggacga accgccagc atg ggg aga     298
                                                      
                                                      1

gac cag cgc gcg gtg gcc ggc cct gcc cta cgg cgg tgg ctg ctg ctg       346

    5                   10                  15

ggg aca gtg acc gtg ggg ttc ctc gcc cag agc gtc ttg gcg ggt gtg       394

20                  25                  30                  35

aag aag ttt gat gtg ccg tgt gga gga aga gat tgc agt ggg ggc tgc       442

                40                  45                  50

cag tgc tac cct gag aaa ggt gga cgt ggt cag cct ggg cca gtg ggc       490

            55                  60                  65

ccc cag ggg tac aat ggg cca cca gga tta caa gga ttc ccg gga ctg       538

        70                  75                  80

cag gga cgt aaa gga gac aag ggt gaa agg gga gcc ccc gga gta acg       586

    85                  90                  95

gga ccc aag ggc gac gtg gga gca aga ggc gtt tct gga ttc cct ggt       634

100                 105                 110                 115

gcc gat gga att cct gga cac ccg ggg caa ggt ggg ccc agg gga agg 682

                120                 125                 130

ccg ggc tac gat ggc tgc aac gga acc cag gga gac tca ggt cca cag 730

            135                 140                 145

ggg ccc ccc ggc tct gag ggg ttc acc ggg cct ccc ggg ccc caa gga 778

        150                 155                 160

cca aaa ggg cag aaa ggt gag cct tat gca ctg cct aaa gag gag cgc 826

    165                 170                 175

gac aga tat cgg ggt gaa cct gga gag cct gga ttg gtc ggt ttc cag 874

180                 185                 190                 195

gga cct ccc ggc cgc cct ggg cat gtg gga cag atg ggt cca gtt gga 922

                200                 205                 210

gct cca ggg aga cca gga cca cct gga ccc cct gga cca aaa gga cag 970

            215                 220                 225

caa ggc aac aga gga ctt ggt ttc tac gga gtt aag ggt gaa aag ggt 1018

        230                 235                 240

gac gta ggg cag ccg gga ccc aac ggg att cca tca gac acc ctc cac 1066

    245                 250                 255

ccc atc atc gcg ccc aca gga gtc acc ttc cac cca gat cag tac aag 1114

260                 265                 270                 275

ggt gaa aaa ggc agt gag ggg gaa cca gga ata aga ggc att tcc ttg 1162

                280                 285                 290

aag gga gaa gaa gga atc atg ggc ttt cct gga ctg agg ggt tac cct 1210

            295                 300                 305

ggc ttg agt ggt gaa aaa gga tca cca gga cag aag gga agc cga ggc 1258

        310                 315                 320

ctg gat ggc tat caa ggg cct gat gga ccc cgg gga ccc aag gga gaa 1306

    325                 330                 335

gcc gga gac cca ggg ccc cct gga cta cct gcc tac tcc cct cac cct 1354

340                 345                 350                 355

tcc cta gca aaa ggt gcc aga ggt gac ccg gga ttc cca ggg gcc caa 1402

                360                 365                 370

ggg gag cca gga agc cag ggt gag cca gga gac ccg ggc ctc cca ggt     1450

            375                 380                 385

ccc cct ggc ctc tcc atc gga gat gga gat cag agg aga ggc ctg ccg     1498

        390                 395                 400

ggt gag atg gga ccc aag ggc ttc atc gga gac ccc ggc atc cct gcg     1546

    405                 410                 415

ctc tac ggg ggc cca cct gga cct gat gga aag cga ggg cct cca gga     1594

420                 425                 430                 435

ccc ccc ggg ctc cct gga cca cct gga cct gat ggc ttc ctg ttt ggg     1642

                440                 445                 450

ctg aaa gga gca aaa gga aga gca ggc ttc cct ggg ctt ccc ggc tcc     1690

            455                 460                 465

cct gga gcc cgc gga cca aag ggg tgg aaa ggt gac gct ggg gaa tgc     1738

        470                 475                 480

aga tgt aca gaa ggc gac gaa gct atc aaa ggt ctt ccg gga ctg cca     1786

    485                 490                 495

gga ccc aag ggc ttc gca ggc atc aac ggg gag ccg ggg agg aaa ggg     1834

500                 505                 510                 515

gac aga gga gac ccc ggc caa cac ggc ctc cct ggg ttc cca ggg ctc     1882

                520                 525                 530

aag gga gtg cct ggc aac att ggt gct ccc gga ccc aaa gga gca aaa     1930

            535                 540                 545

gga gat tcc aga aca atc aca acc aaa ggt gag cgg gga cag ccc ggc     1978

        550                 555                 560

gtc cca ggt gtg ccc ggg atg aaa ggt gac gat ggc agc cca ggc cgc     2026

    565                 570                 575

gat ggg ctc gat gga ttc ccc ggc ctc cca ggc cct ccc ggt gat ggc     2074

580                 585                 590                 595

atc aag ggc cct cca ggg gac cca ggc tat cca gga ata cct gga acg     2122

                600                 605                 610

aag ggt act cca gga gaa atg ggc ccc cca gga ctg ggc ctt ccc ggc     2170


615                 620                 625

ctc aaa ggc caa cgt ggt ttc cct gga gac gcc ggc tta cct gga cca        2218

        630                 635                 640

cca ggc ttc ctg ggc cct cct ggc ccc gca ggg acc cca gga caa ata        2266

    645                 650                 655

gat tgt gac aca gat gtg aaa agg gcc gtt gga ggt gac aga cag gag        2314

660                 665                 670                 675

gcc atc cag cca ggt tgc ata gga ggg ccc aag gga ttg cca ggc ctg        2362

                680                 685                 690

cca gga ccc cca ggc ccc aca ggt gcc aaa ggc ctc cga gga atc cca        2410

            695                 700                 705

ggc ttc gca gga gct gat gga gga cca ggg ccc agg ggc ttg cca gga        2458

        710                 715                 720

gac gca ggt cgt gaa ggg ttc cca gga ccc cca ggg ttc ata gga ccc        2506

    725                 730                 735

cga gga tcc aaa ggt gca gtg ggc ctc cct ggc cca gat gga tcc cca        2554

740                 745                 750                 755

ggt ccc atc ggc ctg cca ggg cca gat ggg ccc cct ggg gaa agg ggc        2602

                760                 765                 770

ctc cct gga gaa gtc ctg gga gct cag ccc ggg cca cgg gga gat gct        2650

            775                 780                 785

ggt gtg cct gga cag cct ggg ctt aaa ggc ctt ccc gga gac aga ggc        2698

        790                 795                 800

ccc cct gga ttc aga gga agc caa ggg atg cct ggg atg cca ggg ctg        2746

    805                 810                 815

aag ggc cag cca ggc ctc cca gga cct tcc ggc cag cca ggc ctg tat        2794

820                 825                 830                 835

ggg cct cca gga ctg cat gga ttc cca gga gct cct ggc caa gag ggg        2842

                840                 845                 850

ccc ttg ggg ctg cca gga atc cca ggc cgt gaa ggt ctg cct ggt gat        2890

            855                 860                 865

aga ggg gac cct ggg gac aca ggc gct cct ggc cct gtg ggc atg aaa        2938


        870                 875                 880

ggt ctc tct ggt gac aga gga gat gct ggc ttc aca ggg gag caa ggc        2986

    885                 890                 895

cat cca gga agc cct gga ttt aaa gga att gat gga atg cct ggg acc        3034

900                 905                 910                 915

ccc ggg cta aaa gga gat aga ggc tca cct ggg atg gat ggt ttc caa        3082

                920                 925                 930

ggc atg cct gga ctc aaa ggg aga ccc ggg ttt cca ggg agc aaa ggc        3130

            935                 940                 945

gag gct gga ttt ttc gga ata ccc ggt ctg aag ggt ctg gct ggt gag        3178

        950                 955                 960

cca ggt ttt aaa ggc agc cga ggg gac cct ggg ccc cca gga cca cct        3226

    965                 970                 975

cct gtc atc ctg cca gga atg aaa gac att aaa gga gag aaa gga gat        3274

980                 985                 990                 995

gaa ggg cct atg ggg  ctg aaa gga tac ctg  ggc gca aaa ggt atc        3319

                1000                 1005                 1010

caa gga atg cca ggc  atc cca ggg ctg tca  gga atc cct ggg ctg        3364

                1015                 1020                 1025

cct ggg agg ccc ggc  cac atc aaa gga gtc  aag gga gac atc gga        3409

                1030                 1035                 1040

gtc ccc ggc atc ccc  ggt ttg cca gga ttc  cct ggg gtg gct ggc        3454

                1045                 1050                 1055

ccc cct gga att acg  gga ttc cca gga ttc  ata gga agc cgg ggt        3499

                1060                 1065                 1070

gac aaa ggt gcc cca  ggg aga gca ggc ctg  tat ggc gag att ggc        3544

                1075                 1080                 1085

gcg act ggt gat ttc  ggt gac atc ggg gac  act ata aat tta cca        3589

                1090                 1095                 1100

gga aga cca ggc ctg  aag ggg gag cgg ggc  acc act gga ata cca        3634

                1105                 1110                 1115

ggt ctg aag gga ttc  ttt gga gag aag gga  aca gaa ggt gac atc  3679

                1120                 1125                 1130

ggc ttc cct ggg ata  aca ggc gtg act gga  gtc caa ggc cct cct  3724

                1135                 1140                 1145

gga ctt aaa gga caa  aca ggc ttt cca ggg  ctg act ggg cct cca  3769

                1150                 1155                 1160

ggg tcg cag gga gag  ctg ggg cgg att gga  ctg cct ggt ggc aaa  3814

                1165                 1170                 1175

gga gat gat ggc tgg  ccg gga gct ccg ggc  tta cca ggt ttt ccg  3859

                1180                 1185                 1190

gga ctc cgt ggg atc  cgc ggc tta cac ggc  ttg cca ggc acc aag  3904

                1195                 1200                 1205

ggc ttt cca gga tcc  cca ggt tct gac atc  cac gga gac cca ggc  3949

                1210                 1215                 1220

ttc cca ggc cct cct  ggg gaa aga ggt gac  cca gga gag gcc aac  3994

                1225                 1230                 1235

acc ctt cca ggc cct  gtg gga gtc cca gga  cag aaa gga gac caa  4039

                1240                 1245                 1250

gga gct cca ggg gaa  cga ggc cca cct ggg  agc cca gga ctt cag  4084

                1255                 1260                 1265

ggg ttc cct ggt atc  aca ccc cct tcc aac  atc tct ggg gca cct  4129

                1270                 1275                 1280

ggt gac aaa ggg gcg  cca ggg ata ttt ggc  ctg aaa ggt tat cgg  4174

                1285                 1290                 1295

ggc cca cca ggg cca  cca ggt tct gct gct  ctt cct gga agc aaa  4219

                1300                 1305                 1310

ggt gac aca ggg aac  cca gga gct cca gga  acc cca ggg acc aaa  4264

                1315                 1320                 1325

gga tgg gcc ggg gac  tcc ggg ccc cag ggc  agg cct ggt gtg ttt  4309

                1330                 1335                 1340

ggt ctc cca gga gaa  aaa ggg ccc agg ggt  gaa caa ggc ttc atg  4354

                1345                 1350                 1355

ggg aac act gga ccc act ggg gcg gtg ggc gac aga ggc ccc aag 4399

                1360                1365                1370

gga ccc aag gga gac cca gga ttc cct ggt gcc ccc ggg act gtg 4444

                1375                1380                1385

gga gcc ccc ggg att gca gga atc ccc cag aag att gcc gtc caa 4489

                1390                1395                1400

cca ggg aca gtg ggt ccc cag ggg agg cga ggc ccc cct ggg gca 4534

                1405                1410                1415

ccg ggg gag atg ggg ccc cag ggc ccc ccc gga gaa cca ggt ttc 4579

                1420                1425                1430

cgt ggg gct cca ggg aaa gct ggg ccc caa gga aga ggt ggt gtg 4624

                1435                1440                1445

tct gct gtt ccc ggc ttc cgg gga gat gaa gga ccc ata ggc cac 4669

                1450                1455                1460

cag ggg ccg att ggc caa gaa ggt gca cca ggc cgt cca ggg agc 4714

                1465                1470                1475

ccg ggc ctg ccg ggt atg cca ggc cgc agc gtc agc atc ggc tac 4759

                1480                1485                1490

ctc ctg gtg aag cac agc cag acg gac cag gag ccc atg tgc cca 4804

                1495                1500                1505

gtg ggc atg aac aaa ctc tgg agt gga tac agc ctg ctg tac ttc 4849

                1510                1515                1520

gag ggc cag gag aag gcg cac aac cag gac ctg ggg ctg gcg ggc 4894

                1525                1530                1535

tcc tgc ctg gcg cgg ttc agc acc atg ccc ttc ctg tac tgc aac 4939

                1540                1545                1550

cct ggt gat gtc tgc tac tat gcc agc cgg aac gac aag tcc tac 4984

                1555                1560                1565

tgg ctc tct acc act gcg ccg ctg ccc atg atg ccc gtg gcc gag 5029

                1570                1575                1580

gac gag atc aag ccc tac atc agc cgc tgt tct gtg tgt gag gcc 5074


1585                1590                1595

ccg gcc atc gcc atc  gcg gtc cac agt cag  gat gtc tcc atc cca      5119

                1600                1605                1610

cac tgc cca gct ggg  tgg cgg agt ttg tgg  atc gga tat tcc ttc      5164

                1615                1620                1625

ctc atg cac acg gcg  gcg gga gac gaa ggc  ggt ggc caa tca ctg      5209

                1630                1635                1640

gtg tca ccg ggc agc  tgt cta gag gac ttc  cgc gcc aca cca ttc      5254

                1645                1650                1655

atc gaa tgc aat gga  ggc cgc ggc acc tgc  cac tac tac gcc aac      5299

                1660                1665                1670

aag tac agc ttc tgg  ctg acc acc att ccc  gag cag agc ttc cag      5344

                1675                1680                1685

ggc tcg ccc tcc gcc  gac acg ctc aag gcc  ggc ctc atc cgc aca      5389

                1690                1695                1700

cac atc agc cgc tgc  cag gtg tgc atg aag  aac ctg tga gccggcgcgt      5438

                1705                1710

gccaggaagg gccattttgg tgcttattct taacttatta cctcaggtgc caacccaaaa      5498

attggtttta tttttttctt aaaaaaaaaa aagtctacca aaggaatttg catccagcag      5558

cagcacttag acctgccagc cactgtcacc gagcgggtgc aagcactcgg ggtccctgga      5618

gggcaagccc tgcccacaga aagccaggag cagccctggc ccccatcagc cctgctagac      5678

gcaccgcctg aaggcacagc taaccacttc gcacacaccc atgtaaccac tgcactttcc      5738

aatgccacag acaactcaca ttgttcaact cccttctcgg ggtgggacag acgagacaac      5798

agcacacagg cagccagccg tggccagagg ctcgaggggc tcagggcctc aggcacccgt      5858

ccccacacga gggccccgtg ggtgggcctg gccctgcttt ctacgccaat gttatgccag      5918

ctccatgttc tcccaaatac cgttgatgtg aattatttta aaggcaaaac cgtgctcttt      5978

attttaaaaa acactgataa tcacactgcg gtaggtcatt cttttgccac atccctatag      6038

accactgggt ttggcaaaac tcaggcagaa gtggagacct ttctagacat cattgtcagc      6098

cttgctactt gaaggtacac cccatagggt cggaggtgct gtccccactg ccccacgttg      6158

tccctgagat ttaacccctc cactgctggg ggtgagctgt actcttctga ctgccccctc      6218

ctgtgtaacg actacaaaat aaaacttggt tctgaatatt tttaaacccc gagttgttga      6278

ccgccttaat ctcgtgtcca tagagcaaaa cgtctgctca gatggatgcg aggcacagcg      6338

tccgcccacg ctgctgtttt taatccatct cagtagagtt gaacccattc gtggtattac 6398

agccatttct cggggaatgt gtttgtttat aactcactaa tgcttaca 6446

<210> 4
<211> 1712
<212> PRT
<213> Homo sapiens

<400> 4

<210>  5
<211>  8114
<212>  DNA
<213>  Homo sapiens

<220>
<221>  CDS
<222>  (163)..(5175)

<220>
<221>  sig_peptide
<222>  (163)..(246)

<400>  5
gggagggacg aaccgcgcga ccgagcccta caaaacccgc cccggccgag tggcgaggcg      60
agctttccag ccgggctccc agagccgcgc tgcgcaggag acgcggtggc ctgagagcct     120
gagggtcccc ggactcgccc aggctctgag cgcgcgccca cc atg agc gcc cgg        174
acc gcc ccc agg ccg cag gtg ctc ctg ctg ccg ctc ctg ctg gtg ctc       222
ctg gcg gcg gcg ccc gca gcc agc aag ggt tgt gtc tgt aaa gac aaa       270
ggc cag tgc ttc tgt gac ggg gcc aaa ggg gag aag ggg gag aag ggc       318
ttt cct gga ccc ccc ggt tct cct ggc cag aaa gga ttc aca ggt cct       366
gaa ggc ttg cct gga ccg cag gga ccc aag ggc ttt cca gga ctt cca       414
gga ctc acg ggt tcc aaa ggt gta agg gga ata agt gga ttg cca gga       462
ttt tct ggt tct cct gga ctt cca ggc acc cca ggc aat acc ggg cct       510
tac gga ctt gtc ggt gta cca gga tgc agt ggt tct aag ggt gag cag 558
ggg ttt cca gga ctc cca ggg aca ctg ggc tac cca ggg atc ccg ggt 606
gct gct ggt ttg aaa gga caa aag ggt gct cct gct aaa gaa gaa gat 654
ata gaa ctt gat gca aaa ggc gac ccc ggg ttg cca ggg gct cca gga 702
ccc cag ggt ttg cca ggc cct cca ggt ttt cct ggg cct gtt ggc cca 750
cct ggt cct ccg gga ttc ttt ggc ttt cca gga gcc atg gga cct aga 798
gga cct aag ggt cac atg ggt gaa aga gtg ata gga cat aaa gga gag 846
cgg ggt gtg aaa ggg tta aca gga ccc ccg gga cca cca gga aca gtt 894
att gtg acc cta act ggc cca gat aac aga acg gac ctc aag ggg gaa 942
aag gga gac aag gga gca atg ggc gag cct gga cct cct gga ccc tca 990
gga ctg cct gga gaa tca tat gga tct gaa aag ggt gct cct gga gac 1038
cct ggc ctg cag gga aaa ccc gga aaa gat ggt gtt cct ggc ttc cct 1086
gga agt gag gga gtc aag ggc aac agg ggt ttc cct ggg tta atg ggt 1134
gaa gat ggc att aag gga cag aaa ggg gac att ggc cct cca gga ttt 1182
cgt ggt cca aca gaa tat tat gac aca tac cag gaa aag gga gat gaa 1230
ggc act cca ggc cca cca ggg ccc aga gga gct cgt ggc cca caa ggt 1278
ccc agt ggt ccc ccc gga gtt cct gga agt cct gga tca tca agg cct 1326
ggc ctc aga gga gcc cct gga tgg cca ggc ctg aaa gga agt aaa ggg 1374
gaa cga ggc cgc cca gga aag gat gcc atg ggg act cct ggg tcc cca 1422
ggt tgt gct ggt tca cca ggt ctt cca gga tca ccg gga cct cca gga 1470
ccg cca ggt gac atc gtt ttt cgc aag ggt cca cct gga gat cac gga 1518
ctg cca ggc tat cta ggg tct cca gga atc cca gga gtt gat ggg ccc 1566
aaa gga gaa cca ggc ctc ctg tgt aca cag tgc cct tat atc cca ggg 1614
cct ccc ggt ctc cca gga ttg cca ggg tta cat ggt gta aaa gga atc 1662
cca gga aga caa ggc gca gct ggc ttg aaa gga agc cca ggg tcc cca 1710
gga aat aca ggt ctt cca gga ttt cca ggt ttc cca ggt gcc cag ggt 1758
gac cca gga ctt aaa gga gaa aaa ggt gaa aca ctt cag cct gag ggg 1806
caa gtg ggt gtc cca ggt gac ccg ggg ctc aga ggc caa cct ggg aga 1854
aag ggc ttg gat gga att cct gga act ccg gga gtg aaa gga tta cca 1902
gga cct aaa ggc gaa ctg gct ctg agt ggt gag aaa ggg gac caa ggt 1950
cct cca ggg gat cct ggc tcc cct ggg tcc cca gga cct gca gga cca 1998
gct gga cca cct ggc tac gga ccc caa gga gaa cct ggt ctc cag ggc 2046
acg caa gga gtt cct gga gcc ccc gga cca ccc gga gaa gcc ggc cct          2094
agg gga gag ctc agt gtt tca aca cca gtt cca ggc cca cca gga cct          2142
cca ggg ccc cct ggc cat cct ggc ccc caa ggt cca cct ggt atc cct          2190
gga tcc ctg ggg aaa tgt gga gat cct ggt ctt cca ggg cct gat ggt          2238
gaa cca gga att cca gga att gga ttt cct ggg cct cct gga cct aag          2286
gga gac caa ggt ttt cca ggt aca aaa gga tca ctg ggt tgt cct gga          2334
aaa atg gga gag cct ggg tta cct gga aag cca ggc ctc cca gga gcc          2382
aag gga gaa cca gca gta gcc atg cct gga gga cca gga aca cca ggt          2430
ttt cca gga gaa aga ggc aat tct ggg gaa cat gga gaa att gga ctc          2478
cct gga ctt cca ggt ctc cct gga act cca gga aat gaa ggg ctt gat          2526
gga cca cga gga gat cca ggg cag cct gga cca cct gga gaa caa gga          2574
ccc cca gga agg tgc ata gag ggt ccc agg gga gcc caa gga ctt cca          2622
ggc tta aat gga ttg aaa ggg caa caa ggc aga aga ggt aaa acg ggg          2670
cca aag gga gac cca gga att cca ggc ttg gat aga tca gga ttt cct          2718
gga gaa act gga tca cca gga att cca ggt cat caa ggt gaa atg gga          2766
cca ctg ggt caa aga gga tat cca gga aat ccg gga att tta ggg cca          2814
cca ggt gaa gat gga gtg att ggg atg atg ggc ttt cct gga gcc att      2862
ggc cct cca ggg ccc cct ggg aac cca ggc aca cca ggg cag agg ggg      2910
agc cct gga att cca gga gta aag ggc cag aga gga acc cca gga gcc      2958
aag ggg gaa caa gga gat aaa gga aat ccc ggg cct tca gag ata tcc      3006
cac gta ata ggg gac aaa gga gaa cca ggt ctc aaa gga ttc gca gga      3054
aat cca ggt gag aaa gga aac aga ggc gtt cca ggg atg cca ggt tta      3102
aag ggc ctc aaa gga cta ccc gga cca gca gga cca cca ggc ccc aga      3150
gga gat ttg ggc  agc act ggg aat cct  gga gaa cca gga ctg  cgt      3195
ggt ata cca gga  agc atg ggg aac atg  ggc atg cca ggt tct  aaa      3240
gga aaa agg gga  act ttg gga ttc cca  ggt cga gca gga aga  cca      3285
ggc ctc cca ggt  att cat ggt ctc cag  gga gat aag gga gag  cca      3330
ggt tat tca gaa  ggt aca agg cca gga  cca ccg gga cca acg  ggg      3375
gat cca gga ctg  ccg ggt gat atg gga  aag aaa gga gaa atg  ggg      3420
caa cct ggc cca  cct gga cat ttg ggg  cct gct gga cct gag  gga      3465
gcc cct gga agt  cct gga agt cct ggc  ctc cca gga aag cca  ggt      3510
cct cat ggt gat ttg ggt ttt aaa gga atc aaa ggc ctc ctg ggc 3555
cct cca gga atc aga ggc cct cca ggt ctt cca gga ttt cca gga 3600
tct cct gga cca atg ggt ata aga ggt gac caa gga cgt gat gga 3645
att cct ggt cca gcc gga gaa aag gga gaa acg ggt tta ttg agg 3690
gcc cct cca ggc cca aga ggg aac cct ggt gct caa gga gcc aaa 3735
gga gac agg gga gcc cca ggt ttt cct ggc ctc ccg ggc aga aaa 3780
ggg gcc atg gga gat gct gga cct cga gga ccc aca ggc ata gaa 3825
gga ttc cca ggg cca cca ggt ctg ccc ggt gca att atc cct ggc 3870
cag aca gga aat cgt ggt cca cca ggc tca aga gga agc cca ggt 3915
gcg cct ggt ccc cct gga cct cca ggg agt cat gta ata ggc ata 3960
aaa gga gac aaa ggg tct atg ggc cac cct ggc cca aaa ggt cca 4005
cct gga act gca gga gac atg gga cca cca ggt cgt ctg gga gca 4050
cca ggt act cca ggt ctt cca gga ccc aga ggt gat cct gga ttc 4095
cag ggg ttt cca ggc gtg aaa gga gaa aag ggt aat cct gga ttt 4140
cta gga tcc att gga cct cca gga cca att ggg cca aaa gga cca 4185
cct ggt gta cgt gga gac cct ggc aca ctt aag att atc tcc ctt 4230
cca gga agc cca ggg cca cct ggc aca cct gga gaa cca ggg atg 4275
cag gga gaa cct ggg cca cca ggg cca cct gga aac cta gga ccc 4320
tgt ggg cca aga ggt aag cca ggc aag gat gga aaa cca gga act 4365
cct gga cca gct gga gaa aaa ggc aac aaa ggt tct aaa gga gag 4410
cca gga cca gct gga tca gat gga ttg cca ggt ttg aaa gga aaa 4455
cgt gga gac agt gga tca cct gca acc tgg aca acg aga ggc ttt 4500
gtc ttc acc cga cac agt caa acc aca gca att cct tca tgt cca 4545
gag ggg aca gtg cca ctc tac agt ggg ttt tct ttt ctt ttt gta 4590
caa gga aat caa cga gcc cac gga caa gac ctt gga act ctt ggc 4635
agc tgc ctg cag cga ttt acc aca atg cca ttc tta ttc tgc aat 4680
gtc aat gat gta tgt aat ttt gca tct cga aat gat tat tca tac 4725
tgg ctg tca aca cca gct ctg atg cca atg aac atg gct ccc att 4770
act ggc aga gcc ctt gag cct tat ata agc aga tgc act gtt tgt 4815
gaa ggt cct gcg atc gcc ata gcc gtt cac agc caa acc act gac 4860
att cct cca tgt cct cac ggc tgg att tct ctc tgg aaa gga ttt 4905
tca ttc atc atg ttc aca agt gca ggt tct gag ggc acc ggg caa 4950
gca ctg gcc tcc  cct ggc tcc tgc ctg  gaa gaa ttc cga gcc  agc      4995
cca ttt cta gaa  tgt cat gga aga gga  acg tgc aac tac tat  tca      5040
aat tcc tac agt  ttc tgg ctg gct tca  tta aac cca gaa aga  atg      5085
ttc aga aag cct  att cca tca act gtg  aaa gct ggg gaa tta  gaa      5130
aaa ata ata agt  cgc tgt cag gtg tgc  atg aag aaa aga cac  tga      5175
agctaaaaaa gacagcagaa ctgctatttt tcatcctaaa gaacaaagta atgacagaac      5235
atgctgttat ttaggtattt ttctttaacc aaacaatatt gctccatgat gacttagtac      5295
aaagtttcaa tttgtttccc cacaaaacaa agcaattctt tcaagtcagt tctgtgatct      5355
gggtctctaa tctgtgctgt ttcaaagttc tctgtggcaa agcagcaact attcacaaaa      5415
tatcaccaaa aacctattcc acttacatcc aaggcactgt cactacggtg attgtatgaa      5475
gtttgaatgc tgcaagttat gaaatatttg gcccgctgga ttcccacatt tgtcttcttt      5535
ctgtctttaa gactcaggga ggctaaatca gtgtttgatt gccccgccaa cccttcctga      5595
aacttcagac cctgggtagg ggaagagaag ggggcatgtg gtatcctgga gcattgtgta      5655
tagaactgga ttttcagacc tgctgaggac cgtaaggcct gatggaacac agaactgaac      5715
tgaggttcat ggattttcca ggactgtttc aaacatgccc attactaacg gcaaaagggg      5775
gattccctga tggaaccata atacccttgg aaatactgta tggttttgtt ttgttttgtt      5835
ggtttttaaa gatttttgtt tgtttattga attcatttca ctgtagctct aaaatctgct      5895
tgtattccaa gcatataaaa ttttccccct tagtgaatta gttttaaaat gatattgtta      5955
tatacatact atgaaatatg tataacttta acttctgttt taccagcata cccacacaaa      6015
taacaagaat actacttatg aaatgtgcac tttatcctca ttccataaat gtcggtgcat      6075
accttatgta agggagcagt tcaataatcc atgaaagaac ttaaggcatt tgttggttta      6135
tcagactcgg aatctatttt ctcattgctc tgaatatgtc atcactctag gttttacaga      6195
tttattcctt tgttacttct ctaattcttc ctttgtaaaa aaaaaaaaaa gcaacacttt      6255
ttatgttata tgttgttctt acaaaccata ctgaaagagt ccattgttta aaaatcttaa      6315
tgtatcaaac tgtataactt ggccgctgta tgtcttaaaa cctgcttttc aatgtgttga      6375
tacattccca aggttactta attcaactta actatcatct tattcagcac caagcatgtc      6435
ccaggcactg tactaaccta cagagatgct aagagaaaaa aaagacttgt ttctgatcta 6495
atatcccaga aaaagtaact cattgctctg ttaataatct cacatataca agtagcttcc 6555
ctcccctcta gtttttcttt ccttttcact gctgttatat ttcatcatga taattcagca 6615
ggcccaagta aaggttaaaa ataaggtcta tgcctaggga aactcagggc ttctagtttc 6675
tcttagaaaa gctaagagaa gataaggtct gaataatagc agaaaaacca acatctacaa 6735
aacattaaac tagtgttata cttgatgata acactatttg atgagtctta gagtccagac 6795
acaaagagac aaagctttga agatgctttt tgatctacct aggtggagtt ggtggtgctg 6855
atatttaaat tcaggctact gcttcaatct caattgcttt gtaagtgaaa aacatgaccc 6915
agaggacagc acagactatg gccatggctc acatggttta catccttcac tgctcacgtg 6975
tttgctgtca agccattttt acatctaaac taagatgtgc agcatttcac ttatttagat 7035
tcacttaaca aacaaatttt tctgctttaa aaatgtctta ttgtcccaag tgtactatag 7095
cggcatatag agctagctaa tctctacaaa ccctctgtag gccagtagtt ctcaaagtgt 7155
ggtctctgga agagcagtat cagcatcatc tgggaacttg tcacagatgc agattctagg 7215
gaccactcca gacctacaca atcagaaact cttgggggag ggcccgaaat atctatgttt 7275
taccaagccc accacatgat tctgatgtac tctaaatact gagaaaacct gttctagaca 7335
aatacccaag caacaactcc gcaggcagtt accaagtacg gctggctaca actgctccat 7395
ccgtgcctct ttttaaagtt caaactcaca ggtgactcta aggttatcta cttttactca 7455
taagtaaaag ccctagactg gtgctaatgt caaaccactg gcctccactc aggcctccat 7515
cttctcatgc cctcttacca gtatttaact tctgaggaag acaagtgatg ctaaaacctg 7575
aaattccaat gaagccatat gaacagctgt tcagttgcac ttctaagact ttacttagca 7635
gtaaattata gctcatgtgc attattttcc agataactta gcttatgagt agcttataca 7695
attatgaaga tttaatatta cagataaaat gtaaactgtt tctttaaaat tggggcttca 7755
actttggaat ttcacagcgt gctaaaataa cagatttctc agaagtcttt cagcaagata 7815
aacattatta agtaacttat ttatgaaagt attaaaatgc ttacatttga acttgatggc 7875
taacttacaa agattctcta tgtatcaaat gtaacttact gcgactaaac ttaatttaat 7935
atttactcta taaccaaatg aaatatattt aaaatatatt gaatatttta tattgttata 7995
tcctgacaag attataatat tttaatgtac taatatttct gtaattatat ctaaaatatt 8055
attttattat attgcctaag aataaacatt tgttaaattg gaaaaaaaaa aaaaaaaaa 8114

<210> 6
<211> 1670
<212> PRT
<213> Homo sapiens

<400>  6

<210> 7
<211> 10341
<212> DNA
<213> Homo sapiens

<220>

<221>  CDS
<222>  (655)..(5727)

<220>
<221>  sig_peptide
<222>  (655)..(768)

<400>  7
aggctttttg gttgtggctt caagggaggg aagacgtggc tcaggtcccg ccctcatacc      60
ccctccctcc caacttacca ggcagtgtcc cttctacccg ggcatcgtgc ccaggaggcg     120
agaaagggct gggcgcctga ggggcaggtg ggcgcggatc cagggtaagg ggttaaggag     180
gtgatgctcc caaggtccgg gggcccaggc ggggaagtgg ggtgcggtcg gaggtggggc     240
gtttgggggt gtgcggtggg gaggtggctg cgcggtgggg aggtggctgc gcagggtgga     300
gggagtggcc agcggccctg cccagattaa gcgggaggcg cgccgggcgt ctgcactcgg     360
gaggccccgc ttgttccccg cgcccgcggc ggtccgcgtt ccccgcctcc agcccgcctc     420
caacttgcgg ccgccgggct tggctgcgag agcgccgcga agaggcagcg gggcgcgggt     480
ggattggggc tggaggtgcg cgtcccgtgg ggtggcaagg cggcagctcc tggcgctgcg     540
ggcgtcccca caggaacaga ctttgaccca gaacacagaa cctcacttgt caacaagaac     600
cttctggaag agaagactgg cagaatattt tttaagtact aagacttgcc tgcg atg      657
tgg tct ctg cac ata gta cta atg agg tgc tcc ttc aga ttg acc aag      705
tcc ttg gcc aca ggt ccc tgg tca ctt ata ctc att ctc ttt tct gta      753
caa tat gta tat ggg agt gga aag aaa tac att ggt cct tgt gga gga      801
aga gat tgc tct gtt tgc cac tgt gtt cct gaa aag ggg tct cgg ggt      849
cca cca gga cca cca ggg cca cag ggt cca att gga ccc ctg gga gcc      897
cca gga ccc att ggg ctt tca gga gag aaa gga atg aga ggg gac cgc      945
ggc cct cct gga gca gca ggg gac aaa gga gat aag ggt cca act ggt      993
gtt cct gga ttt cca ggt tta gat ggc ata cct ggg cac cca ggg cct     1041
cct gga ccc aga ggc aaa cct ggt atg agt ggc cac aat ggc tca aga 1089
ggt gac cca ggg ttt cca gga gga aga gga gct ctt ggc cca gga ggc 1137
ccc cta ggc cat cct ggg gaa aag gga gaa aaa gga aat tca gtg ttc 1185
att tta ggt gcc gtt aaa ggt att cag gga gac aga ggg gac cca gga 1233
ctg cct ggc tta cca gga tct tgg ggt gca gga gga ccg gca ggt ccc 1281
aca gga tat cct gga gag cca ggg tta gtg gga cct ccg ggc caa cca 1329
ggg cgt cca ggt ttg aag gga aat ccc ggt gtg gga gta aag ggg caa 1377
atg gga gac ccg ggt gag gtt ggt cag caa ggt tct cct gga ccc acc 1425
ctg ttg gta gag cca cct gac ttt tgt ctc tat aaa gga gaa aag ggt 1473
ata aaa gga att cct gga atg gtt gga ctg cca gga cca cca gga cgc 1521
aag gga gaa tct ggt att ggg gca aaa gga gaa aaa ggt att cct gga 1569
ttt cca ggg cct cgg ggg gat cct ggt tcc tat gga tct cca ggt ttt 1617
cca gga tta aag gga gaa cta gga ctg gtt gga gat cct ggg cta ttt 1665
gga tta att ggc cca aag ggg gat cct gga aat cga ggg cac cca gga 1713
cca cca ggt gtt ttg gtg act cca cct ctt cca ctc aaa ggc cca cca 1761
ggg gac cca ggg ttc cct ggc cgc tat gga gaa aca ggg gat gtt gga 1809
cca cct ggt ccc cca ggt ctc ttg ggc aga cca ggg gaa gcc tgt gca        1857
ggc atg ata gga ccc cct ggg cca caa gga ttt cct ggt ctt cct ggg        1905
ctt cca gga gaa gct ggt att cct ggg aga cct gat tct gct cca gga        1953
aaa cca ggg aag cca gga tca cct ggc ttg cct gga gca cca ggc ctg        2001
cag ggc ctc cca gga tca agt gtg ata tac tgt agt gtt ggg aac ccc        2049
gga cca caa gga ata aaa ggc aaa gtt ggt ccc cca gga gga aga ggc        2097
cca aaa gga gaa aaa gga aat gaa gga ctc tgt gcc tgt gag cct gga        2145
ccc atg ggc ccc cct ggc cct cca gga ctt cct ggg agg cag ggg agt        2193
aag gga gac ttg ggg ctc cct ggc tgg ctt gga aca aaa ggt gac cca        2241
gga cct cct ggt gct gaa gga cct cca ggg cta cca gga aag cat ggt        2289
gcc tct gga cca cct ggc aac aaa ggg gcg aag ggt gac atg gtt gta        2337
tca aga gtt aaa ggg cac aaa gga gaa aga ggt cct gat ggg ccc cca        2385
gga ttt cca ggg cag cca gga tca cat ggt cgg gat gga cat gct gga        2433
gaa aaa ggg gat cca gga cct cca ggg gat cat gaa gat gcg acc cca        2481
ggt ggt aaa gga ttt cct gga cct ctg ggc ccc cca ggc aaa gca gga        2529
cct gtg ggg ccc cca gga ctg gga ttt cct ggt cca cca gga gag cga        2577
ggc cac cca gga gtt cca ggc cac cca ggt gtg agg ggc cct gat ggc      2625
ttg aag ggt cag aaa ggt gac aca att tct tgc aac gta acc tac cct      2673
ggg agg cat ggc cct cca ggt ttt gat gga cct cca ggt ccg aag gga      2721
ttt cca ggt ccc caa ggt gcc cct ggg ctg agt ggt tca gat ggg cat      2769
aaa ggc aga cct ggc aca cca gga aca gcg gaa ata cca ggt cca cct      2817
ggt ttt cgt ggt gac atg gga gat ccg ggt ttt gga ggt gaa aag ggg      2865
tcc tcc cct gtt ggg ccc cca ggc cct ccc ggc tca cca gga gtg aat      2913
ggt cag aaa gga atc ccg gga gac cct gca ttt ggt cac ctg gga ccc      2961
ccg gga aag agg ggt ctt tca gga gtg cca ggg ata aaa gga ccc aga      3009
ggt gat ccg gga tgt cca ggg gct gaa ggg cca gct ggc att cct gga      3057
ttc cta ggt ctc aaa ggt ccc aaa ggc aga gag gga cat gct ggg ttt      3105
cca ggt gtc cca ggt cca cct ggc cat tcc tgt gaa aga ggt gct cca      3153
ggg ata cca ggg caa ccg gga ctc cct ggg tat cca ggt agc cca ggt      3201
gct cca ggt ggg aaa gga cag ccg gga gat gtg ggg cct ccc ggg cca      3249
gct gga atg aaa ggc ctc ccc gga ctc cca gga cgg cct ggg gca cat      3297
ggt ccc cca ggc ctc cca gga atc cca ggt ccc ttt gga gat gat ggg 3345
cta cct ggt cct cca ggt cca aag gga ccc cgg ggg ctg cct ggt ttc 3393
cca ggt ttt ccc gga gaa aga gga aag cct ggt gca gag gga tgt cct 3441
ggc gca aag gga gaa cct gga gag aag ggc atg tct ggc ctt cct gga 3489
gac cgg gga ctg aga ggg gcc aaa gga gcc ata gga cct ccc gga gat 3537
gaa gga gaa atg gct atc att tca caa aag gga aca cct ggg gaa cct 3585
gga cct cct gga gat gat gga ttc cca gga gaa aga ggt gat aaa gga 3633
act ccc ggg atg caa ggg aga aga gga gag ccg gga aga tac gga cca 3681
cct gga ttt cac aga ggg gaa cct ggt gag aaa ggt cag cca ggg 3726
cct cct gga ccc cca ggc cct cca ggc tca act ggt cta aga ggg 3771
ttc att ggt ttt cca gga ctt cca ggt gac cag ggt gag cca ggt 3816
tct cca ggt ccc cct gga ttt tca gga att gat gga gca aga gga 3861
cct aaa gga aac aaa ggt gac cct gcc agt cac ttt ggt cca cct 3906
ggt cca aag ggt gag cca ggt agc cct gga tgt cca ggg cat ttt 3951
gga gca tcc gga gag cag ggc ttg cct ggt att caa ggg ccc aga 3996
gga tca cct gga agg cca ggg cca cct ggc tcc tct gga cca cca 4041
ggg tgc cca ggt gat cac ggg atg cct ggg ctg agg gga cag cca 4086
gga gaa atg gga gac cct ggg cca aga ggc ctc cag ggg gat cca 4131
ggg ata cca ggt cct ccg gga ata aaa ggt ccc tcc gga tca cct 4176
ggc ctg aac ggc ttg cat gga ttg aaa ggt cag aaa gga act aaa 4221
ggt gct tca ggt ttg cat gat gtg ggg cca cct ggt cca gtg gga 4266
ata cct ggg cta aaa ggg gag aga gga gac cct ggg agc cca gga 4311
atc tct cct cca ggt cct cgt gga aag aaa ggt ccc cca gga ccc 4356
cca ggg agt tca gga cca cct ggt cct gca ggt gcc aca gga aga 4401
gct cct aag gac att cct gac ccg ggt cca cct gga gat cag gga 4446
cct cct ggt cct gat ggc cca aga gga gca cct ggg cct cca ggc 4491
ctc cct ggg agt gtt gac ctt ctg aga ggg gag cca ggt gac tgt 4536
ggt cta cca ggg cca cca ggt ccc cct ggc cca cca ggc cct cca 4581
gga tac aaa ggc ttt cca gga tgt gat gga aaa gat ggc cag aaa 4626
gga cca gtg gga ttc ccg gga ccg cag gga cca cat gga ttt cct 4671
ggg cca cct gga gag aag ggt tta cct gga cct cca ggg aga aaa 4716
ggg ccc act ggt ctt ccg ggt ccc aga ggt gaa ccg ggg cca cct 4761
gca gat gtg gat gac tgt ccc cga atc cca ggc ctt cct ggg gcg      4806
cca ggc atg aga gga cca gaa gga gcc atg ggg ctc cct gga atg      4851
aga ggc ccc tca gga cca ggg tgc aaa gga gag cct ggg ctg gat      4896
ggc agg agg ggt gtg gat ggc gtc cct ggg tct cct ggg cct ccc      4941
gga cgt aaa ggt gac aca gga gaa gac ggc tac cct gga gga cca      4986
ggg cct cct ggt ccc att ggg gat cct ggg ccc aaa ggg ttt ggc      5031
cct gga tac ctc ggt ggc ttc ctc ctg gtt ctc cac agt cag acg      5076
gac cag gag ccc acc tgc ccc ctg ggc atg ccc agg ctc tgg act      5121
ggg tat agt ctg tta tac ctg gaa ggg caa gag aaa gct cac aat      5166
caa gac ctt ggt ctg gca ggg tct tgc ctt ccc gta ttt agc acg      5211
ctg ccc ttt gcc tac tgc aac atc cac cag gtg tgc cac tat gcc      5256
cag aga aac gac aga tcc tac tgg ctg gcc agc gct gcg ccc ctc      5301
ccc atg atg cca ctc tct gaa gag gcg atc cgc ccc tat gtc agc      5346
cgc tgt gcg gta tgc gag gcc ccg gcc cag gcg gtg gcg gtg cac      5391
agc cag gac cag tcc atc ccc cca tgt ccg cag acc tgg agg agc      5436
ctc tgg atc ggg tat tca ttc ctg atg cac aca gga gct ggg gac      5481
caa gga gga ggg cag gcc ctt atg tca cct ggc agc tgc ctg gaa 5526
gat ttc aga gca gca cca ttc ctt gaa tgc cag ggc cgg cag gga 5571
act tgc cac ttt ttc gca aat aag tat agc ttc tgg ctc aca acg 5616
gtg aaa gca gac ttg cag ttt tcc tct gct cca gca cca gac acc 5661
tta aaa gaa agc cag gcc caa cgc cag aaa atc agc cgg tgc cag 5706
gtc tgc gtg aag tat agc tag agaatgcgaa attcaccaac acgtggccaa 5757
gagaaacttc ctagggggct aagacttcct agactgtgct aagagatgtc atggtgctca 5817
ttttggactc cctttcagtg gttccttctg ttttgtcgtg gttattccca gagtcctctg 5877
gttccttaca cattaagcaa atgctgcaca gatggatttg tttggacctc ccaatctagg 5937
gagcctagat actcttattt tactgaggat gatcgaagaa ctggctttac ttaaaaatat 5997
gctaattctc agaaaggcaa gtagatgata aaggccagat tacaaattac attactgaaa 6057
acttcattct tgggttaaca gtatctcaaa caattgaagt caattactct ataatacagt 6117
gggcttctgg atggatttta taggaaaaaa taaacaggtc aatgaatgaa actagaaagc 6177
agagattttc aacatttcaa aatgatttcc tctgtaatct atttttccat atactttaaa 6237
taatggtaaa accatgacgc aaagagagat ttttttttaa agaagaaaaa aaaaaattca 6297
cactgccagc gttaacagtt cctttcaaag gagaatgaat catgatggca ggaaggccca 6357
cagtcgccgt attccagaga tgcgacatta gcataaacac atcacagatg aatataaaac 6417
attatgttct cttctgcatt tttcagagaa tagaaatgcc tactttggca acccttttga 6477
aaagtagcaa ttatggaaaa aaaatattca ataagagatt aggagcctaa aagctattag 6537
tgaatattaa ggtagttatt cacaaaaatt gactccccat tgcagtgaac ttccagacag 6597
actgcttttc cccagtcggg gtccggcgtg tcacaggtgc gtgcgtgcta atgggactga 6657
cgctacatgg ggctcactca ggcaggcacg cgcttcatac aaagcatctc actcccctcc 6717
ccaggagagc ctgcacagct tcttgcactc acaacggaca ctttgctcca cacacataat 6777
ggcagctcac acagggacgt gacagagcta tcattatgca cttgggagaa aattaagggc 6837
cgatttaatt aaacttaggt aagaagattc atttaagtca gggttacccc atcaggagga 6897
catggcacta tctttaaacg aaacaaagac aatttataat ttgaatttta tgctcccgtg 6957
gttggctgtt acaggagcat cccattttgc caattttaaa gacattctta tatttcatat 7017
cagtcttgta ccaaggcaac agtttgacat ttggcattag tattttctaa aaaagtttag 7077
aatgtgtgtc aatttataat gattattttt ttctgtaaag caaaagatcc ctttttctgt 7137
tttgctagga atttggtgat ctaatcctaa atttaaaaga tttgttggaa aaaattttta 7197
ggaaactcac cttcctcatc taaaagaaaa aggcatttta gagaaaacta aagaaatttc 7257
tcatgagcgt gacactcatt ttagtgcttt gtttccgtgc acttaaaaat aattgagaag 7317
aaaaactcaa ttaaaatttt gtttataaga aatgttttcc ttgccaaacc ttgatttgta 7377
atgagctctt atatgcagaa cacatttcaa atgagttttg ctatgggctg cccccagggt 7437
ggcaatttat tttaagagta ttttctggta aaaagaaaaa tgtgtatttt aagatgaaat 7497
attttcttga tgtagcagaa tatttcctag ttcatttgac ccatttgata ttttttaaac 7557
catgctctgg catgttgaat atttttgtgc acctaaaact taagccaatt tcaatcttat 7617
ttgtgattac ctttctcctt cccaaaaagc tttatctatt accaaaagtc aaccctccta 7677
aaagttcaac ctgttcatct tgaacttggc ctgagaacat tttctgggaa gaggtaaggg 7737
tgacaaatgg aacatcagaa acgtatcttg cttgctaatt attttaaaca ctttaatgtt 7797
ggtattagaa tattatcttc ataagttaat aaataagtaa aaaaaaaaaa cccacacaaa 7857
gacaggctaa attatgtaga cagtttactc cacagtgagc ttgggaaggt atcagactgt 7917
tcaaaatgtt agtattacaa gagctgtctc taaatgcagc attaacactg ccctctaatt 7977
ttacaaaagt attttaaagg atcaaaaggg cggaaaaata acttcttgga atagaagatc 8037
ctgttacctt aaaccattat tttcttcacc tggttatata tcaatgtgcc ttcaattacc 8097
ctttctctgt ggaggtgatg aacaaagctt ttaagttgtt ctcctaaccc attattggag 8157
atgatcctta tcaaaccctt ctctccctgc ctccccacca accttgtaga ctggaacgtg 8217
cctagattgc cctaatggga actgttctgt tctgtgcccg tgctggaaat ctgggatgct 8277
tccttctggg ttctgattct cagtgagtgg agcccattca tccaggcagg tttccatgct 8337
atgagtggcc ctttcgttct gctctcacac tccccacatt gaaagctgag tcatcgctgc 8397
tttggatggc tacctcctct ttagccccgt gcccgtctca cacagccttt gttttccacc 8457
tccacccttg cagtaggcat ctcccagttg gtgccttagt ttcaaatccc agttgccatc 8517
taagatccac ccaaacttga ttttgtcacc ctctggatta gaatccttct gtgactccgc 8577
tgcccatgaa gtccaaatga cttcctatgg ggtgaaaggt ccagtgggga ctgttcgagg 8637
gttccctcta ggagtatttc ctgtcatgat gaactgtgtg cagctctgca gacactccca 8697
gcacctcttc gactttgctc ctgcttcacc agtgatgtcc tccctggggt cagtttaccc 8757
tgcgtgggag gtgaattccc tgggggaaac ttaggctccc ttctgctgct cttgtgcagt 8817
gcacggggct gtcctcattt tctccgttcc ccattgcttc tttaatgtct cagtgcactt 8877
ccttcattcc attgccaaaa tgcaagcccc ttgagggcag ggaccatgct tgactcgttt 8937
ttgtatgcct aatgcctagc acagagttga acacattact gccaattcat ttttttcaat 8997
gacaattcca taaacatcat taggcattag gtgttcagct ctgcatataa ggtcttcatc 9057
agcaagacgg ttacaagtat aaaccatttc ctatttcaga atattttaat attcaacata 9117
tttgtatacc cacattctct tgttacaatt attgccagaa agccaattga taagctttca 9177
aaatgggctt atcacacccc tccaaaaata gatttttgtt ttttctataa acagaaattt 9237
cagaaatgag atttgagaga tccgactgag aagacccttc agtaaaatgt gacatgactt 9297
accttatgct gtaatctgta ttccctcagg atttaagagc ttcctagcat aaaatctttg 9357
ctaaacgttt gtgtacatgt tattggtgct ccgaaaaata gtcaatggaa tggtgcacta 9417
caattgggct ttatcatctt attttgaacc tcgtgtagat tacagtaaag caaaccattc 9477
agtgtgttcc aggaaattat attagatctg tgtttctatc agctcactgg aataatctga 9537
taattgttac ttttacttgg tatggctgca agaatagtgg aaagaagagg acttgagagt 9597
tggtcgaacc caggtttgaa atctggctta gtcatttttc agctgttatc ctgggtgagt 9657
tttgccaact ttcttagctt catttccctc attaatacgg tgggacataa cagctactct 9717
tgcattgaaa attaagttag attacctgtc tagcataccc atcatcatac acttactatg 9777
gtcacatttt gtatttaaaa taaactaata cgaaaaatat ttcttttttt ttacacagaa 9837
ttatgattct cacagggtat ataaattact gattagaatt atttatatgt ggccaattct 9897
taatgtcatt gggaatgctg tttcatttca atcctcaaag ttactgtagc acagaaaata 9957
tcacaatttc ctgcagggac attatcagta agttctgcag ggaacaaaca attgacatta 10017
aaaatcagta ctctgcaatt gtcactgtta tcatctgcta gaaactctgc ataatgcatt 10077
ttaaaccacc aaggctggct gccacatcca tgtgaaatgc ttgaatttta tggtgcttaa 10137
atatttaatg attcatggga aaaatgtgaa atgtgtctaa taaattgcat ccctttctct 10197
aacctctggt tgtaaagtta aagactttca gcatgtaact tttgcaagat gcctggtctg 10257
ccattggcac ttaaatattt gttgtattac gatttataat atggttcatt atatataaaa 10317
ttctgtgatg acttccaaaa acaa 10341

<210> 8
<211> 1690
<212> PRT
<213> Homo sapiens

<400> 8

<210>  9
<211>  6531
<212>  DNA
<213>  Homo sapiens

<220>
<221>  CDS
<222>  (289)..(5364)

<220>
<221>  sig_peptide
<222>  (289)..(366)

<400>  9
agatctgtag gaattgagtg aagaaaaagt ttgcaagtct ggacagaagg gaaaagttct      60

cctgagagac cggctttggg gaggggaggg gggaaggaag agtagctcct tcttcttctt     120

cttttttttt tcttccactc ttaaaaagct tctttctctt cacccaagcc tcactgtccc     180

tctccggctc tagctctctc catataaacc ctcaagatta tgtcaattgg ttagagccag     240

ccgggaattt cgtgcgggtg ctgaaggagc tgcgggagcc ggagaaga atg aaa ctg     297
                                                     Met Lys Leu
                                                     1

cgt gga gtc agc ctg gct gcc ggc ttg ttc tta ctg gcc ctg agt ctt     345
Arg Gly Val Ser Leu Ala Ala Gly Leu Phe Leu Leu Ala Leu Ser Leu
    5                   10                  15

tgg ggg cag cct gca gag gct gcg gct tgc tat ggg tgt tct cca gga     393
Trp Gly Gln Pro Ala Glu Ala Ala Ala Cys Tyr Gly Cys Ser Pro Gly
20                  25                  30                  35

tca aag tgt gac tgc agt ggc ata aaa ggg gaa aag gga gag aga ggg     441
Ser Lys Cys Asp Cys Ser Gly Ile Lys Gly Glu Lys Gly Glu Arg Gly
                40                  45                  50

ttt cca ggt ttg gaa gga cac cca gga ttg cct gga ttt cca ggt cca     489
Phe Pro Gly Leu Glu Gly His Pro Gly Leu Pro Gly Phe Pro Gly Pro
            55                  60                  65

gaa ggg cct ccg ggg cct cgg gga caa aag ggt gat gat gga att cca     537
Glu Gly Pro Pro Gly Pro Arg Gly Gln Lys Gly Asp Asp Gly Ile Pro
        70                  75                  80

ggg cca cca gga cca aaa gga atc aga ggt cct cct gga ctt cct gga     585
Gly Pro Pro Gly Pro Lys Gly Ile Arg Gly Pro Pro Gly Leu Pro Gly
    85                  90                  95

ttt cca ggg aca cca ggt ctt cct gga atg cca ggc cac gat ggg gcc     633
Phe Pro Gly Thr Pro Gly Leu Pro Gly Met Pro Gly His Asp Gly Ala
100                 105                 110                 115

cca gga cct caa ggt att ccc gga tgc aat gga acc aag gga gaa cgt     681
Pro Gly Pro Gln Gly Ile Pro Gly Cys Asn Gly Thr Lys Gly Glu Arg
                120                 125                 130

gga ttt cca ggc agt ccc ggt ttt cct ggt tta cag ggt cct cca gga     729
Gly Phe Pro Gly Ser Pro Gly Phe Pro Gly Leu Gln Gly Pro Pro Gly
            135                 140                 145

ccc cct ggg atc cca ggt atg aag ggt gaa cca ggt agt ata att atg     777
Pro Pro Gly Ile Pro Gly Met Lys Gly Glu Pro Gly Ser Ile Ile Met
        150                 155                 160

tca tca ctg cca gga cca aag ggt aat cca gga tat cca ggt cct cct     825

Ser Ser Leu Pro Gly Pro Lys Gly Asn Pro Gly Tyr Pro Gly Pro Pro
    165                 170                 175

gga ata caa ggc cta cct ggt ccc act ggt ata cca ggg cca att ggt      873
Gly Ile Gln Gly Leu Pro Gly Pro Thr Gly Ile Pro Gly Pro Ile Gly
180                 185                 190                 195

ccc cca gga cca cca ggt ttg atg ggc cct cct ggt cca cca gga ctt      921
Pro Pro Gly Pro Pro Gly Leu Met Gly Pro Pro Gly Pro Pro Gly Leu
                200                 205                 210

cca gga cct aag ggg aat atg ggc tta aat ttc cag gga ccc aaa ggt      969
Pro Gly Pro Lys Gly Asn Met Gly Leu Asn Phe Gln Gly Pro Lys Gly
            215                 220                 225

gaa aaa ggt gag caa ggt ctt cag ggc cca cct ggg cca cct ggg cag     1017
Glu Lys Gly Glu Gln Gly Leu Gln Gly Pro Pro Gly Pro Pro Gly Gln
        230                 235                 240

atc agt gaa cag aaa aga cca att gat gta gag ttt cag aaa gga gat     1065
Ile Ser Glu Gln Lys Arg Pro Ile Asp Val Glu Phe Gln Lys Gly Asp
    245                 250                 255

cag gga ctt cct ggt gac cga ggg cct cct gga cct cca ggg ata cgt     1113
Gln Gly Leu Pro Gly Asp Arg Gly Pro Pro Gly Pro Pro Gly Ile Arg
260                 265                 270                 275

ggt cct cca ggt ccc cca ggt ggt gag aaa ggt gag aag ggt gag caa     1161
Gly Pro Pro Gly Pro Pro Gly Gly Glu Lys Gly Glu Lys Gly Glu Gln
                280                 285                 290

gga gag cca ggc aaa aga ggt aaa cca ggc aaa gat gga gaa aat ggc     1209
Gly Glu Pro Gly Lys Arg Gly Lys Pro Gly Lys Asp Gly Glu Asn Gly
            295                 300                 305

caa cca gga att cct ggt ttg cct ggt gat cct ggt tac cct ggt gaa     1257
Gln Pro Gly Ile Pro Gly Leu Pro Gly Asp Pro Gly Tyr Pro Gly Glu
        310                 315                 320

ccc gga agg gat ggt gaa aag ggc caa aaa ggt gac act ggc cca cct     1305
Pro Gly Arg Asp Gly Glu Lys Gly Gln Lys Gly Asp Thr Gly Pro Pro
    325                 330                 335

gga cct cct gga ctt gta att cct aga cct ggg act ggt ata act ata     1353
Gly Pro Pro Gly Leu Val Ile Pro Arg Pro Gly Thr Gly Ile Thr Ile
340                 345                 350                 355

gga gaa aaa gga aac att ggg ttg cct ggg ttg cct gga gaa aaa gga     1401
Gly Glu Lys Gly Asn Ile Gly Leu Pro Gly Leu Pro Gly Glu Lys Gly
                360                 365                 370

gag cga gga ttt cct gga ata cag ggt cca cct ggc ctt cct gga cct     1449
Glu Arg Gly Phe Pro Gly Ile Gln Gly Pro Pro Gly Leu Pro Gly Pro
            375                 380                 385

cca ggg gct gca gtt atg ggt cct cct ggc cct cct gga ttt cct gga     1497
Pro Gly Ala Ala Val Met Gly Pro Pro Gly Pro Pro Gly Phe Pro Gly
        390                 395                 400

gaa agg ggt cag aaa ggt gat gaa gga cca cct gga att tcc att cct     1545
Glu Arg Gly Gln Lys Gly Asp Glu Gly Pro Pro Gly Ile Ser Ile Pro
    405                 410                 415

gga cct cct gga ctt gac gga cag cct ggg gct cct ggg ctt cca ggg 1593
Gly Pro Pro Gly Leu Asp Gly Gln Pro Gly Ala Pro Gly Leu Pro Gly
420                 425                 430                 435

cct cct ggc cct gct ggc cct cac att cct cct agt gat gag ata tgt 1641
Pro Pro Gly Pro Ala Gly Pro His Ile Pro Pro Ser Asp Glu Ile Cys
                440                 445                 450

gaa cca ggc cct cca ggc ccc cca gga tct cca ggt gat aaa gga ctc 1689
Glu Pro Gly Pro Pro Gly Pro Pro Gly Ser Pro Gly Asp Lys Gly Leu
            455                 460                 465

caa gga gaa caa gga gtg aaa ggt gac aaa ggt gac act tgc ttc aac 1737
Gln Gly Glu Gln Gly Val Lys Gly Asp Lys Gly Asp Thr Cys Phe Asn
        470                 475                 480

tgc att gga act ggt att tca ggg cct cca ggt caa cct ggt ttg cca 1785
Cys Ile Gly Thr Gly Ile Ser Gly Pro Pro Gly Gln Pro Gly Leu Pro
    485                 490                 495

ggt ctc cca ggt cct cca gga tct ctt ggt ttc cct gga cag aaa ggg 1833
Gly Leu Pro Gly Pro Pro Gly Ser Leu Gly Phe Pro Gly Gln Lys Gly
500                 505                 510                 515

gaa aaa gga caa gct ggt gca act ggt ccc aaa gga tta cca ggc att 1881
Glu Lys Gly Gln Ala Gly Ala Thr Gly Pro Lys Gly Leu Pro Gly Ile
                520                 525                 530

cca gga gct cca ggt gct cca ggc ttt cct gga tct aaa ggt gaa cct 1929
Pro Gly Ala Pro Gly Ala Pro Gly Phe Pro Gly Ser Lys Gly Glu Pro
            535                 540                 545

ggt gat atc ctc act ttt cca gga atg aag ggt gac aaa gga gag ttg 1977
Gly Asp Ile Leu Thr Phe Pro Gly Met Lys Gly Asp Lys Gly Glu Leu
        550                 555                 560

ggt tcc cct gga gct cca ggg ctt cct ggt tta cct ggc act cct gga 2025
Gly Ser Pro Gly Ala Pro Gly Leu Pro Gly Leu Pro Gly Thr Pro Gly
    565                 570                 575

cag gat gga ttg cca ggg ctt cct ggc ccg aaa gga gag cct ggt gga 2073
Gln Asp Gly Leu Pro Gly Leu Pro Gly Pro Lys Gly Glu Pro Gly Gly
580                 585                 590                 595

att act ttt aag ggt gaa aga ggt ccc cct ggg aac cca ggt tta cca 2121
Ile Thr Phe Lys Gly Glu Arg Gly Pro Pro Gly Asn Pro Gly Leu Pro
                600                 605                 610

ggc ctc cca ggg aat ata ggg cct atg ggt ccc cct ggt ttc ggc cct 2169
Gly Leu Pro Gly Asn Ile Gly Pro Met Gly Pro Pro Gly Phe Gly Pro
            615                 620                 625

cca ggc cca gta ggt gaa aaa ggc ata caa ggt gtg gca gga aat cca 2217
Pro Gly Pro Val Gly Glu Lys Gly Ile Gln Gly Val Ala Gly Asn Pro
        630                 635                 640

ggc cag cca gga ata cca ggt cct aaa ggg gat cca ggt cag act ata 2265
Gly Gln Pro Gly Ile Pro Gly Pro Lys Gly Asp Pro Gly Gln Thr Ile
    645                 650                 655

acc cag ccg ggg aag cct ggc ttg cct ggt aac cca ggc aga gat ggt 2313
Thr Gln Pro Gly Lys Pro Gly Leu Pro Gly Asn Pro Gly Arg Asp Gly
660                 665                 670                 675

gat gta ggt ctt cca ggt gac cct gga ctt cca ggg caa cca ggc ttg 2361
Asp Val Gly Leu Pro Gly Asp Pro Gly Leu Pro Gly Gln Pro Gly Leu
                680                 685                 690

cca ggg ata cct ggt agc aaa gga gaa cca ggt atc cct gga att ggg 2409
Pro Gly Ile Pro Gly Ser Lys Gly Glu Pro Gly Ile Pro Gly Ile Gly
            695                 700                 705

ctt cct gga cca cct ggt ccc aaa ggc ttt cct gga att cca gga cct 2457
Leu Pro Gly Pro Pro Gly Pro Lys Gly Phe Pro Gly Ile Pro Gly Pro
        710                 715                 720

cca gga gca cct ggg aca cct gga aga att ggt cta gaa ggc cct cct 2505
Pro Gly Ala Pro Gly Thr Pro Gly Arg Ile Gly Leu Glu Gly Pro Pro
    725                 730                 735

ggg cca ccc ggc ttt cca gga cca aag ggt gaa cca gga ttt gca tta 2553
Gly Pro Pro Gly Phe Pro Gly Pro Lys Gly Glu Pro Gly Phe Ala Leu
740                 745                 750                 755

cct ggg cca cct ggg cca cca gga ctt cca ggt ttc aaa gga gca ctt 2601
Pro Gly Pro Pro Gly Pro Pro Gly Leu Pro Gly Phe Lys Gly Ala Leu
                760                 765                 770

ggt cca aaa ggt gat cgt ggt ttc cca gga cct ccg ggt cct cca gga 2649
Gly Pro Lys Gly Asp Arg Gly Phe Pro Gly Pro Pro Gly Pro Pro Gly
            775                 780                 785

cgc act ggc tta gat ggg ctc cct gga cca aaa ggt gat gtt gga cca 2697
Arg Thr Gly Leu Asp Gly Leu Pro Gly Pro Lys Gly Asp Val Gly Pro
        790                 795                 800

aat gga caa cct gga cca atg gga cct cct ggg ctg cca gga ata ggt 2745
Asn Gly Gln Pro Gly Pro Met Gly Pro Pro Gly Leu Pro Gly Ile Gly
    805                 810                 815

gtt cag gga cca cca gga cca cca ggg att cct ggg cca ata ggt caa 2793
Val Gln Gly Pro Pro Gly Pro Pro Gly Ile Pro Gly Pro Ile Gly Gln
820                 825                 830                 835

cct ggt tta cat gga ata cca gga gag aag ggg gat cca gga cct cct 2841
Pro Gly Leu His Gly Ile Pro Gly Glu Lys Gly Asp Pro Gly Pro Pro
                840                 845                 850

gga ctt gat gtt cca gga ccc cca ggt gaa aga ggc agt cca ggg atc 2889
Gly Leu Asp Val Pro Gly Pro Pro Gly Glu Arg Gly Ser Pro Gly Ile
            855                 860                 865

ccc gga gca cct ggt cct ata gga cct cca gga tca cca ggg ctt cca 2937
Pro Gly Ala Pro Gly Pro Ile Gly Pro Pro Gly Ser Pro Gly Leu Pro
        870                 875                 880

gga aaa gca ggt gcc tct gga ttt cca ggt acc aaa ggt gaa atg ggt 2985
Gly Lys Ala Gly Ala Ser Gly Phe Pro Gly Thr Lys Gly Glu Met Gly
    885                 890                 895

atg atg gga cct cca ggc cca cca gga cct ttg gga att cct ggc agg 3033
Met Met Gly Pro Pro Gly Pro Pro Gly Pro Leu Gly Ile Pro Gly Arg
900                 905                 910                 915

agt ggt gta cct ggt ctt aaa ggt gat gat ggc ttg cag ggt cag cca 3081
Ser Gly Val Pro Gly Leu Lys Gly Asp Asp Gly Leu Gln Gly Gln Pro

920                 925                 930

gga ctt cct ggc cct aca gga gaa aaa ggt agt aaa gga gag cct ggc     3129
Gly Leu Pro Gly Pro Thr Gly Glu Lys Gly Ser Lys Gly Glu Pro Gly
            935                 940                 945

ctt cca ggc cct cct gga cca atg gat cca aat ctt ctg ggc tca aaa     3177
Leu Pro Gly Pro Pro Gly Pro Met Asp Pro Asn Leu Leu Gly Ser Lys
        950                 955                 960

gga gag aag ggg gaa cct ggc tta cca ggt ata cct gga gtt tca ggg     3225
Gly Glu Lys Gly Glu Pro Gly Leu Pro Gly Ile Pro Gly Val Ser Gly
    965                 970                 975

cca aaa ggt tat cag ggt ttg cct gga gac cca ggg caa cct gga ctg     3273
Pro Lys Gly Tyr Gln Gly Leu Pro Gly Asp Pro Gly Gln Pro Gly Leu
980                 985                 990                 995

agt gga caa cct gga  tta cca gga cca cca  ggt ccc aaa ggt aac     3318
Ser Gly Gln Pro Gly  Leu Pro Gly Pro Pro  Gly Pro Lys Gly Asn
                1000                1005                1010

cct ggt ctc cct gga  cag cca ggt ctt ata  gga cct cct gga ctt     3363
Pro Gly Leu Pro Gly  Gln Pro Gly Leu Ile  Gly Pro Pro Gly Leu
                1015                1020                1025

aaa gga acc atc ggt  gat atg ggt ttt cca  ggg cct cag ggt gtg     3408
Lys Gly Thr Ile Gly  Asp Met Gly Phe Pro  Gly Pro Gln Gly Val
                1030                1035                1040

gaa ggg cct cct gga  cct tct gga gtt cct  gga caa cct ggc tcc     3453
Glu Gly Pro Pro Gly  Pro Ser Gly Val Pro  Gly Gln Pro Gly Ser
                1045                1050                1055

cca gga tta cct gga  cag aaa ggc gac aaa  ggt gat cct ggt att     3498
Pro Gly Leu Pro Gly  Gln Lys Gly Asp Lys  Gly Asp Pro Gly Ile
                1060                1065                1070

tca agc att ggt ctt  cca ggt ctt cct ggt  cca aag ggt gag cct     3543
Ser Ser Ile Gly Leu  Pro Gly Leu Pro Gly  Pro Lys Gly Glu Pro
                1075                1080                1085

ggt ctg cct gga tac  cca ggg aac cct ggt  atc aaa ggt tct gtg     3588
Gly Leu Pro Gly Tyr  Pro Gly Asn Pro Gly  Ile Lys Gly Ser Val
                1090                1095                1100

gga gat cct ggt ttg  ccc gga tta cca gga  acc cct gga gca aaa     3633
Gly Asp Pro Gly Leu  Pro Gly Leu Pro Gly  Thr Pro Gly Ala Lys
                1105                1110                1115

gga caa cca ggc ctt  cct gga ttc cca gga  acc cca ggc cct cct     3678
Gly Gln Pro Gly Leu  Pro Gly Phe Pro Gly  Thr Pro Gly Pro Pro
                1120                1125                1130

gga cca aaa ggt att  agt ggc cct cct ggg  aac ccc ggc ctt cca     3723
Gly Pro Lys Gly Ile  Ser Gly Pro Pro Gly  Asn Pro Gly Leu Pro
                1135                1140                1145

gga gaa cct ggt cct  gta ggt ggt gga ggt  cat cct ggg caa cca     3768
Gly Glu Pro Gly Pro  Val Gly Gly Gly Gly  His Pro Gly Gln Pro
                1150                1155                1160

ggg cct cca ggc gaa  aaa ggc aaa ccc ggt  caa gat ggt att cct     3813

Gly Pro Pro Gly Glu Lys Gly Lys Pro Gly Gln Asp Gly Ile Pro
                1165                1170                1175

gga cca gct gga cag aag ggt gaa cca ggt caa cca ggc ttt gga 3858
Gly Pro Ala Gly Gln Lys Gly Glu Pro Gly Gln Pro Gly Phe Gly
                1180                1185                1190

aac cca gga ccc cct gga ctt cca gga ctt tct ggc caa aag ggt 3903
Asn Pro Gly Pro Pro Gly Leu Pro Gly Leu Ser Gly Gln Lys Gly
                1195                1200                1205

gat gga gga tta cct ggg att cca gga aat cct ggc ctt cca ggt 3948
Asp Gly Gly Leu Pro Gly Ile Pro Gly Asn Pro Gly Leu Pro Gly
                1210                1215                1220

cca aag ggc gaa cca ggc ttt cac ggt ttc cct ggt gtg cag ggt 3993
Pro Lys Gly Glu Pro Gly Phe His Gly Phe Pro Gly Val Gln Gly
                1225                1230                1235

ccc cca ggc cct cct ggt tct ccg ggt cca gct ctg gaa gga cct 4038
Pro Pro Gly Pro Pro Gly Ser Pro Gly Pro Ala Leu Glu Gly Pro
                1240                1245                1250

aaa ggc aac cct ggg ccc caa ggt cct cct ggg aga cca ggt cct 4083
Lys Gly Asn Pro Gly Pro Gln Gly Pro Pro Gly Arg Pro Gly Pro
                1255                1260                1265

aca ggt ttt caa ggt cta cca ggt cca gaa ggt cct cca ggt ctc 4128
Thr Gly Phe Gln Gly Leu Pro Gly Pro Glu Gly Pro Pro Gly Leu
                1270                1275                1280

cct gga aat gga ggt att aaa gga gag aag gga aat cca ggc caa 4173
Pro Gly Asn Gly Gly Ile Lys Gly Glu Lys Gly Asn Pro Gly Gln
                1285                1290                1295

cct ggg cta cct ggc ttg cct ggt ttg aaa gga gat caa gga cca 4218
Pro Gly Leu Pro Gly Leu Pro Gly Leu Lys Gly Asp Gln Gly Pro
                1300                1305                1310

cca gga ctc cag ggt aat cct ggc cgg ccg ggt ctc aat gga atg 4263
Pro Gly Leu Gln Gly Asn Pro Gly Arg Pro Gly Leu Asn Gly Met
                1315                1320                1325

aaa gga gat cct ggt ctc cct ggt gtt cca gga ttc cca ggc atg 4308
Lys Gly Asp Pro Gly Leu Pro Gly Val Pro Gly Phe Pro Gly Met
                1330                1335                1340

aaa gga ccc agt gga gta cct gga tca gct ggc cct gag ggg gaa 4353
Lys Gly Pro Ser Gly Val Pro Gly Ser Ala Gly Pro Glu Gly Glu
                1345                1350                1355

ccg gga ctt att ggt cct cca ggt cct cct gga tta cct ggt cct 4398
Pro Gly Leu Ile Gly Pro Pro Gly Pro Pro Gly Leu Pro Gly Pro
                1360                1365                1370

tca gga cag agt atc ata att aaa gga gat gct ggt cct cca gga 4443
Ser Gly Gln Ser Ile Ile Ile Lys Gly Asp Ala Gly Pro Pro Gly
                1375                1380                1385

atc cct ggc cag cct ggg cta aag ggt cta cca gga ccc caa gga 4488
Ile Pro Gly Gln Pro Gly Leu Lys Gly Leu Pro Gly Pro Gln Gly
                1390                1395                1400

cct caa ggc tta cca ggt cca act ggc cct cca gga gat cct gga 4533
Pro Gln Gly Leu Pro Gly Pro Thr Gly Pro Pro Gly Asp Pro Gly
                1405                1410                1415

cgc aat gga ctc cct ggc ttt gat ggt gca gga ggg cgc aaa gga 4578
Arg Asn Gly Leu Pro Gly Phe Asp Gly Ala Gly Gly Arg Lys Gly
                1420                1425                1430

gac cca ggt ctg cca gga cag cca ggt acc cgt ggt ttg gat ggt 4623
Asp Pro Gly Leu Pro Gly Gln Pro Gly Thr Arg Gly Leu Asp Gly
                1435                1440                1445

ccc cct ggt cca gat gga ttg caa ggt ccc cca ggt ccc cct gga 4668
Pro Pro Gly Pro Asp Gly Leu Gln Gly Pro Pro Gly Pro Pro Gly
                1450                1455                1460

acc tcc tct gtt gca cat gga ttt ctt att aca cgc cac agc cag 4713
Thr Ser Ser Val Ala His Gly Phe Leu Ile Thr Arg His Ser Gln
                1465                1470                1475

aca acg gat gca cca caa tgc cca cag gga aca ctt cag gtc tat 4758
Thr Thr Asp Ala Pro Gln Cys Pro Gln Gly Thr Leu Gln Val Tyr
                1480                1485                1490

gaa ggc ttt tct ctc ctg tat gta caa gga aat aaa aga gcc cac 4803
Glu Gly Phe Ser Leu Leu Tyr Val Gln Gly Asn Lys Arg Ala His
                1495                1500                1505

ggt caa gac ttg ggg acg gct ggc agc tgc ctt cgt cgc ttt agt 4848
Gly Gln Asp Leu Gly Thr Ala Gly Ser Cys Leu Arg Arg Phe Ser
                1510                1515                1520

acc atg cct ttc atg ttc tgc aac atc aat aat gtt tgc aac ttt 4893
Thr Met Pro Phe Met Phe Cys Asn Ile Asn Asn Val Cys Asn Phe
                1525                1530                1535

gct tca aga aat gac tat tct tac tgg ctc tct acc cca gag ccc 4938
Ala Ser Arg Asn Asp Tyr Ser Tyr Trp Leu Ser Thr Pro Glu Pro
                1540                1545                1550

atg cca atg agc atg caa ccc cta aag ggc cag agc atc cag cca 4983
Met Pro Met Ser Met Gln Pro Leu Lys Gly Gln Ser Ile Gln Pro
                1555                1560                1565

ttc att agt cga tgt gca gta tgt gaa gct cca gct gtg gtg atc 5028
Phe Ile Ser Arg Cys Ala Val Cys Glu Ala Pro Ala Val Val Ile
                1570                1575                1580

gca gtt cac agt cag acg atc cag att ccc cat tgt cct cag gga 5073
Ala Val His Ser Gln Thr Ile Gln Ile Pro His Cys Pro Gln Gly
                1585                1590                1595

tgg gat tct ctg tgg att ggt tat tcc ttc atg atg cat aca agt 5118
Trp Asp Ser Leu Trp Ile Gly Tyr Ser Phe Met Met His Thr Ser
                1600                1605                1610

gca ggg gca gaa ggc tca ggt caa gcc cta gcc tcc cct ggt tcc 5163
Ala Gly Ala Glu Gly Ser Gly Gln Ala Leu Ala Ser Pro Gly Ser
                1615                1620                1625

tgc ttg gaa gag ttt cgt tca gct ccc ttc atc gaa tgt cat ggg 5208
Cys Leu Glu Glu Phe Arg Ser Ala Pro Phe Ile Glu Cys His Gly
                1630                1635                1640

agg ggt acc tgt aac tac tat gcc aac tcc tac agc ttt tgg ctg 5253
Arg Gly Thr Cys Asn Tyr Tyr Ala Asn Ser Tyr Ser Phe Trp Leu
                1645                1650                1655

gca act gta gat gtg tca gac atg ttc agt aaa cct cag tca gaa 5298
Ala Thr Val Asp Val Ser Asp Met Phe Ser Lys Pro Gln Ser Glu
                1660                1665                1670

acg ctg aaa gca gga gac ttg agg aca cga att agc cga tgt caa 5343
Thr Leu Lys Ala Gly Asp Leu Arg Thr Arg Ile Ser Arg Cys Gln
                1675                1680                1685

gtg tgc atg aag agg aca taa cattttgaag aattcctttt gtgttttaaa 5394
Val Cys Met Lys Arg Thr
                1690

atgtgatata tatatatata aaattcctag gatgcagtgt ctcattgtcc ccaactttac 5454

tactgctgcc gtcaatggtg ctactatata tgatcaagat aacatgctga ctagtaacca 5514

tgaagattca gatgtacctc agcaatgcgc cagagcaaag tctctattat ttttctacta 5574

aagaaataag gaagtgaatt tactttttgg gtccagaatg actttctcca agaattataa 5634

gatgaaaatt atatattttg cccagttact aaaatggtac attaaaaatt caattaagag 5694

aagagtcaca ttgagtaaaa taaaagactg cagtttgtgg gaagaattat ttttcacggt 5754

gctactaatc ctgctgtatc ccgggttttt aatataaagg tgttaagctt attttgcttt 5814

gtaagtaaag aatgtgtata ttgtgaacag ccttttagct caaaatgttg agtcatttac 5874

atatgacata gcatgaatca ctctttacag aaaatgtagg aaaccctaga atacagacag 5934

caatatttta tattcatgtt tatcaaagtg agaggactta tattcctaca tcaagttact 5994

actgagagta aatttatttt gagttttatc ccgtaagttc tgttttgatt ttttttaaaa 6054

aacaaaccct tttagtcact ttaatcagaa ttttaaatgt tcatgttaca taccaaatta 6114

taatatctaa tggagcaatt tgtcttttgc tatattctcc aagattatct cttaagacca 6174

tatgccccct gttttaatgt ttcttacatc ttgtttttac tcatttctga ctggacaaag 6234

ttcttccaaa caattctgag aaacaaaaac acacacgcag aattaacaat tcttttccct 6294

gtgcttctta tgtaagaatc ctcctgtggc ctctgcttgt acagaactgg gaaacaacac 6354

ttggttagtc tcttttaagt tacaaaaagc caattgatgt ttcttattct ttttaaattt 6414

taaatatttt gttataaata ctcacaggat accttatttc cctagctatc atctcctgac 6474

ttaatgtttt ttaaacccac caatataaat ttaattaaag atatatgttg taaggat 6531

<210> 10
<211> 1691
<212> PRT
<213> Homo sapiens

<400> 10

Met Lys Leu Arg Gly Val Ser Leu Ala Ala Gly Leu Phe Leu Leu Ala
1               5                   10                  15

Leu Ser Leu Trp Gly Gln Pro Ala Glu Ala Ala Ala Cys Tyr Gly Cys
            20                  25                  30

Ser Pro Gly Ser Lys Cys Asp Cys Ser Gly Ile Lys Gly Glu Lys Gly
        35                  40                  45

Glu Arg Gly Phe Pro Gly Leu Glu Gly His Pro Gly Leu Pro Gly Phe
    50                  55                  60

Pro Gly Pro Glu Gly Pro Pro Gly Pro Arg Gly Gln Lys Gly Asp Asp
65                  70                  75                  80

Gly Ile Pro Gly Pro Pro Gly Pro Lys Gly Ile Arg Gly Pro Pro Gly
                85                  90                  95

Leu Pro Gly Phe Pro Gly Thr Pro Gly Leu Pro Gly Met Pro Gly His
            100                 105                 110

Asp Gly Ala Pro Gly Pro Gln Gly Ile Pro Gly Cys Asn Gly Thr Lys
        115                 120                 125

Gly Glu Arg Gly Phe Pro Gly Ser Pro Gly Phe Pro Gly Leu Gln Gly
    130                 135                 140

Pro Pro Gly Pro Pro Gly Ile Pro Gly Met Lys Gly Glu Pro Gly Ser
145                 150                 155                 160

Ile Ile Met Ser Ser Leu Pro Gly Pro Lys Gly Asn Pro Gly Tyr Pro
                165                 170                 175

Gly Pro Pro Gly Ile Gln Gly Leu Pro Gly Pro Thr Gly Ile Pro Gly
            180                 185                 190

Pro Ile Gly Pro Pro Gly Pro Pro Gly Leu Met Gly Pro Pro Gly Pro
        195                 200                 205

Pro Gly Leu Pro Gly Pro Lys Gly Asn Met Gly Leu Asn Phe Gln Gly
    210                 215                 220

Pro Lys Gly Glu Lys Gly Glu Gln Gly Leu Gln Gly Pro Pro Gly Pro
225                 230                 235                 240

Pro Gly Gln Ile Ser Glu Gln Lys Arg Pro Ile Asp Val Glu Phe Gln
                245                 250                 255

Lys Gly Asp Gln Gly Leu Pro Gly Asp Arg Gly Pro Pro Gly Pro Pro
        260                 265                 270

Gly Ile Arg Gly Pro Pro Gly Pro Pro Gly Gly Glu Lys Gly Glu Lys
    275                 280                 285

Gly Glu Gln Gly Glu Pro Gly Lys Arg Gly Lys Pro Gly Lys Asp Gly
    290                 295                 300

Glu Asn Gly Gln Pro Gly Ile Pro Gly Leu Pro Gly Asp Pro Gly Tyr
305                 310                 315                 320

Pro Gly Glu Pro Gly Arg Asp Gly Glu Lys Gly Gln Lys Gly Asp Thr
                325                 330                 335

Gly Pro Pro Gly Pro Pro Gly Leu Val Ile Pro Arg Pro Gly Thr Gly
            340                 345                 350

Ile Thr Ile Gly Glu Lys Gly Asn Ile Gly Leu Pro Gly Leu Pro Gly
        355                 360                 365

Glu Lys Gly Glu Arg Gly Phe Pro Gly Ile Gln Gly Pro Pro Gly Leu
    370                 375                 380

Pro Gly Pro Pro Gly Ala Ala Val Met Gly Pro Pro Gly Pro Pro Gly
385                 390                 395                 400

Phe Pro Gly Glu Arg Gly Gln Lys Gly Asp Glu Gly Pro Pro Gly Ile
                405                 410                 415

Ser Ile Pro Gly Pro Pro Gly Leu Asp Gly Gln Pro Gly Ala Pro Gly
            420                 425                 430

Leu Pro Gly Pro Pro Gly Pro Ala Gly Pro His Ile Pro Pro Ser Asp
        435                 440                 445

Glu Ile Cys Glu Pro Gly Pro Pro Gly Pro Pro Gly Ser Pro Gly Asp
    450                 455                 460

Lys Gly Leu Gln Gly Glu Gln Gly Val Lys Gly Asp Lys Gly Asp Thr
465                 470                 475                 480

Cys Phe Asn Cys Ile Gly Thr Gly Ile Ser Gly Pro Pro Gly Gln Pro
                485                 490                 495

Gly Leu Pro Gly Leu Pro Gly Pro Pro Gly Ser Leu Gly Phe Pro Gly
            500                 505                 510

Gln Lys Gly Glu Lys Gly Gln Ala Gly Ala Thr Gly Pro Lys Gly Leu
        515                 520                 525

Pro Gly Ile Pro Gly Ala Pro Gly Ala Pro Gly Phe Pro Gly Ser Lys
    530                 535                 540

Gly Glu Pro Gly Asp Ile Leu Thr Phe Pro Gly Met Lys Gly Asp Lys
545                 550                 555                 560

Gly Glu Leu Gly Ser Pro Gly Ala Pro Gly Leu Pro Gly Leu Pro Gly
                565                 570                 575

Thr Pro Gly Gln Asp Gly Leu Pro Gly Leu Pro Gly Pro Lys Gly Glu
            580                 585                 590

Pro Gly Gly Ile Thr Phe Lys Gly Glu Arg Gly Pro Pro Gly Asn Pro
        595                 600                 605

Gly Leu Pro Gly Leu Pro Gly Asn Ile Gly Pro Met Gly Pro Pro Gly
    610                 615                 620

Phe Gly Pro Pro Gly Pro Val Gly Glu Lys Gly Ile Gln Gly Val Ala
625                 630                 635                 640

Gly Asn Pro Gly Gln Pro Gly Ile Pro Gly Pro Lys Gly Asp Pro Gly
                645                 650                 655

Gln Thr Ile Thr Gln Pro Gly Lys Pro Gly Leu Pro Gly Asn Pro Gly
            660                 665                 670

Arg Asp Gly Asp Val Gly Leu Pro Gly Asp Pro Gly Leu Pro Gly Gln
        675                 680                 685

Pro Gly Leu Pro Gly Ile Pro Gly Ser Lys Gly Glu Pro Gly Ile Pro
    690                 695                 700

Gly Ile Gly Leu Pro Gly Pro Pro Gly Pro Lys Gly Phe Pro Gly Ile
705                 710                 715                 720

Pro Gly Pro Pro Gly Ala Pro Gly Thr Pro Gly Arg Ile Gly Leu Glu
                725                 730                 735

Gly Pro Pro Gly Pro Pro Gly Phe Pro Gly Pro Lys Gly Glu Pro Gly
            740                 745                 750

Phe Ala Leu Pro Gly Pro Pro Gly Pro Pro Gly Leu Pro Gly Phe Lys

        755                 760                 765

Gly Ala Leu Gly Pro Lys Gly Asp Arg Gly Phe Pro Gly Pro Pro Gly
    770                 775                 780

Pro Pro Gly Arg Thr Gly Leu Asp Gly Leu Pro Gly Pro Lys Gly Asp
785                 790                 795                 800

Val Gly Pro Asn Gly Gln Pro Gly Pro Met Gly Pro Pro Gly Leu Pro
                805                 810                 815

Gly Ile Gly Val Gln Gly Pro Pro Gly Pro Pro Gly Ile Pro Gly Pro
            820                 825                 830

Ile Gly Gln Pro Gly Leu His Gly Ile Pro Gly Glu Lys Gly Asp Pro
        835                 840                 845

Gly Pro Pro Gly Leu Asp Val Pro Gly Pro Pro Gly Glu Arg Gly Ser
    850                 855                 860

Pro Gly Ile Pro Gly Ala Pro Gly Pro Ile Gly Pro Pro Gly Ser Pro
865                 870                 875                 880

Gly Leu Pro Gly Lys Ala Gly Ala Ser Gly Phe Pro Gly Thr Lys Gly
                885                 890                 895

Glu Met Gly Met Met Gly Pro Pro Gly Pro Pro Gly Pro Leu Gly Ile
            900                 905                 910

Pro Gly Arg Ser Gly Val Pro Gly Leu Lys Gly Asp Asp Gly Leu Gln
        915                 920                 925

Gly Gln Pro Gly Leu Pro Gly Pro Thr Gly Glu Lys Gly Ser Lys Gly
    930                 935                 940

Glu Pro Gly Leu Pro Gly Pro Pro Gly Pro Met Asp Pro Asn Leu Leu
945                 950                 955                 960

Gly Ser Lys Gly Glu Lys Gly Glu Pro Gly Leu Pro Gly Ile Pro Gly
                965                 970                 975

Val Ser Gly Pro Lys Gly Tyr Gln Gly Leu Pro Gly Asp Pro Gly Gln
            980                 985                 990

Pro Gly Leu Ser Gly Gln Pro Gly Leu Pro Gly Pro Pro Gly Pro Lys
        995                 1000                1005

Gly Asn Pro Gly Leu Pro Gly Gln Pro Gly Leu Ile Gly Pro Pro
    1010                1015                1020

Gly Leu Lys Gly Thr Ile Gly Asp Met Gly Phe Pro Gly Pro Gln
    1025                1030                1035

Gly Val Glu Gly Pro Pro Gly Pro Ser Gly Val Pro Gly Gln Pro
    1040                1045                1050

Gly Ser Pro Gly Leu Pro Gly Gln Lys Gly Asp Lys Gly Asp Pro
    1055                1060                1065

Gly Ile Ser Ser Ile Gly Leu Pro Gly Leu Pro Gly Pro Lys Gly
    1070                1075                1080

Glu Pro Gly Leu Pro Gly Tyr Pro Gly Asn Pro Gly Ile Lys Gly
    1085                1090                1095

Ser Val Gly Asp Pro Gly Leu Pro Gly Leu Pro Gly Thr Pro Gly
    1100                1105                1110

Ala Lys Gly Gln Pro Gly Leu Pro Gly Phe Pro Gly Thr Pro Gly
    1115                1120                1125

Pro Pro Gly Pro Lys Gly Ile Ser Gly Pro Pro Gly Asn Pro Gly
    1130                1135                1140

Leu Pro Gly Glu Pro Gly Pro Val Gly Gly Gly Gly His Pro Gly
    1145                1150                1155

Gln Pro Gly Pro Pro Gly Glu Lys Gly Lys Pro Gly Gln Asp Gly
    1160                1165                1170

Ile Pro Gly Pro Ala Gly Gln Lys Gly Glu Pro Gly Gln Pro Gly
    1175                1180                1185

Phe Gly Asn Pro Gly Pro Pro Gly Leu Pro Gly Leu Ser Gly Gln
    1190                1195                1200

Lys Gly Asp Gly Gly Leu Pro Gly Ile Pro Gly Asn Pro Gly Leu
    1205                1210                1215

Pro Gly Pro Lys Gly Glu Pro Gly Phe His Gly Phe Pro Gly Val
    1220                1225                1230

Gln Gly Pro Pro Gly Pro Pro Gly Ser Pro Gly Pro Ala Leu Glu
    1235                1240                1245

Gly Pro Lys Gly Asn Pro Gly Pro Gln Gly Pro Pro Gly Arg Pro
    1250                1255                1260

Gly Pro Thr Gly Phe Gln Gly Leu Pro Gly Pro Glu Gly Pro Pro
    1265                1270                1275

Gly Leu Pro Gly Asn Gly Gly Ile Lys Gly Glu Lys Gly Asn Pro
    1280                1285                1290

Gly Gln Pro Gly Leu Pro Gly Leu Pro Gly Leu Lys Gly Asp Gln
    1295                1300                1305

Gly Pro Pro Gly Leu Gln Gly Asn Pro Gly Arg Pro Gly Leu Asn
    1310                1315                1320

Gly Met Lys Gly Asp Pro Gly Leu Pro Gly Val Pro Gly Phe Pro
    1325                1330                1335

Gly Met Lys Gly Pro Ser Gly Val Pro Gly Ser Ala Gly Pro Glu
    1340                1345                1350

Gly Glu Pro Gly Leu Ile Gly Pro Pro Gly Pro Pro Gly Leu Pro
    1355                1360                1365

Gly Pro Ser Gly Gln Ser Ile Ile Ile Lys Gly Asp Ala Gly Pro
    1370                1375                1380

Pro Gly Ile Pro Gly Gln Pro Gly Leu Lys Gly Leu Pro Gly Pro
    1385                1390                1395

Gln Gly Pro Gln Gly Leu Pro Gly Pro Thr Gly Pro Pro Gly Asp
    1400                1405                1410

Pro Gly Arg Asn Gly Leu Pro Gly Phe Asp Gly Ala Gly Gly Arg
    1415                1420                1425

Lys Gly Asp Pro Gly Leu Pro Gly Gln Pro Gly Thr Arg Gly Leu
    1430                1435                1440

Asp Gly Pro Pro Gly Pro Asp Gly Leu Gln Gly Pro Pro Gly Pro
    1445                1450                1455

Pro Gly Thr Ser Ser Val Ala His Gly Phe Leu Ile Thr Arg His
    1460                1465                1470

Ser Gln Thr Thr Asp Ala Pro Gln Cys Pro Gln Gly Thr Leu Gln
    1475                1480                1485

Val Tyr Glu Gly Phe Ser Leu Leu Tyr Val Gln Gly Asn Lys Arg
    1490                1495                1500

Ala His Gly Gln Asp Leu Gly Thr Ala Gly Ser Cys Leu Arg Arg
    1505                1510                1515

Phe Ser Thr Met Pro Phe Met Phe Cys Asn Ile Asn Asn Val Cys
    1520                1525                1530

Asn Phe Ala Ser Arg Asn Asp Tyr Ser Tyr Trp Leu Ser Thr Pro
    1535                1540                1545

Glu Pro Met Pro Met Ser Met Gln Pro Leu Lys Gly Gln Ser Ile
    1550                1555                1560

Gln Pro Phe Ile Ser Arg Cys Ala Val Cys Glu Ala Pro Ala Val
    1565                1570                1575

Val Ile Ala Val His Ser Gln Thr Ile Gln Ile Pro His Cys Pro
    1580                1585                1590

Gln Gly Trp Asp Ser Leu Trp Ile Gly Tyr Ser Phe Met Met His
    1595                1600                1605

Thr Ser Ala Gly Ala Glu Gly Ser Gly Gln Ala Leu Ala Ser Pro
    1610                1615                1620

Gly Ser Cys Leu Glu Glu Phe Arg Ser Ala Pro Phe Ile Glu Cys
    1625                1630                1635

His Gly Arg Gly Thr Cys Asn Tyr Tyr Ala Asn Ser Tyr Ser Phe
    1640                1645                1650

Trp Leu Ala Thr Val Asp Val Ser Asp Met Phe Ser Lys Pro Gln
    1655                1660                1665

Ser Glu Thr Leu Lys Ala Gly Asp Leu Arg Thr Arg Ile Ser Arg
    1670                1675                1680

Cys Gln Val Cys Met Lys Arg Thr
    1685                1690

<210>  11
<211>  6774
<212>  DNA
<213>  Homo sapiens

<220>
<221>  CDS
<222>  (235)..(5358)

<220>
<221>  sig_peptide
<222>  (235)..(297)

<400>  11
atggaactat ctcctgagtg ctgcaagttg taacgggcac cgctgagcct gtttcccttt      60

ggagcacttc ttatctagaa gcagtgttta gtttcttcca aactgggcca cttcgtccac     120

ctactctgtt ctgagtaagg aaacagcctc caagcatcag cagagcccag atgagcacgg     180

gccgcggagc cgcttagcag tctcccggga cccagctccg gaggagccgc aagc atg       237
                                                            Met
                                                            1

cac cct ggg ttg tgg ctg ctc ctg gtt acg ttg tgc ctg acc gag gaa       285
His Pro Gly Leu Trp Leu Leu Leu Val Thr Leu Cys Leu Thr Glu Glu
            5                   10                  15

ctg gca gca gcg gga gag aag tct tat gga aag cca tgt ggg ggc cag       333
Leu Ala Ala Ala Gly Glu Lys Ser Tyr Gly Lys Pro Cys Gly Gly Gln
        20                  25                  30

gac tgc agt ggg agc tgt cag tgt ttt cct gag aaa gga gcg aga gga       381
Asp Cys Ser Gly Ser Cys Gln Cys Phe Pro Glu Lys Gly Ala Arg Gly
    35                  40                  45

cga cct gga cca att gga att caa ggc cca aca ggt cct caa gga ttc       429
Arg Pro Gly Pro Ile Gly Ile Gln Gly Pro Thr Gly Pro Gln Gly Phe
50                  55                  60                  65

act ggc tct act ggt tta tcg gga ttg aaa gga gaa agg ggt ttc cca       477
Thr Gly Ser Thr Gly Leu Ser Gly Leu Lys Gly Glu Arg Gly Phe Pro
                70                  75                  80

ggc ctt ctg gga cct tat gga cca aaa gga gat aag ggt ccc atg gga       525
Gly Leu Leu Gly Pro Tyr Gly Pro Lys Gly Asp Lys Gly Pro Met Gly
            85                  90                  95

gtt cct ggc ttt ctt ggc atc aat ggg att ccg ggc cac cct gga caa       573
Val Pro Gly Phe Leu Gly Ile Asn Gly Ile Pro Gly His Pro Gly Gln
        100                 105                 110

cca ggc ccc aga ggc cca cct ggt ctg gat ggc tgt aat gga act caa       621
Pro Gly Pro Arg Gly Pro Pro Gly Leu Asp Gly Cys Asn Gly Thr Gln
    115                 120                 125

gga gct gtt gga ttt cca ggc cct gat ggc tat cct ggg ctt ctc gga       669
Gly Ala Val Gly Phe Pro Gly Pro Asp Gly Tyr Pro Gly Leu Leu Gly
130                 135                 140                 145

cca ccc ggg ctt cct ggt cag aaa gga tca aaa ggt gac cct gtc ctt       717
Pro Pro Gly Leu Pro Gly Gln Lys Gly Ser Lys Gly Asp Pro Val Leu
                150                 155                 160

gct cca ggt agt ttc aaa gga atg aag ggg gat cct ggg ctg cct gga       765
Ala Pro Gly Ser Phe Lys Gly Met Lys Gly Asp Pro Gly Leu Pro Gly
            165                 170                 175

ctg gat gga atc act ggc cca caa gga gca ccc gga ttt cct gga gct 813
Leu Asp Gly Ile Thr Gly Pro Gln Gly Ala Pro Gly Phe Pro Gly Ala
        180                 185                 190

gta gga cct gca gga cca cca gga tta caa ggt cct cca ggg cct cct 861
Val Gly Pro Ala Gly Pro Pro Gly Leu Gln Gly Pro Pro Gly Pro Pro
    195                 200                 205

ggt cct ctt ggt cct gat ggg aat atg ggg cta ggt ttt caa gga gag 909
Gly Pro Leu Gly Pro Asp Gly Asn Met Gly Leu Gly Phe Gln Gly Glu
210                 215                 220                 225

aaa gga gtc aag ggg gat gtt ggc ctc cct ggc cca gca gga cct cca 957
Lys Gly Val Lys Gly Asp Val Gly Leu Pro Gly Pro Ala Gly Pro Pro
                230                 235                 240

cca tct act gga gag ctg gaa ttc atg gga ttc ccc aaa ggg aag aaa 1005
Pro Ser Thr Gly Glu Leu Glu Phe Met Gly Phe Pro Lys Gly Lys Lys
            245                 250                 255

gga tcc aag ggt gaa cca ggg cct aag ggt ttt cca ggc ata agt ggc 1053
Gly Ser Lys Gly Glu Pro Gly Pro Lys Gly Phe Pro Gly Ile Ser Gly
        260                 265                 270

cct cca ggc ttc ccg ggc ctt gga act act gga gaa aag gga gaa aag 1101
Pro Pro Gly Phe Pro Gly Leu Gly Thr Thr Gly Glu Lys Gly Glu Lys
    275                 280                 285

gga gaa aag gga atc cct ggt ttg cca gga cct agg ggt ccc atg ggt 1149
Gly Glu Lys Gly Ile Pro Gly Leu Pro Gly Pro Arg Gly Pro Met Gly
290                 295                 300                 305

tca gaa gga gtc caa ggc cct cca ggg caa cag ggc aag aaa ggg acc 1197
Ser Glu Gly Val Gln Gly Pro Pro Gly Gln Gln Gly Lys Lys Gly Thr
                310                 315                 320

ctg gga ttt cct ggg ctt aat gga ttc caa gga att gag ggt caa aag 1245
Leu Gly Phe Pro Gly Leu Asn Gly Phe Gln Gly Ile Glu Gly Gln Lys
            325                 330                 335

ggt gac att ggc ctg cca ggc cca gat gtt ttc atc gat ata gat ggt 1293
Gly Asp Ile Gly Leu Pro Gly Pro Asp Val Phe Ile Asp Ile Asp Gly
        340                 345                 350

gct gtg atc tca ggt aat cct gga gat cct ggt gta cct ggc ctc cca 1341
Ala Val Ile Ser Gly Asn Pro Gly Asp Pro Gly Val Pro Gly Leu Pro
    355                 360                 365

ggc ctt aaa gga gat gaa ggc atc caa ggc cta cgt ggc cct tct ggt 1389
Gly Leu Lys Gly Asp Glu Gly Ile Gln Gly Leu Arg Gly Pro Ser Gly
370                 375                 380                 385

gtc cct gga ttg cca gca tta tca ggt gtc cca gga gcc cta ggg cct 1437
Val Pro Gly Leu Pro Ala Leu Ser Gly Val Pro Gly Ala Leu Gly Pro
                390                 395                 400

cag gga ttt cca ggg ctg aag ggg gac caa gga aac cca ggc cgt acc 1485
Gln Gly Phe Pro Gly Leu Lys Gly Asp Gln Gly Asn Pro Gly Arg Thr
            405                 410                 415

aca att gga gca gct ggc ctc cct ggc aga gat ggt ttg cca ggc cca 1533
Thr Ile Gly Ala Ala Gly Leu Pro Gly Arg Asp Gly Leu Pro Gly Pro

420                 425                 430

cca ggt cca cca ggc cca cct agt cca gaa ttt gag act gaa act cta      1581
Pro Gly Pro Pro Gly Pro Pro Ser Pro Glu Phe Glu Thr Glu Thr Leu
    435                 440                 445

cac aac aaa gag tca ggg ttc cct ggt ctc cga gga gaa caa ggt cca      1629
His Asn Lys Glu Ser Gly Phe Pro Gly Leu Arg Gly Glu Gln Gly Pro
450                 455                 460                 465

aaa gga aac cta ggc ctc aaa gga ata aaa gga gac tca ggt ttc tgt      1677
Lys Gly Asn Leu Gly Leu Lys Gly Ile Lys Gly Asp Ser Gly Phe Cys
                470                 475                 480

gct tgt gac ggt ggt gtt ccc aac act gga cca ccc ggg gaa cca ggc      1725
Ala Cys Asp Gly Gly Val Pro Asn Thr Gly Pro Pro Gly Glu Pro Gly
            485                 490                 495

cca cct ggt cca tgg ggt ctc ata ggc ctt cca ggc ctt aaa gga gcc      1773
Pro Pro Gly Pro Trp Gly Leu Ile Gly Leu Pro Gly Leu Lys Gly Ala
        500                 505                 510

aga gga gat cga ggc tct ggg ggt gca cag ggc cca gca ggg gct cca      1821
Arg Gly Asp Arg Gly Ser Gly Gly Ala Gln Gly Pro Ala Gly Ala Pro
    515                 520                 525

ggc tta gtt ggg cct ctg ggt cct tca gga ccc aaa gga aag aag ggg      1869
Gly Leu Val Gly Pro Leu Gly Pro Ser Gly Pro Lys Gly Lys Lys Gly
530                 535                 540                 545

gaa cca att ctc agt aca atc caa gga atg cca gga gat cgg ggt gat      1917
Glu Pro Ile Leu Ser Thr Ile Gln Gly Met Pro Gly Asp Arg Gly Asp
                550                 555                 560

tct ggc tcc cag ggc ttc cgt ggt gta ata gga gaa cca ggc aag gac      1965
Ser Gly Ser Gln Gly Phe Arg Gly Val Ile Gly Glu Pro Gly Lys Asp
            565                 570                 575

gga gta cca ggt tta cca ggt ctg cca ggc ctt ccg ggt gat ggt gga      2013
Gly Val Pro Gly Leu Pro Gly Leu Pro Gly Leu Pro Gly Asp Gly Gly
        580                 585                 590

cag ggc ttc cca ggt gaa aag ggg tta cct gga ctt cct ggt gaa aaa      2061
Gln Gly Phe Pro Gly Glu Lys Gly Leu Pro Gly Leu Pro Gly Glu Lys
    595                 600                 605

ggc cat cct ggt cca cct ggc ctc cca gga aat ggg tta cca gga ctt      2109
Gly His Pro Gly Pro Pro Gly Leu Pro Gly Asn Gly Leu Pro Gly Leu
610                 615                 620                 625

cct gga ccc cgt ggg ctt cct gga gat aaa ggc aag gat gga tta ccg      2157
Pro Gly Pro Arg Gly Leu Pro Gly Asp Lys Gly Lys Asp Gly Leu Pro
                630                 635                 640

gga caa caa ggc ctt ccc gga tct aag ggt gac tgc tgc tgt aga gag      2205
Gly Gln Gln Gly Leu Pro Gly Ser Lys Gly Asp Cys Cys Cys Arg Glu
            645                 650                 655

gtt ggt aaa gga gac tta gac aca gag aga gga atc acc ctg ccc tgt      2253
Val Gly Lys Gly Asp Leu Asp Thr Glu Arg Gly Ile Thr Leu Pro Cys
        660                 665                 670

att att cct ggg tca tac ggt cca tca gga ttt cca ggc act ccc gga      2301

Ile Ile Pro Gly Ser Tyr Gly Pro Ser Gly Phe Pro Gly Thr Pro Gly
    675                 680                 685

ttc cca ggc cct aaa ggg tct cga ggc ctc cct ggg acc cca ggc cag      2349
Phe Pro Gly Pro Lys Gly Ser Arg Gly Leu Pro Gly Thr Pro Gly Gln
690                 695                 700                 705

cct ggg tca agt gga agt aaa gga gag cca ggg agt cca gga ttg gtt      2397
Pro Gly Ser Ser Gly Ser Lys Gly Glu Pro Gly Ser Pro Gly Leu Val
                710                 715                 720

cat ctt cct gaa tta cca gga ttt cct gga cct cgt ggg gag aag ggc      2445
His Leu Pro Glu Leu Pro Gly Phe Pro Gly Pro Arg Gly Glu Lys Gly
            725                 730                 735

ttg cct ggg ttt cct ggg ctc cct gga aaa gat ggc ttg cct ggg atg      2493
Leu Pro Gly Phe Pro Gly Leu Pro Gly Lys Asp Gly Leu Pro Gly Met
        740                 745                 750

att ggc agt cca ggc tta cct ggt tcc aag gga gcc act ggt gac atc      2541
Ile Gly Ser Pro Gly Leu Pro Gly Ser Lys Gly Ala Thr Gly Asp Ile
    755                 760                 765

ttt ggt gct gaa aat ggt gct ccg ggg gaa caa ggc cta caa gga tta      2589
Phe Gly Ala Glu Asn Gly Ala Pro Gly Glu Gln Gly Leu Gln Gly Leu
770                 775                 780                 785

aca ggg cac aaa gga ttt ctt gga gac tct ggc ctt cca gga ctc aag      2637
Thr Gly His Lys Gly Phe Leu Gly Asp Ser Gly Leu Pro Gly Leu Lys
                790                 795                 800

ggt gtg cac ggg aag cct ggc tta cta ggc ccc aaa ggt gag cgg ggc      2685
Gly Val His Gly Lys Pro Gly Leu Leu Gly Pro Lys Gly Glu Arg Gly
            805                 810                 815

agc cct ggg aca cca gga cag gtg gga cag cca ggc acc cca gga tct      2733
Ser Pro Gly Thr Pro Gly Gln Val Gly Gln Pro Gly Thr Pro Gly Ser
        820                 825                 830

agt ggt cca tat ggc atc aag ggc aaa tct ggg ctc cca gga gca cca      2781
Ser Gly Pro Tyr Gly Ile Lys Gly Lys Ser Gly Leu Pro Gly Ala Pro
    835                 840                 845

ggc ttc cca ggc atc tca gga cat cct gga aag aaa gga aca aga ggc      2829
Gly Phe Pro Gly Ile Ser Gly His Pro Gly Lys Lys Gly Thr Arg Gly
850                 855                 860                 865

aag aaa ggt cct cct gga tca att gta aag aaa ggg ctg cca ggg cta      2877
Lys Lys Gly Pro Pro Gly Ser Ile Val Lys Lys Gly Leu Pro Gly Leu
                870                 875                 880

aaa ggc ctt cct gga aat cca ggc cta gta gga ctg aaa gga agc cca      2925
Lys Gly Leu Pro Gly Asn Pro Gly Leu Val Gly Leu Lys Gly Ser Pro
            885                 890                 895

ggc tct cca ggg gtc gct ggg ttg cca gcc ctc tct gga ccc aag gga      2973
Gly Ser Pro Gly Val Ala Gly Leu Pro Ala Leu Ser Gly Pro Lys Gly
        900                 905                 910

gag aag ggg tct gtt gga ttc gta ggt ttt cca gga ata cca ggt ctg      3021
Glu Lys Gly Ser Val Gly Phe Val Gly Phe Pro Gly Ile Pro Gly Leu
    915                 920                 925

cct ggt att cct gga aca aga gga tta aaa gga att cca gga tca act 3069
Pro Gly Ile Pro Gly Thr Arg Gly Leu Lys Gly Ile Pro Gly Ser Thr
930                 935                 940                 945

gga aaa atg gga cca tct gga cgt gct ggt act cct ggt gaa aag gga 3117
Gly Lys Met Gly Pro Ser Gly Arg Ala Gly Thr Pro Gly Glu Lys Gly
                950                 955                 960

gac aga ggc aat ccg ggg cca gtc gga ata cct agt cca aga cgt cca 3165
Asp Arg Gly Asn Pro Gly Pro Val Gly Ile Pro Ser Pro Arg Arg Pro
            965                 970                 975

atg tca aac ctt tgg ctc aaa gga gac aaa ggc tct caa ggc tca gcc 3213
Met Ser Asn Leu Trp Leu Lys Gly Asp Lys Gly Ser Gln Gly Ser Ala
        980                 985                 990

gga tcc aat gga ttt cct ggg cca aga ggt gac aaa gga gag gct ggt 3261
Gly Ser Asn Gly Phe Pro Gly Pro Arg Gly Asp Lys Gly Glu Ala Gly
    995                 1000                1005

cga cct gga cca cca ggc cta cct gga gct cct ggc ctc cca ggc 3306
Arg Pro Gly Pro Pro Gly Leu Pro Gly Ala Pro Gly Leu Pro Gly
1010                1015                1020

att atc aaa gga gtt agt gga aag cca ggg ccc cct ggc ttc atg 3351
Ile Ile Lys Gly Val Ser Gly Lys Pro Gly Pro Pro Gly Phe Met
1025                1030                1035

gga atc cgg ggc tta cct ggc ctg aag ggg tcc tct ggg atc aca 3396
Gly Ile Arg Gly Leu Pro Gly Leu Lys Gly Ser Ser Gly Ile Thr
1040                1045                1050

ggt ttc cca gga atg cca gga gaa agt ggt tca caa ggt atc aga 3441
Gly Phe Pro Gly Met Pro Gly Glu Ser Gly Ser Gln Gly Ile Arg
1055                1060                1065

ggg tcg cct gga ctc cca gga gca tct ggt ctc cca ggc ctg aaa 3486
Gly Ser Pro Gly Leu Pro Gly Ala Ser Gly Leu Pro Gly Leu Lys
1070                1075                1080

gga gac aac ggc cag aca gtt gaa att tcc ggt agc cca gga ccc 3531
Gly Asp Asn Gly Gln Thr Val Glu Ile Ser Gly Ser Pro Gly Pro
1085                1090                1095

aag gga cag cct ggc gaa tct ggt ttt aaa ggc aca aaa gga aga 3576
Lys Gly Gln Pro Gly Glu Ser Gly Phe Lys Gly Thr Lys Gly Arg
1100                1105                1110

gat gga cta ata ggc aat ata ggc ttc cct gga aac aaa ggt gaa 3621
Asp Gly Leu Ile Gly Asn Ile Gly Phe Pro Gly Asn Lys Gly Glu
1115                1120                1125

gat gga aaa gtt ggt gtt tct gga gat gtt ggc ctt cct gga gct 3666
Asp Gly Lys Val Gly Val Ser Gly Asp Val Gly Leu Pro Gly Ala
1130                1135                1140

cca gga ttt cca gga gtt gcc ggc atg aga gga gaa cca gga ctt 3711
Pro Gly Phe Pro Gly Val Ala Gly Met Arg Gly Glu Pro Gly Leu
1145                1150                1155

cca ggt tct tct ggt cac caa ggg gca att ggg cct cta gga tcc 3756
Pro Gly Ser Ser Gly His Gln Gly Ala Ile Gly Pro Leu Gly Ser
1160                1165                1170

ccc gga tta ata gga ccc aaa ggc ttc cct gga ttt cct ggt tta 3801
Pro Gly Leu Ile Gly Pro Lys Gly Phe Pro Gly Phe Pro Gly Leu
1175                1180                1185

cat gga ctg aat ggg ctt ccg ggc acc aag ggt acc cat ggc act 3846
His Gly Leu Asn Gly Leu Pro Gly Thr Lys Gly Thr His Gly Thr
1190                1195                1200

cca gga cct agt atc acc ggt gtg cct ggg cct gct ggt ctc cct 3891
Pro Gly Pro Ser Ile Thr Gly Val Pro Gly Pro Ala Gly Leu Pro
1205                1210                1215

gga ccc aaa gga gaa aaa gga tat cca gga att ggc atc gga gct 3936
Gly Pro Lys Gly Glu Lys Gly Tyr Pro Gly Ile Gly Ile Gly Ala
1220                1225                1230

cca ggg aag ccg ggc ctg aga ggg caa aaa ggt gat cga ggt ttc 3981
Pro Gly Lys Pro Gly Leu Arg Gly Gln Lys Gly Asp Arg Gly Phe
1235                1240                1245

cca ggt ctc cag ggc cct gct ggt ctc ccc ggt gcc cca ggc atc 4026
Pro Gly Leu Gln Gly Pro Ala Gly Leu Pro Gly Ala Pro Gly Ile
1250                1255                1260

tcc ttg ccc tca ctc ata gca gga cag cct ggt gac ccc ggg cga 4071
Ser Leu Pro Ser Leu Ile Ala Gly Gln Pro Gly Asp Pro Gly Arg
1265                1270                1275

cca ggc cta gat gga gaa cga ggc cgc cca ggc ccc gct gga ccc 4116
Pro Gly Leu Asp Gly Glu Arg Gly Arg Pro Gly Pro Ala Gly Pro
1280                1285                1290

cca ggt ccc cct ggg cca tcc tcg aat caa ggc gac acc gga gac 4161
Pro Gly Pro Pro Gly Pro Ser Ser Asn Gln Gly Asp Thr Gly Asp
1295                1300                1305

cct ggc ttc cct gga att cct gga cct aaa ggg cct aag gga gac 4206
Pro Gly Phe Pro Gly Ile Pro Gly Pro Lys Gly Pro Lys Gly Asp
1310                1315                1320

caa gga att cca ggt ttt tct ggc ctc cct gga gag cta gga ctg 4251
Gln Gly Ile Pro Gly Phe Ser Gly Leu Pro Gly Glu Leu Gly Leu
1325                1330                1335

aaa ggc atg aga ggt gag cct ggc ttc atg ggg act cca ggc aag 4296
Lys Gly Met Arg Gly Glu Pro Gly Phe Met Gly Thr Pro Gly Lys
1340                1345                1350

gtt ggg cca cct gga gac cca gga ttt ccc gga atg aag ggg aag 4341
Val Gly Pro Pro Gly Asp Pro Gly Phe Pro Gly Met Lys Gly Lys
1355                1360                1365

gca ggg cca aga ggc tct tct ggc ctc caa ggt gat cct gga caa 4386
Ala Gly Pro Arg Gly Ser Ser Gly Leu Gln Gly Asp Pro Gly Gln
1370                1375                1380

aca cca act gca gaa gct gtc cag gtt cct cct gga ccc ttg ggt 4431
Thr Pro Thr Ala Glu Ala Val Gln Val Pro Pro Gly Pro Leu Gly
1385                1390                1395

cta cca ggg atc gat ggc atc cct ggc ctc act ggg gac cct ggg 4476
Leu Pro Gly Ile Asp Gly Ile Pro Gly Leu Thr Gly Asp Pro Gly

1400                    1405                    1410

gct caa ggc cct gta ggc cta caa ggc tcc aaa ggt tta cct ggc      4521
Ala Gln Gly Pro Val Gly Leu Gln Gly Ser Lys Gly Leu Pro Gly
1415                    1420                    1425

atc ccc ggt aaa gat ggc ccc agt ggg ctc cca ggc cca cct ggg      4566
Ile Pro Gly Lys Asp Gly Pro Ser Gly Leu Pro Gly Pro Pro Gly
1430                    1435                    1440

gct ctt ggt gat cct ggt ctg cct gga ctg caa ggc cct cca gga      4611
Ala Leu Gly Asp Pro Gly Leu Pro Gly Leu Gln Gly Pro Pro Gly
1445                    1450                    1455

ttt gaa gga gct cca ggg cag caa ggc ccc ttc ggg atg cct gga      4656
Phe Glu Gly Ala Pro Gly Gln Gln Gly Pro Phe Gly Met Pro Gly
1460                    1465                    1470

atg cct ggc cag agc atg aga gtg ggc tac acg ttg gta aag cac      4701
Met Pro Gly Gln Ser Met Arg Val Gly Tyr Thr Leu Val Lys His
1475                    1480                    1485

agc cag tcg gaa cag gtg ccc ccg tgt ccc atc ggg atg agc cag      4746
Ser Gln Ser Glu Gln Val Pro Pro Cys Pro Ile Gly Met Ser Gln
1490                    1495                    1500

ctg tgg gtg ggg tac agc tta ctg ttt gtg gag ggg caa gag aaa      4791
Leu Trp Val Gly Tyr Ser Leu Leu Phe Val Glu Gly Gln Glu Lys
1505                    1510                    1515

gcc cac aac cag gac ctg ggc ttt gct ggc tcc tgt ctg ccc cgc      4836
Ala His Asn Gln Asp Leu Gly Phe Ala Gly Ser Cys Leu Pro Arg
1520                    1525                    1530

ttc agc acc atg ccc ttc atc tac tgc aac atc aac gag gtg tgc      4881
Phe Ser Thr Met Pro Phe Ile Tyr Cys Asn Ile Asn Glu Val Cys
1535                    1540                    1545

cac tat gcc agg cgc aat gat aaa tct tac tgg ctc tcc act acc      4926
His Tyr Ala Arg Arg Asn Asp Lys Ser Tyr Trp Leu Ser Thr Thr
1550                    1555                    1560

gcc cct atc ccc atg atg ccc gtc agc cag acc cag att ccc cag      4971
Ala Pro Ile Pro Met Met Pro Val Ser Gln Thr Gln Ile Pro Gln
1565                    1570                    1575

tac atc agc cgc tgc tct gtg tgt gag gca ccc tcg caa gcc att      5016
Tyr Ile Ser Arg Cys Ser Val Cys Glu Ala Pro Ser Gln Ala Ile
1580                    1585                    1590

gct gtg cac agc cag gac atc acc atc ccg cag tgc ccc ctg ggc      5061
Ala Val His Ser Gln Asp Ile Thr Ile Pro Gln Cys Pro Leu Gly
1595                    1600                    1605

tgg cgc agc ctc tgg att ggg tac tct ttc ctc atg cac act gcc      5106
Trp Arg Ser Leu Trp Ile Gly Tyr Ser Phe Leu Met His Thr Ala
1610                    1615                    1620

gct ggt gcc gag ggt gga ggc cag tcc ctg gtc tca cct ggc tcc      5151
Ala Gly Ala Glu Gly Gly Gly Gln Ser Leu Val Ser Pro Gly Ser
1625                    1630                    1635

tgc cta gag gac ttt cgg gcc act cct ttc atc gaa tgc agt ggt      5196

Cys Leu Glu Asp Phe Arg Ala Thr Pro Phe Ile Glu Cys Ser Gly
1640                1645                1650

gcc cga ggc acc tgc cac tac ttt gca aac aag tac agt ttc tgg 5241
Ala Arg Gly Thr Cys His Tyr Phe Ala Asn Lys Tyr Ser Phe Trp
1655                1660                1665

ttg acc aca gtg gag gag agg cag cag ttt ggg gag ttg cct gtg 5286
Leu Thr Thr Val Glu Glu Arg Gln Gln Phe Gly Glu Leu Pro Val
1670                1675                1680

tct gaa acg ctg aaa gct ggg cag ctc cac act cga gtc agt cgc 5331
Ser Glu Thr Leu Lys Ala Gly Gln Leu His Thr Arg Val Ser Arg
1685                1690                1695

tgc cag gtg tgt atg aaa agc ctg tag ggtggcacct gccactctgc 5378
Cys Gln Val Cys Met Lys Ser Leu
1700                1705

cccttgccct cccctgcccc tcacaacagt cacctcacaa acctgaatgg tctgaagaag 5438

gaaggcctga gcccctttgc ctgtcaagtt gtacattgga gtctcatttg ggctagacta 5498

ccggacactc gtcaccccag ccctcgggtc catagagatg agcccaccct gctgagatct 5558

gctgtcctgt ttctgtcaag ctggtgctac tgtttgattt ggatgattgt gtgactattc 5618

atggctacct cagaaagatt tgatgggcca caactgtctt agactgctag ctttctcctt 5678

accgtcttga tcggaaagct cttcctaatc gctaatcagt catttcttca tgtacagagg 5738

tcagcacaca ttatttggct taaaccagaa cccagtgttt ccacacttaa attctctaac 5798

cgaatattca tggatggctc aagtctgcac agagcaagtc ctcactcttc aaggaggccc 5858

actgtgtcta ggcaggcaag agaattgaaa tgaggtgcca cccagtagcc cagagtgagc 5918

tttagctctc tctagaatga gcaagactgg gccccacatg gcttagagag gcttgaaggc 5978

cagcagctgg gttgggggtg gtggtcatta atggcatatg gtcctagaca aaccatctcc 6038

tccttgccgg ctccccctcc agccagagac agaggatgtg gcctggttca aagtaaagca 6098

gaggatgcaa caaatgtggc caagcctatc aaaggaaatg agaatgacag ccttttttcc 6158

tgggccagaa gtagaggggt gggtgcgtaa ggatgtgtga gttttgcttt tgactccagg 6218

aacaaaaagg taaatcccac atcccagttt ctcagaagtc cctgtttatt ccaaatgcca 6278

tccagatgtg tgcaatgtgg caaactgaag ctgcacagtg ttggtttcct tgtattctga 6338

ggatgttaaa gactttgtta aatggttatc caattgctct ttcacaggta gcctattaaa 6398

ctattttaat atgttttttt aaacctcata aaaatctagc acactcttct cttgagcagt 6458

tagcagacct aaagcaagcc tgaattggct atgcagtaca ttgtattctg tttgggggaa 6518

tttgttttag ccattttctt taattaccag ttttccagaa cactcttagc tatgttgaca 6578

tgaggcagtt ccttccaggt gattctgttt ccttaagtat tatataaact gtgccaatac 6638

agacaaagca taatcaatat aatctgaatt attgttatct ttacctcctg agtaataagc 6698

atggtgtcag ttttgtacat agcaaataaa ataaatgaaa tctgaacatg tgaaaaaaaa 6758

aaaaaaaaaa aaaaaa 6774

<210> 12
<211> 1707
<212> PRT
<213> Homo sapiens

<400> 12

Met His Pro Gly Leu Trp Leu Leu Leu Val Thr Leu Cys Leu Thr Glu
1               5                   10                  15

Glu Leu Ala Ala Ala Gly Glu Lys Ser Tyr Gly Lys Pro Cys Gly Gly
            20                  25                  30

Gln Asp Cys Ser Gly Ser Cys Gln Cys Phe Pro Glu Lys Gly Ala Arg
        35                  40                  45

Gly Arg Pro Gly Pro Ile Gly Ile Gln Gly Pro Thr Gly Pro Gln Gly
    50                  55                  60

Phe Thr Gly Ser Thr Gly Leu Ser Gly Leu Lys Gly Glu Arg Gly Phe
65                  70                  75                  80

Pro Gly Leu Leu Gly Pro Tyr Gly Pro Lys Gly Asp Lys Gly Pro Met
                85                  90                  95

Gly Val Pro Gly Phe Leu Gly Ile Asn Gly Ile Pro Gly His Pro Gly
            100                 105                 110

Gln Pro Gly Pro Arg Gly Pro Pro Gly Leu Asp Gly Cys Asn Gly Thr
        115                 120                 125

Gln Gly Ala Val Gly Phe Pro Gly Pro Asp Gly Tyr Pro Gly Leu Leu
    130                 135                 140

Gly Pro Pro Gly Leu Pro Gly Gln Lys Gly Ser Lys Gly Asp Pro Val
145                 150                 155                 160

Leu Ala Pro Gly Ser Phe Lys Gly Met Lys Gly Asp Pro Gly Leu Pro
                165                 170                 175

Gly Leu Asp Gly Ile Thr Gly Pro Gln Gly Ala Pro Gly Phe Pro Gly
            180                 185                 190

Ala Val Gly Pro Ala Gly Pro Pro Gly Leu Gln Gly Pro Pro Gly Pro
        195                 200                 205

Pro Gly Pro Leu Gly Pro Asp Gly Asn Met Gly Leu Gly Phe Gln Gly
    210                 215                 220

Glu Lys Gly Val Lys Gly Asp Val Gly Leu Pro Gly Pro Ala Gly Pro
225                 230                 235                 240

Pro Pro Ser Thr Gly Glu Leu Glu Phe Met Gly Phe Pro Lys Gly Lys
                245                 250                 255

Lys Gly Ser Lys Gly Glu Pro Gly Pro Lys Gly Phe Pro Gly Ile Ser
            260                 265                 270

Gly Pro Pro Gly Phe Pro Gly Leu Gly Thr Thr Gly Glu Lys Gly Glu
        275                 280                 285

Lys Gly Glu Lys Gly Ile Pro Gly Leu Pro Gly Pro Arg Gly Pro Met
    290                 295                 300

Gly Ser Glu Gly Val Gln Gly Pro Pro Gly Gln Gln Gly Lys Lys Gly
305                 310                 315                 320

Thr Leu Gly Phe Pro Gly Leu Asn Gly Phe Gln Gly Ile Glu Gly Gln
                325                 330                 335

Lys Gly Asp Ile Gly Leu Pro Gly Pro Asp Val Phe Ile Asp Ile Asp
            340                 345                 350

Gly Ala Val Ile Ser Gly Asn Pro Gly Asp Pro Gly Val Pro Gly Leu
        355                 360                 365

Pro Gly Leu Lys Gly Asp Glu Gly Ile Gln Gly Leu Arg Gly Pro Ser
    370                 375                 380

Gly Val Pro Gly Leu Pro Ala Leu Ser Gly Val Pro Gly Ala Leu Gly
385                 390                 395                 400

Pro Gln Gly Phe Pro Gly Leu Lys Gly Asp Gln Gly Asn Pro Gly Arg
                405                 410                 415

Thr Thr Ile Gly Ala Ala Gly Leu Pro Gly Arg Asp Gly Leu Pro Gly
            420                 425                 430

Pro Pro Gly Pro Pro Gly Pro Pro Ser Pro Glu Phe Glu Thr Glu Thr
        435                 440                 445

Leu His Asn Lys Glu Ser Gly Phe Pro Gly Leu Arg Gly Glu Gln Gly
    450                 455                 460

Pro Lys Gly Asn Leu Gly Leu Lys Gly Ile Lys Gly Asp Ser Gly Phe
465                 470                 475                 480

Cys Ala Cys Asp Gly Gly Val Pro Asn Thr Gly Pro Pro Gly Glu Pro
                485                 490                 495

Gly Pro Pro Gly Pro Trp Gly Leu Ile Gly Leu Pro Gly Leu Lys Gly
            500                 505                 510

Ala Arg Gly Asp Arg Gly Ser Gly Gly Ala Gln Gly Pro Ala Gly Ala
        515                 520                 525

Pro Gly Leu Val Gly Pro Leu Gly Pro Ser Gly Pro Lys Gly Lys Lys
    530                 535                 540

Gly Glu Pro Ile Leu Ser Thr Ile Gln Gly Met Pro Gly Asp Arg Gly
545                 550                 555                 560

Asp Ser Gly Ser Gln Gly Phe Arg Gly Val Ile Gly Glu Pro Gly Lys
                565                 570                 575

Asp Gly Val Pro Gly Leu Pro Gly Leu Pro Gly Leu Pro Gly Asp Gly
            580                 585                 590

Gly Gln Gly Phe Pro Gly Glu Lys Gly Leu Pro Gly Leu Pro Gly Glu
        595                 600                 605

Lys Gly His Pro Gly Pro Pro Gly Leu Pro Gly Asn Gly Leu Pro Gly
    610                 615                 620

Leu Pro Gly Pro Arg Gly Leu Pro Gly Asp Lys Gly Lys Asp Gly Leu
625                 630                 635                 640

Pro Gly Gln Gln Gly Leu Pro Gly Ser Lys Gly Asp Cys Cys Cys Arg
                645                 650                 655

Glu Val Gly Lys Gly Asp Leu Asp Thr Glu Arg Gly Ile Thr Leu Pro
            660                 665                 670

Cys Ile Ile Pro Gly Ser Tyr Gly Pro Ser Gly Phe Pro Gly Thr Pro
        675                 680                 685

Gly Phe Pro Gly Pro Lys Gly Ser Arg Gly Leu Pro Gly Thr Pro Gly
    690                 695                 700

Gln Pro Gly Ser Ser Gly Ser Lys Gly Glu Pro Gly Ser Pro Gly Leu

705                 710                 715                 720

Val His Leu Pro Glu Leu Pro Gly Phe Pro Gly Pro Arg Gly Glu Lys
                725                 730                 735

Gly Leu Pro Gly Phe Pro Gly Leu Pro Gly Lys Asp Gly Leu Pro Gly
            740                 745                 750

Met Ile Gly Ser Pro Gly Leu Pro Gly Ser Lys Gly Ala Thr Gly Asp
        755                 760                 765

Ile Phe Gly Ala Glu Asn Gly Ala Pro Gly Glu Gln Gly Leu Gln Gly
    770                 775                 780

Leu Thr Gly His Lys Gly Phe Leu Gly Asp Ser Gly Leu Pro Gly Leu
785                 790                 795                 800

Lys Gly Val His Gly Lys Pro Gly Leu Leu Gly Pro Lys Gly Glu Arg
                805                 810                 815

Gly Ser Pro Gly Thr Pro Gly Gln Val Gly Gln Pro Gly Thr Pro Gly
            820                 825                 830

Ser Ser Gly Pro Tyr Gly Ile Lys Gly Lys Ser Gly Leu Pro Gly Ala
        835                 840                 845

Pro Gly Phe Pro Gly Ile Ser Gly His Pro Gly Lys Lys Gly Thr Arg
    850                 855                 860

Gly Lys Lys Gly Pro Pro Gly Ser Ile Val Lys Lys Gly Leu Pro Gly
865                 870                 875                 880

Leu Lys Gly Leu Pro Gly Asn Pro Gly Leu Val Gly Leu Lys Gly Ser
                885                 890                 895

Pro Gly Ser Pro Gly Val Ala Gly Leu Pro Ala Leu Ser Gly Pro Lys
            900                 905                 910

Gly Glu Lys Gly Ser Val Gly Phe Val Gly Phe Pro Gly Ile Pro Gly
        915                 920                 925

Leu Pro Gly Ile Pro Gly Thr Arg Gly Leu Lys Gly Ile Pro Gly Ser
    930                 935                 940

Thr Gly Lys Met Gly Pro Ser Gly Arg Ala Gly Thr Pro Gly Glu Lys
945                 950                 955                 960

Gly Asp Arg Gly Asn Pro Gly Pro Val Gly Ile Pro Ser Pro Arg Arg
                965                 970                 975

Pro Met Ser Asn Leu Trp Leu Lys Gly Asp Lys Gly Ser Gln Gly Ser
            980                 985                 990

Ala Gly Ser Asn Gly Phe Pro Gly  Pro Arg Gly Asp Lys  Gly Glu Ala
        995                 1000                 1005

Gly Arg  Pro Gly Pro Pro Gly  Leu Pro Gly Ala Pro  Gly Leu Pro
    1010                 1015                 1020

Gly Ile  Ile Lys Gly Val Ser  Gly Lys Pro Gly Pro  Pro Gly Phe
    1025                 1030                 1035

Met Gly  Ile Arg Gly Leu Pro  Gly Leu Lys Gly Ser  Ser Gly Ile
    1040                 1045                 1050

Thr Gly  Phe Pro Gly Met Pro  Gly Glu Ser Gly Ser  Gln Gly Ile
    1055                 1060                 1065

Arg Gly  Ser Pro Gly Leu Pro  Gly Ala Ser Gly Leu  Pro Gly Leu
    1070                 1075                 1080

Lys Gly  Asp Asn Gly Gln Thr  Val Glu Ile Ser Gly  Ser Pro Gly
    1085                 1090                 1095

Pro Lys  Gly Gln Pro Gly Glu  Ser Gly Phe Lys Gly  Thr Lys Gly
    1100                 1105                 1110

Arg Asp  Gly Leu Ile Gly Asn  Ile Gly Phe Pro Gly  Asn Lys Gly
    1115                 1120                 1125

Glu Asp  Gly Lys Val Gly Val  Ser Gly Asp Val Gly  Leu Pro Gly
    1130                 1135                 1140

Ala Pro  Gly Phe Pro Gly Val  Ala Gly Met Arg Gly  Glu Pro Gly
    1145                 1150                 1155

Leu Pro  Gly Ser Ser Gly His  Gln Gly Ala Ile Gly  Pro Leu Gly
    1160                 1165                 1170

Ser Pro  Gly Leu Ile Gly Pro  Lys Gly Phe Pro Gly  Phe Pro Gly
    1175                 1180                 1185

Leu His  Gly Leu Asn Gly Leu  Pro Gly Thr Lys Gly  Thr His Gly
    1190                 1195                 1200

Thr Pro Gly Pro Ser Ile Thr Gly Val Pro Gly Pro Ala Gly Leu
    1205                1210                1215

Pro Gly Pro Lys Gly Glu Lys Gly Tyr Pro Gly Ile Gly Ile Gly
    1220                1225                1230

Ala Pro Gly Lys Pro Gly Leu Arg Gly Gln Lys Gly Asp Arg Gly
    1235                1240                1245

Phe Pro Gly Leu Gln Gly Pro Ala Gly Leu Pro Gly Ala Pro Gly
    1250                1255                1260

Ile Ser Leu Pro Ser Leu Ile Ala Gly Gln Pro Gly Asp Pro Gly
    1265                1270                1275

Arg Pro Gly Leu Asp Gly Glu Arg Gly Arg Pro Gly Pro Ala Gly
    1280                1285                1290

Pro Pro Gly Pro Pro Gly Pro Ser Ser Asn Gln Gly Asp Thr Gly
    1295                1300                1305

Asp Pro Gly Phe Pro Gly Ile Pro Gly Pro Lys Gly Pro Lys Gly
    1310                1315                1320

Asp Gln Gly Ile Pro Gly Phe Ser Gly Leu Pro Gly Glu Leu Gly
    1325                1330                1335

Leu Lys Gly Met Arg Gly Glu Pro Gly Phe Met Gly Thr Pro Gly
    1340                1345                1350

Lys Val Gly Pro Pro Gly Asp Pro Gly Phe Pro Gly Met Lys Gly
    1355                1360                1365

Lys Ala Gly Pro Arg Gly Ser Ser Gly Leu Gln Gly Asp Pro Gly
    1370                1375                1380

Gln Thr Pro Thr Ala Glu Ala Val Gln Val Pro Pro Gly Pro Leu
    1385                1390                1395

Gly Leu Pro Gly Ile Asp Gly Ile Pro Gly Leu Thr Gly Asp Pro
    1400                1405                1410

Gly Ala Gln Gly Pro Val Gly Leu Gln Gly Ser Lys Gly Leu Pro
    1415                1420                1425

Gly Ile Pro Gly Lys Asp Gly Pro Ser Gly Leu Pro Gly Pro Pro
    1430                1435                1440

Gly Ala Leu Gly Asp Pro Gly Leu Pro Gly Leu Gln Gly Pro Pro
    1445                1450                1455

Gly Phe Glu Gly Ala Pro Gly Gln Gln Gly Pro Phe Gly Met Pro
    1460                1465                1470

Gly Met Pro Gly Gln Ser Met Arg Val Gly Tyr Thr Leu Val Lys
    1475                1480                1485

His Ser Gln Ser Glu Gln Val Pro Pro Cys Pro Ile Gly Met Ser
    1490                1495                1500

Gln Leu Trp Val Gly Tyr Ser Leu Leu Phe Val Glu Gly Gln Glu
    1505                1510                1515

Lys Ala His Asn Gln Asp Leu Gly Phe Ala Gly Ser Cys Leu Pro
    1520                1525                1530

Arg Phe Ser Thr Met Pro Phe Ile Tyr Cys Asn Ile Asn Glu Val
    1535                1540                1545

Cys His Tyr Ala Arg Arg Asn Asp Lys Ser Tyr Trp Leu Ser Thr
    1550                1555                1560

Thr Ala Pro Ile Pro Met Met Pro Val Ser Gln Thr Gln Ile Pro
    1565                1570                1575

Gln Tyr Ile Ser Arg Cys Ser Val Cys Glu Ala Pro Ser Gln Ala
    1580                1585                1590

Ile Ala Val His Ser Gln Asp Ile Thr Ile Pro Gln Cys Pro Leu
    1595                1600                1605

Gly Trp Arg Ser Leu Trp Ile Gly Tyr Ser Phe Leu Met His Thr
    1610                1615                1620

Ala Ala Gly Ala Glu Gly Gly Gly Gln Ser Leu Val Ser Pro Gly
    1625                1630                1635

Ser Cys Leu Glu Asp Phe Arg Ala Thr Pro Phe Ile Glu Cys Ser
    1640                1645                1650

Gly Ala Arg Gly Thr Cys His Tyr Phe Ala Asn Lys Tyr Ser Phe
    1655                1660                1665

Trp Leu Thr Thr Val Glu Glu Arg Gln Gln Phe Gly Glu Leu Pro

1670                 1675                 1680

Val Ser Glu Thr Leu Lys Ala Gly Gln Leu His Thr Arg Val Ser
    1685                 1690                 1695

Arg Cys Gln Val Cys Met Lys Ser Leu
    1700                 1705

<210>  13
<211>  36
<212>  DNA
<213>  Artificial Sequence

<220>
<223>  primer

<400>  13
ccctcctggt tctccggatc cagctctgga aggacc                                36

<210>  14
<211>  36
<212>  DNA
<213>  Artificial Sequence

<220>
<223>  primer

<400>  14
ggtccttcca gagctggatc cggagaacca ggaggg                                36

<210>  15
<211>  38
<212>  DNA
<213>  Artificial Sequence

<220>
<223>  primer

<400>  15
gtcccccagg ccctcctgtt tctccgggtc cagctctg                              38

<210>  16
<211>  38
<212>  DNA
<213>  Artificial Sequence

<220>
<223>  primer

<400>  16
cagagctgga cccggagaaa caggagggcc tgggggac                              38

<210>  17
<211>  19
<212>  RNA
<213>  Artificial Sequence

<220>
<223>  PPIA siRNA

<400>  17
caagaugacu aaugucaaa                                                    19


<210>  18
<211>  19
<212>  RNA
<213>  Artificial Sequence

<220>
<223>  PPIA siRNA

<400>  18
uuugacauua gucaucuug                                                    19


<210>  19
<211>  25
<212>  RNA
<213>  Artificial Sequence

<220>
<223>  PPIB siRNA

<400>  19
ggucucuucg gaaagacugu uccaa                                             25


<210>  20
<211>  25
<212>  RNA
<213>  Artificial Sequence

<220>
<223>  PPIB siRNA

<400>  20
uuggaacagu cuuuccgaag agacc                                             25


<210>  21
<211>  25
<212>  RNA
<213>  Artificial Sequence

<220>
<223>  PPIC siRNA

<400>  21
gcaacaggag agaaaggaua uggau                                             25


<210>  22
<211>  25
<212>  RNA
<213>  Artificial Sequence

<220>
<223>  PPIC siRNA

<400>  22
auccauaucc uuucucuccu guugc                                            25


<210>  23
<211>  25
<212>  RNA
<213>  Artificial Sequence

<220>
<223>  PPID siRNA

<400>  23
gccaaguaau uaaaggaaua ggagu                                            25


<210>  24
<211>  25
<212>  RNA
<213>  Artificial Sequence

<220>
<223>  PPID siRNA

<400>  24
acuccuauuc cuuuaauuac uuggc                                            25


<210>  25
<211>  25
<212>  RNA
<213>  Artificial Sequence

<220>
<223>  PPIE siRNA

<400>  25
ccaaugagaa uuaaggaagg cucuu                                            25


<210>  26
<211>  25
<212>  RNA
<213>  Artificial Sequence

<220>
<223>  PPIE siRNA

<400>  26
aagagccuuc cuuaauucuc auugg                                            25


<210>  27
<211>  19
<212>  RNA
<213>  Artificial Sequence

<220>
<223>  PPIF siRNA

<400>  27
ggcagauguc gucccaaag                                                   19

<210>  28
<211>  19
<212>  RNA
<213>  Artificial Sequence

<220>
<223>  PPIF siRNA

<400>  28
cuuugggacg acaucugcc                                                    19


<210>  29
<211>  25
<212>  RNA
<213>  Artificial Sequence

<220>
<223>  PPIG siRNA

<400>  29
gggauaagag ugaguugaau gaaau                                             25


<210>  30
<211>  25
<212>  RNA
<213>  Artificial Sequence

<220>
<223>  PPIG siRNA

<400>  30
auuucauuca acucacucuu auccc                                             25


<210>  31
<211>  25
<212>  RNA
<213>  Artificial Sequence

<220>
<223>  PPIH siRNA

<400>  31
cgugguguuc uuugauguca guauu                                             25


<210>  32
<211>  25
<212>  RNA
<213>  Artificial Sequence

<220>
<223>  PPIH siRNA

<400>  32
aauacugaca ucaaagaaca ccacg                                             25


<210>  33

<211>  25
<212>  RNA
<213>  Artificial Sequence

<220>
<223>  PPIL1 siRNA

<400>  33
gaacuuugcu gaguuggcuc gucga                                              25


<210>  34
<211>  25
<212>  RNA
<213>  Artificial Sequence

<220>
<223>  PPIL1 siRNA

<400>  34
ucgacgagcc aacucagcaa aguuc                                              25


<210>  35
<211>  25
<212>  RNA
<213>  Artificial Sequence

<220>
<223>  NKTR siRNA

<400>  35
ggaaagcagc auguccgaaa guaaa                                              25


<210>  36
<211>  25
<212>  RNA
<213>  Artificial Sequence

<220>
<223>  NKTR siRNA

<400>  36
uuuacuuucg gacaugcugc uuucc                                              25


<210>  37
<211>  25
<212>  RNA
<213>  Artificial Sequence

<220>
<223>  PPWD1 siRNA

<400>  37
gacccaacaa uagucuguac aucau                                              25


<210>  38
<211>  25
<212>  RNA
<213>  Artificial Sequence

<220>
<223>  PPWD1 siRNA

<400>  38
augauguaca gacuauuguu ggguc                                              25


<210>  39
<211>  34
<212>  DNA
<213>  Artificial Sequence

<220>
<223>  loxP

<400>  39
ataacttcgt atagcataca ttatacgaag ttat                                    34


<210>  40
<211>  34
<212>  DNA
<213>  Artificial Sequence

<220>
<223>  loxKMR3

<400>  40
ataacttcgt atagcataca ttataccttg ttat                                    34


<210>  41
<211>  34
<212>  DNA
<213>  Artificial Sequence

<220>
<223>  lox2272

<400>  41
ataacttcgt ataggatact ttatacgaag ttat                                    34


<210>  42
<211>  30
<212>  DNA
<213>  Artificial Sequence

<220>
<223>  primer

<400>  42
aagggttccg gatccccatc aagcttatcg                                         30


<210>  43
<211>  28
<212>  DNA
<213>  Artificial Sequence

<220>
<223>  primer

<400>  43
gggtagggat ggaagttcac caaagccc                                          28


<210>  44
<211>  36
<212>  DNA
<213>  Artificial Sequence

<220>
<223>  primer

<400>  44
cctgtgctca ctctttagta gatcaatttt gcatcc                                 36


<210>  45
<211>  30
<212>  DNA
<213>  Artificial Sequence

<220>
<223>  primer

<400>  45
gctacacata atgacataca cctgaggctg                                        30


<210>  46
<211>  32
<212>  DNA
<213>  Artificial Sequence

<220>
<223>  primer

<400>  46
ccgagctcgt cgacaagctc gaattagctt gg                                     32


<210>  47
<211>  31
<212>  DNA
<213>  Artificial Sequence

<220>
<223>  primer

<400>  47
gagtcaattt gaccatgctg tgctgctgaa g                                      31


<210>  48
<211>  32
<212>  DNA
<213>  Artificial Sequence

<220>
<223>  primer

<400>  48
ggaggtgaca tgtgccagtc tgcgggaact gg                                     32

<210>  49
<211>  31
<212>  DNA
<213>  Artificial Sequence

<220>
<223>  primer

<400>  49
gagtcaattt gaccatgctg tgctgctgaa g                                    31


<210>  50
<211>  36
<212>  DNA
<213>  Artificial Sequence

<220>
<223>  primer

<400>  50
acacagatct gatatcatcg atgaattcga gcttgc                               36


<210>  51
<211>  27
<212>  DNA
<213>  Artificial Sequence

<220>
<223>  primer

<400>  51
ggtcggtggg agagactggt cttcctg                                         27


<210>  52
<211>  25
<212>  DNA
<213>  Artificial Sequence

<220>
<223>  primer

<400>  52
tggagcctgg tactccaatg ggtcc                                           25


<210>  53
<211>  20
<212>  PRT
<213>  Artificial Sequence

<220>
<223>  inhibitor

<400>  53

Glu Phe Gly Gly Val Met Cys Val Glu Ser Val Asn Arg Glu Met Ser
1               5                   10                  15

Pro Leu Val Asp
            20

<210>  54
<211>  20
<212>  PRT
<213>  Artificial Sequence

<220>
<223>  inhibitor

<400>  54

Arg Glu Met Ser Pro Leu Val Asp Asn Ile Ala Leu Trp Met Thr Glu
1               5                   10                  15

Tyr Leu Asn Arg
            20

<210>  55
<211>  20
<212>  PRT
<213>  Artificial Sequence

<220>
<223>  inhibitor

<400>  55

Met Cys Val Glu Ser Val Asn Arg Glu Met Ser Pro Leu Val Asp Asn
1               5                   10                  15

Ile Ala Leu Trp
            20

<210>  56
<211>  20
<212>  PRT
<213>  Artificial Sequence

<220>
<223>  inhibitor

<400>  56

Leu Leu Ser Leu Ala Leu Val Gly Ala Cys Ile Thr Leu Gly Ala Tyr
1               5                   10                  15

Leu Gly His Lys
            20

<210>  57
<211>  20
<212>  DNA
<213>  Artificial Sequence

<220>
<223>  siRNA

<400>  57
gtcctcccac tcttagagcc                                                   20


<210>  58
<211>  20
<212>  DNA
<213>  Artificial Sequence

<220>
<223>  siRNA

<400>  58
gtcctcccac tcttagagcc                                                   20


<210>  59
<211>  20
<212>  DNA
<213>  Artificial Sequence

<220>
<223>  siRNA

<400>  59
cttcccgcct gtgccattgt                                                   20


<210>  60
<211>  20
<212>  DNA
<213>  Artificial Sequence

<220>
<223>  siRNA

<400>  60
gatgtcctcc cactcttaga                                                   20


<210>  61
<211>  21
<212>  DNA
<213>  Artificial Sequence

<220>
<223>  siRNA

<400>  61
tgtcctccca ctcttagagc c                                                 21


<210>  62
<211>  24
<212>  RNA
<213>  Artificial Sequence

<220>
<223>  siRNA

<400>  62
ggaggacauc caagaagauu guca                                            24


<210>  63
<211>  27
<212>  RNA
<213>  Artificial Sequence

<220>
<223>  siRNA

<400>  63
augacaaucu ucuuggaugu ccuccca                                         27


<210>  64
<211>  25
<212>  RNA
<213>  Artificial Sequence

<220>
<223>  siRNA

<400>  64
cccaaagaca gcugagaacu ucaga                                           25


<210>  65
<211>  27
<212>  RNA
<213>  Artificial Sequence

<220>
<223>  siRNA

<400>  65
ucugaaguuc ucagcugucu uugggac                                         27


<210>  66
<211>  25
<212>  RNA
<213>  Artificial Sequence

<220>
<223>  siRNA

<400>  66
   gcuccaccuu ccacagggug auccc                                            25


<210>  67
<211>  27
<212>  RNA
<213>  Artificial Sequence

<220>
<223>  siRNA

<400>  67
gggaucaccc uguggaaggu ggagccu                                         27

<210>  68
<211>  24
<212>  RNA
<213>  Artificial Sequence

<220>
<223>  siRNA

<400>  68
cagacugguu ggauggcaag caug                                              24


<210>  69
<211>  27
<212>  RNA
<213>  Artificial Sequence

<220>
<223>  siRNA

<400>  69
acaugcuugc cauccaacca gucuguc                                           27


<210>  70
<211>  25
<212>  RNA
<213>  Artificial Sequence

<220>
<223>  siRNA

<400>  70
ggcuaaugcu gguccuaaca ccaac                                             25


<210>  71
<211>  27
<212>  RNA
<213>  Artificial Sequence

<220>
<223>  siRNA

<400>  71
guugguguua ggaccagcau uagccau                                           27